# EUROPEAN PATENT SPECIFICATION

(11) **EP 2 459 204 B1**
(45) Date of publication and mention of the grant of the patent: **31.12.2014**
(21) Application number: 10751820.1
(22) Date of filing: 28.07.2010
(51) Int. Cl.: A61K 35/74, A61K 39/04, C12N 9/10, C12N 9/16

(54) **MYCOBACTERIUM MUTANTS FOR VACCINES WITH IMPROVED PROTECTIVE EFFICACY**
MYKOBAKTERIENMUTANTEN FÜR IMPFSTOFFE MIT ERHÖHTER SCHUTZWIRKUNG
MUTANTS DE MYCOBACTÉRIE POUR VACCINS PRÉSENTANT UNE EFFICACITÉ PROTECTRICE AMÉLIORÉE

(30) Priority: 28.07.2009 US 229062 P
(43) Date of publication of application: 06.06.2012
(73) Proprietor: VIB vzw, 9052 Ghent (BE); Universiteit Gent, 9000 Gent (BE); Wetenschappelijk Instituut Volksgezondheid, 1050 Brussel (BE)
(72) Inventor: CALLEWAERT, Nico, B-9850 Nevele (BE); BATNI, Anjana, B-9000 Gent (BE); FESTJENS, Nele, B-9690 Kluisbergen (BE); HUYGEN, Christiane, B-1180 Ukkel (BE)
(86) International application number: PCT/EP2010/060986
(87) International publication number: WO 2011/012662

(56) References cited:
- WO-A2-2004/033677
- WO-A2-2006/078164
- WO-A2-2007/084353
- WO-A2-2009/009798

### SEQUENCE LISTING

<110> VIB vzw
   Universiteit Gent
   Wetenschappelijk Instituut Volksgezondheid
   Callewaert, Nico
   Batni, Anjana
   Festjens, Nele
   Huygen, Christiane
<120> Mycobacterium mutants for vaccines with improved protective efficacy
<130> VIB-069-PCT
<150> US 61/229,062
   <151> 2009-07-28
<160> 22
<170> PatentIn version 3.3
<210> 1
   <211> 900
   <212> DNA
   <213> Mycobacterium bovis
<220>
   <221> CDS
   <222> (1)..(900)
<400> 1
   atg ctc cgc gga atc cag gct ctc agc cgg ccc ctg acc agg gta tac       48
   Met Leu Arg Gly Ile Gln Ala Leu Ser Arg Pro Leu Thr Arg Val Tyr
   1               5                   10                  15
   cgt gcc ttg gcg gtg atc ggt gtc ctg gca gca tcg ttg ctg gcc tca       96
   Arg Ala Leu Ala Val Ile Gly Val Leu Ala Ala Ser Leu Leu Ala Ser
               20                  25                  30
   tgg gtc ggc gct gtc cca caa gtg ggt ctg gca gcg agt gcc ctg ccg      144
   Trp Val Gly Ala Val Pro Gln Val Gly Leu Ala Ala Ser Ala Leu Pro
           35                  40                  45
   acc ttc gcg cac gtg gtc atc gtg gtg gag gag aac cgc tcg cag gcc      192
   Thr Phe Ala His Val Val Ile Val Val Glu Glu Asn Arg Ser Gln Ala
       50                  55                  60
   gcc atc atc ggt aac aag tcg gct ccc ttc atc aat tcg ctg gcc gcc      240
   Ala Ile Ile Gly Asn Lys Ser Ala Pro Phe Ile Asn Ser Leu Ala Ala
   65                  70                  75                  80
   aac ggc gcg atg atg gcc cag gcg ttc gcc gaa aca cac ccg agc gaa      288
   Asn Gly Ala Met Met Ala Gln Ala Phe Ala Glu Thr His Pro Ser Glu
                   85                  90                  95
   ccg aac tac ctg gca ctg ttc gct ggc aac aca ttc ggg ttg acg aag      336
   Pro Asn Tyr Leu Ala Leu Phe Ala Gly Asn Thr Phe Gly Leu Thr Lys
               100                 105                 110
   aac acc tgc ccc gtc aac ggc ggc gcg ctg ccc aac ctg ggt tct gag      384
   Asn Thr Cys Pro Val Asn Gly Gly Ala Leu Pro Asn Leu Gly Ser Glu
           115                 120                 125
   ttg ctc agc gcc ggt tac aca ttc atg ggg ttc gcc gaa gac ttg cct      432
   Leu Leu Ser Ala Gly Tyr Thr Phe Met Gly Phe Ala Glu Asp Leu Pro
       130                 135                 140
   gcg gtc ggc tcc acg gtg tgc agt gcg ggc aaa tac gca cgc aaa cac      480
   Ala Val Gly Ser Thr Val Cys Ser Ala Gly Lys Tyr Ala Arg Lys His
   145                 150                 155                 160
   gtg ccg tgg gtc aac ttc agt aac gtg ccg gcg aca ctg tcg gtg ccg      528
   Val Pro Trp Val Asn Phe Ser Asn Val Pro Ala Thr Leu Ser Val Pro
                   165                 170                 175
   ttt tcg gca ttt ccg aag ccg cag aat tac ccc ggc ctg ccg acg gtg      576
   Phe Ser Ala Phe Pro Lys Pro Gln Asn Tyr Pro Gly Leu Pro Thr Val
               180                 185                 190
   tcg ttt gtc atc cct aac gcc gac aac gac atg cac gac ggc tcg atc      624
   Ser Phe Val Ile Pro Asn Ala Asp Asn Asp Met His Asp Gly Ser Ile
           195                 200                 205
   gcc caa ggc gac gcc tgg ctg aac cgc cac ctg tcg gca tat gcc aac      672
   Ala Gln Gly Asp Ala Trp Leu Asn Arg His Leu Ser Ala Tyr Ala Asn
       210                 215                 220
   tgg gcc aag aca aac aac agc ctg ctc gtt gtg acc tgg gac gaa gac      720
   Trp Ala Lys Thr Asn Asn Ser Leu Leu Val Val Thr Trp Asp Glu Asp
   225                 230                 235                 240
   gac ggc agc agc cgc aat cag atc ccg acg gtg ttc tac ggc gcg cac      768
   Asp Gly Ser Ser Arg Asn Gln Ile Pro Thr Val Phe Tyr Gly Ala His
                   245                 250                 255
   gtg cgg ccc gga act tac aac gag acc atc agc cac tac aac gtg ctg      816
   Val Arg Pro Gly Thr Tyr Asn Glu Thr Ile Ser His Tyr Asn Val Leu
               260                 265                 270
   tcc aca ttg gag cag atc tac gga ctg ccc aag acg ggt tat gcg acc      864
   Ser Thr Leu Glu Gln Ile Tyr Gly Leu Pro Lys Thr Gly Tyr Ala Thr
           275                 280                 285
   aat gct ccg cca ata acc gat att tgg ggc gac tag                      900
   Asn Ala Pro Pro Ile Thr Asp Ile Trp Gly Asp
       290                 295
<210> 2
   <211> 299
   <212> PRT
   <213> Mycobacterium bovis
<400> 2
   Met Leu Arg Gly Ile Gln Ala Leu Ser Arg Pro Leu Thr Arg Val Tyr
   1               5                   10                  15
   Arg Ala Leu Ala Val Ile Gly Val Leu Ala Ala Ser Leu Leu Ala Ser
               20                  25                  30
   Trp Val Gly Ala Val Pro Gln Val Gly Leu Ala Ala Ser Ala Leu Pro
           35                  40                  45
   Thr Phe Ala His Val Val Ile Val Val Glu Glu Asn Arg Ser Gln Ala
           50                  55                  60
   Ala Ile Ile Gly Asn Lys Ser Ala Pro Phe Ile Asn Ser Leu Ala Ala
   65                  70                  75                  80
   Asn Gly Ala Met Met Ala Gln Ala Phe Ala Glu Thr His Pro Ser Glu
                   85                  90                  95
   Pro Asn Tyr Leu Ala Leu Phe Ala Gly Asn Thr Phe Gly Leu Thr Lys
               100                 105                 110
   Asn Thr Cys Pro Val Asn Gly Gly Ala Leu Pro Asn Leu Gly Ser Glu
           115                 120                 125
   Leu Leu Ser Ala Gly Tyr Thr Phe Met Gly Phe Ala Glu Asp Leu Pro
       130                 135                 140
   Ala Val Gly Ser Thr Val Cys Ser Ala Gly Lys Tyr Ala Arg Lys His
   145                 150                 155                 160
   Val Pro Trp Val Asn Phe Ser Asn Val Pro Ala Thr Leu Ser Val Pro
                   165                 170                 175
   Phe Ser Ala Phe Pro Lys Pro Gln Asn Tyr Pro Gly Leu Pro Thr Val
               180                 185                 190
   Ser Phe Val Ile Pro Asn Ala Asp Asn Asp Met His Asp Gly Ser Ile
           195                 200                 205
   Ala Gln Gly Asp Ala Trp Leu Asn Arg His Leu Ser Ala Tyr Ala Asn
       210                 215                 220
   Trp Ala Lys Thr Asn Asn Ser Leu Leu Val Val Thr Trp Asp Glu Asp
   225                 230                 235                 240
   Asp Gly Ser Ser Arg Asn Gln Ile Pro Thr Val Phe Tyr Gly Ala His
                   245                 250                 255
   Val Arg Pro Gly Thr Tyr Asn Glu Thr Ile Ser His Tyr Asn Val Leu
               260                 265                 270
   Ser Thr Leu Glu Gln Ile Tyr Gly Leu Pro Lys Thr Gly Tyr Ala Thr
           275                 280                 285
   Asn Ala Pro Pro Ile Thr Asp Ile Trp Gly Asp
       290                 295
<210> 3
   <211> 1671
   <212> DNA
   <213> Mycobacterium bovis
<220>
   <221> CDS
   <222> (1)..(1671)
<400> 3
   atg cat gcg agt cgt ccc ggc gca ccg ccg cac gcc ggg ctg ccc agt       48
   Met His Ala Ser Arg Pro Gly Ala Pro Pro His Ala Gly Leu Pro Ser
   1               5                   10                  15
   cgc cga acc gcc ggt gac cag gat cat cgc gcg gac cct aag gtg acc       96
   Arg Arg Thr Ala Gly Asp Gln Asp His Arg Ala Asp Pro Lys Val Thr
               20                  25                  30
   cgc atc atg tcc gcc tcc act ctg gag cag ccc gcg gca gca cac gtc      144
   Arg Ile Met Ser Ala Ser Thr Leu Glu Gln Pro Ala Ala Ala His Val
           35                  40                  45
   gac gag ttg gtg gcg cgg atg cgc ggc cgg ctg ctc gac ccg ctg gcg      192
   Asp Glu Leu Val Ala Arg Met Arg Gly Arg Leu Leu Asp Pro Leu Ala
       50                  55                  60
   att gca gtg ctg gcc gcg gtc atc agc ggc gcc tgg gca agc agg cca      240
   Ile Ala Val Leu Ala Ala Val Ile Ser Gly Ala Trp Ala Ser Arg Pro
   65                  70                  75                  80
   tcg ttg tgg ttc gac gag ggg gca acg atc tcg gct tca gcc agc cgg      288
   Ser Leu Trp Phe Asp Glu Gly Ala Thr Ile Ser Ala Ser Ala Ser Arg
                   85                  90                  95
   aca ttg cca gag cta tgg agt ctg ctg ggc cat atc gac gcc gtg cac      336
   Thr Leu Pro Glu Leu Trp Ser Leu Leu Gly His Ile Asp Ala Val His
               100                 105                 110
   ggc ctg tac tac ctg ttg atg cat ggc tgg ttc gcc ata ttt ccg ccc      384
   Gly Leu Tyr Tyr Leu Leu Met His Gly Trp Phe Ala Ile Phe Pro Pro
           115                 120                 125
   acc gaa tta tgg tcg cgg ctt ccc agc tgc ctg gcc att gga gcg gcc      432
   Thr Glu Leu Trp Ser Arg Leu Pro Ser Cys Leu Ala Ile Gly Ala Ala
       130                 135                 140
   gcc gcc ggc gtg gtg gtt ttt gcc aaa cag ttt tcg gga cgc acc acg      480
   Ala Ala Gly Val Val Val Phe Ala Lys Gln Phe Ser Gly Arg Thr Thr
   145                 150                 155                 160
   gcg gtg tgt gcg gga gcc gtg ttc gcg att ctg ccc agg gtg acg tgg      528
   Ala Val Cys Ala Gly Ala Val Phe Ala Ile Leu Pro Arg Val Thr Trp
                   165                 170                 175
   gcc gga atc gaa gca cgc tcc tcc gcg ctg tcg gta gca gcc gcc gtc      576
   Ala Gly Ile Glu Ala Arg Ser Ser Ala Leu Ser Val Ala Ala Ala Val
               180                 185                 190
   tgg ctg acc gta tta ctc gtg gcc gcg gtg cgg tgc aac acc cag cgg      624
   Trp Leu Thr Val Leu Leu Val Ala Ala Val Arg Cys Asn Thr Gln Arg
           195                 200                 205
   cgg tgg ctg ctc tac gcg ctg gtt ttg atg ctg tcg atc ttg gtc agt      672
   Arg Trp Leu Leu Tyr Ala Leu Val Leu Met Leu Ser Ile Leu Val Ser
       210                 215                 220
   atc aac ctg gcc ctg ttg gta ccg gcc tat gcg acg atg gtg ccg ctg      720
   Ile Asn Leu Ala Leu Leu Val Pro Ala Tyr Ala Thr Met Val Pro Leu
   225                 230                 235                 240
   ctg gcg tcc ggg aaa tca cgc aaa tct ccc gtg atc tgg tgg acg gtc      768
   Leu Ala Ser Gly Lys Ser Arg Lys Ser Pro Val Ile Trp Trp Thr Val
                   245                 250                 255
   gtc acg gca gcc gcg ctc ggg gcc atg aca ccg ttc ata ctg ttc gcc      816
   Val Thr Ala Ala Ala Leu Gly Ala Met Thr Pro Phe Ile Leu Phe Ala
               260                 265                 270
   cac ggc cag gtt tgg cag gtc ggg tgg atc gca ggg ttg aac aga aac      864
   His Gly Gln Val Trp Gln Val Gly Trp Ile Ala Gly Leu Asn Arg Asn
           275                 280                 285
   atc att ctc gac gtc ata cac cgc cag tat ttc gat cac agt gtt ccg      912
   Ile Ile Leu Asp Val Ile His Arg Gln Tyr Phe Asp His Ser Val Pro
       290                 295                 300
   ttc gcc atc ctc gcg ggc ctc atc gtc gct gcc ggc atc gcg gcg cat      960
   Phe Ala Ile Leu Ala Gly Leu Ile Val Ala Ala Gly Ile Ala Ala His
   305                 310                 315                 320
   ctg gcc gga gct cgt gga ccc ggt ggc gat acc cac cgg ctc gtg ctc     1008
   Leu Ala Gly Ala Arg Gly Pro Gly Gly Asp Thr His Arg Leu Val Leu
                   325                 330                 335
   gtc agc gca gcc tgg atc gtc gtg ccc acc gcc gtc gtc ctc atc tac     1056
   Val Ser Ala Ala Trp Ile Val Val Pro Thr Ala Val Val Leu Ile Tyr
               340                 345                 350
   tcg gcg acc gtc gaa ccg atc tac tac ccg cgc tac ctg atc ctc acc     1104
   Ser Ala Thr Val Glu Pro Ile Tyr Tyr Pro Arg Tyr Leu Ile Leu Thr
           355                 360                 365
   gcc ccc gcc gcg gcc gtc atc ctg gcg gtt tgc gtc gtc acc atc gcc     1152
   Ala Pro Ala Ala Ala Val Ile Leu Ala Val Cys Val Val Thr Ile Ala
       370                 375                 380
   cgc aag ccg tgg ctc atc gcc ggg gtc gtg ttt ctc ctt gcc gcc gca     1200
   Arg Lys Pro Trp Leu Ile Ala Gly Val Val Phe Leu Leu Ala Ala Ala
   385                 390                 395                 400
   gcg ttt ccg aac tac ttc ttc aca cag cgg ggg ccg tac gcg aaa gag     1248
   Ala Phe Pro Asn Tyr Phe Phe Thr Gln Arg Gly Pro Tyr Ala Lys Glu
                   405                 410                 415
   ggc tgg gat tac agc cag gtg gca gat gtc atc agc gcc cat gcc aag     1296
   Gly Trp Asp Tyr Ser Gln Val Ala Asp Val Ile Ser Ala His Ala Lys
               420                 425                 430
   ccc ggg gat tgc ctg ctg gtg gac aac acc gcg ggt tgg cga ccc ggg     1344
   Pro Gly Asp Cys Leu Leu Val Asp Asn Thr Ala Gly Trp Arg Pro Gly
           435                 440                 445
   ccc atc cgc gcc ctg ctg gcc acc cgg ccg gcg gcg ttc cgg tcg ctg     1392
   Pro Ile Arg Ala Leu Leu Ala Thr Arg Pro Ala Ala Phe Arg Ser Leu
       450                 455                 460
   att gac gtc gag cgc ggc acc tac ggc ccc aag gtc ggc act ttg tgg     1440
   Ile Asp Val Glu Arg Gly Thr Tyr Gly Pro Lys Val Gly Thr Leu Trp
   465                 470                 475                 480
   gat ggc cat gtc gct gtg tgg ctt acg acg gcc aag atc gac aag tgc     1488
   Asp Gly His Val Ala Val Trp Leu Thr Thr Ala Lys Ile Asp Lys Cys
                   485                 490                 495
   ccc acg ctg tgg acg ata gcc aat cgt gac aag tcg ttg ccc gat cat      1536
   Pro Thr Leu Trp Thr Ile Ala Asn Arg Asp Lys Ser Leu Pro Asp His
               500                 505                 510
   cag gtc ggc gaa atg ttg tca ccg gga aca ggc ttc ggg cgc acg ccc      1584
   Gln Val Gly Glu Met Leu Ser Pro Gly Thr Gly Phe Gly Arg Thr Pro
           515                 520                 525
   gta tac cgg ttc ccg agc tac ctc ggc ttc cgc atc gtc gag cgc tgg      1632
   Val Tyr Arg Phe Pro Ser Tyr Leu Gly Phe Arg Ile Val Glu Arg Trp
       530                 535                 540
   cag ttc cac tac tcg cag gtg gtc aag tca acg cgg taa                  1671
   Gln Phe His Tyr Ser Gln Val Val Lys Ser Thr Arg
   545                 550                 555
<210> 4
   <211> 556
   <212> PRT
   <213> Mycobacterium bovis
<400> 4
   Met His Ala Ser Arg Pro Gly Ala Pro Pro His Ala Gly Leu Pro Ser
   1               5                   10                  15
   Arg Arg Thr Ala Gly Asp Gln Asp His Arg Ala Asp Pro Lys Val Thr
               20                  25                  30
   Arg Ile Met Ser Ala Ser Thr Leu Glu Gln Pro Ala Ala Ala His Val
           35                  40                  45
   Asp Glu Leu Val Ala Arg Met Arg Gly Arg Leu Leu Asp Pro Leu Ala
       50                  55                  60
   Ile Ala Val Leu Ala Ala Val Ile Ser Gly Ala Trp Ala Ser Arg Pro
   65                  70                  75                  80
   Ser Leu Trp Phe Asp Glu Gly Ala Thr Ile Ser Ala Ser Ala Ser Arg
                   85                  90                  95
   Thr Leu Pro Glu Leu Trp Ser Leu Leu Gly His Ile Asp Ala Val His
               100                 105                 110
   Gly Leu Tyr Tyr Leu Leu Met His Gly Trp Phe Ala Ile Phe Pro Pro
           115                 120                 125
   Thr Glu Leu Trp Ser Arg Leu Pro Ser Cys Leu Ala Ile Gly Ala Ala
       130                 135                 140
   Ala Ala Gly Val Val Val Phe Ala Lys Gln Phe Ser Gly Arg Thr Thr
   145                 150                 155                 160
   Ala Val Cys Ala Gly Ala Val Phe Ala Ile Leu Pro Arg Val Thr Trp
                   165                 170                 175
   Ala Gly Ile Glu Ala Arg Ser Ser Ala Leu Ser Val Ala Ala Ala Val
               180                 185                 190
   Trp Leu Thr Val Leu Leu Val Ala Ala Val Arg Cys Asn Thr Gln Arg
           195                 200                 205
   Arg Trp Leu Leu Tyr Ala Leu Val Leu Met Leu Ser Ile Leu Val Ser
       210                 215                 220
   Ile Asn Leu Ala Leu Leu Val Pro Ala Tyr Ala Thr Met Val Pro Leu
   225                 230                 235                 240
   Leu Ala Ser Gly Lys Ser Arg Lys Ser Pro Val Ile Trp Trp Thr Val
                   245                 250                 255
   Val Thr Ala Ala Ala Leu Gly Ala Met Thr Pro Phe Ile Leu Phe Ala
               260                 265                 270
   His Gly Gln Val Trp Gln Val Gly Trp Ile Ala Gly Leu Asn Arg Asn
           275                 280                 285
   Ile Ile Leu Asp Val Ile His Arg Gln Tyr Phe Asp His Ser Val Pro
       290                 295                 300
   Phe Ala Ile Leu Ala Gly Leu Ile Val Ala Ala Gly Ile Ala Ala His
   305                 310                 315                 320
   Leu Ala Gly Ala Arg Gly Pro Gly Gly Asp Thr His Arg Leu Val Leu
                   325                 330                 335
   Val Ser Ala Ala Trp Ile Val Val Pro Thr Ala Val Val Leu Ile Tyr
               340                 345                 350
   Ser Ala Thr Val Glu Pro Ile Tyr Tyr Pro Arg Tyr Leu Ile Leu Thr
           355                 360                 365
   Ala Pro Ala Ala Ala Val Ile Leu Ala Val Cys Val Val Thr Ile Ala
       370                 375                 380
   Arg Lys Pro Trp Leu Ile Ala Gly Val Val Phe Leu Leu Ala Ala Ala
   385                 390                 395                 400
   Ala Phe Pro Asn Tyr Phe Phe Thr Gln Arg Gly Pro Tyr Ala Lys Glu
                   405                 410                 415
   Gly Trp Asp Tyr Ser Gln Val Ala Asp Val Ile Ser Ala His Ala Lys
               420                 425                 430
   Pro Gly Asp Cys Leu Leu Val Asp Asn Thr Ala Gly Trp Arg Pro Gly
           435                 440                 445
   Pro Ile Arg Ala Leu Leu Ala Thr Arg Pro Ala Ala Phe Arg Ser Leu
       450                 455                 460
   Ile Asp Val Glu Arg Gly Thr Tyr Gly Pro Lys Val Gly Thr Leu Trp
   465                 470                 475                 480
   Asp Gly His Val Ala Val Trp Leu Thr Thr Ala Lys Ile Asp Lys Cys
                   485                 490                 495
   Pro Thr Leu Trp Thr Ile Ala Asn Arg Asp Lys Ser Leu Pro Asp His
               500                 505                 510
   Gln Val Gly Glu Met Leu Ser Pro Gly Thr Gly Phe Gly Arg Thr Pro
           515                 520                 525
   Val Tyr Arg Phe Pro Ser Tyr Leu Gly Phe Arg Ile Val Glu Arg Trp
       530                 535                 540
   Gln Phe His Tyr Ser Gln Val Val Lys Ser Thr Arg
   545                 550                 555
<210> 5
   <211> 1284
   <212> DNA
   <213> Mycobacterium bovis
<220>
   <221> CDS
   <222> (1)..(1284)
<400> 5
   atg agt gca tgg cgg gcg ccc gag gtg ggc agt cga ctc ggg cgg agg      48
   Met Ser Ala Trp Arg Ala Pro Glu Val Gly Ser Arg Leu Gly Arg Arg
   1               5                   10                  15
   gtg ttg tgg tgc ctg ctg tgg ctg ctg gcc ggc gtg gcg ttg ggc tac      96
   Val Leu Trp Cys Leu Leu Trp Leu Leu Ala Gly Val Ala Leu Gly Tyr
               20                  25                  30
   gtg gcc tgg cgg ttg ttc ggc cac acg ccg tat cgc atc gat atc gac     144
   Val Ala Trp Arg Leu Phe Gly His Thr Pro Tyr Arg Ile Asp Ile Asp
           35                  40                  45
   atc tat cag atg ggc gct cga gct tgg ctg gac ggg cgt ccg ctg tat     192
   Ile Tyr Gln Met Gly Ala Arg Ala Trp Leu Asp Gly Arg Pro Leu Tyr
       50                  55                  60
   ggc ggt ggt gtg ttg ttc cac aca ccc atc ggg ctg aac ctc ccg ttc     240
   Gly Gly Gly Val Leu Phe His Thr Pro Ile Gly Leu Asn Leu Pro Phe
   65                  70                  75                  80
   acc tat cct cca ctg gcg gcc gtc ctg ttc agc cca ttc gcc tgg ttg      288
   Thr Tyr Pro Pro Leu Ala Ala Val Leu Phe Ser Pro Phe Ala Trp Leu
                   85                  90                  95
   cag atg ccg gct gcc agc gtc gcg atc acg gtg cta acc ctg gtg ctg      336
   Gln Met Pro Ala Ala Ser Val Ala Ile Thr Val Leu Thr Leu Val Leu
               100                 105                 110
   ctg atc gcg tcg acg gcg atc gtg ctg acc ggc ctc gac gca tgg cca      384
   Leu Ile Ala Ser Thr Ala Ile Val Leu Thr Gly Leu Asp Ala Trp Pro
           115                 120                 125
   acc tcc cga ctg gta ccc gcg ccg gct cgg tta cgc cgg ttg tgg ttg      432
   Thr Ser Arg Leu Val Pro Ala Pro Ala Arg Leu Arg Arg Leu Trp Leu
       130                 135                 140
   gcc gtg ctc atc gtg gct ccg gca acg att tgg ctg gag ccg atc agc      480
   Ala Val Leu Ile Val Ala Pro Ala Thr Ile Trp Leu Glu Pro Ile Ser
   145                 150                 155                 160
   tcg aac ttc gct ttc ggt cag atc aat gtg gtg ctg atg acc ctg gtg      528
   Ser Asn Phe Ala Phe Gly Gln Ile Asn Val Val Leu Met Thr Leu Val
                   165                 170                 175
   atc gtc gac tgc ttc cca cgc cga acg cca tgg cca cgc ggg ctg atg      576
   Ile Val Asp Cys Phe Pro Arg Arg Thr Pro Trp Pro Arg Gly Leu Met
               180                 185                 190
   ttg ggg ctg ggg ata gcc ctc aaa ctc acc ccc gcg gtg ttt ctc ctc      624
   Leu Gly Leu Gly Ile Ala Leu Lys Leu Thr Pro Ala Val Phe Leu Leu
           195                 200                 205
   tac ttc ctg cta cgt cgg gac ggt cgg gcc gcg ctg acg gcg ctg gcg      672
   Tyr Phe Leu Leu Arg Arg Asp Gly Arg Ala Ala Leu Thr Ala Leu Ala
       210                 215                 220
   tcg ttc gcg gtc gcc acg ctg ctc ggt ttc gtc ctg gcg tgg cgc gac      720
   Ser Phe Ala Val Ala Thr Leu Leu Gly Phe Val Leu Ala Trp Arg Asp
   225                 230                 235                 240
   tcc tgg gag tac tgg acg cat acc ctt cac cac acg gac cgg atc ggc      768
   Ser Trp Glu Tyr Trp Thr His Thr Leu His His Thr Asp Arg Ile Gly
                   245                 250                 255
   gct gcc gcc ttg aac aca gac cag aac atc gcg ggc gca ctc gcg cgg      816
   Ala Ala Ala Leu Asn Thr Asp Gln Asn Ile Ala Gly Ala Leu Ala Arg
               260                 265                 270
   ttg acg att ggc gat gac gaa cgc ttc gca ctg tgg gtg gcc gga tcc      864
   Leu Thr Ile Gly Asp Asp Glu Arg Phe Ala Leu Trp Val Ala Gly Ser
           275                 280                 285
   ctg ctc gtg ttg gca gcg acc ata tgg gcg atg cgg cga gtg ttg cgg      912
   Leu Leu Val Leu Ala Ala Thr Ile Trp Ala Met Arg Arg Val Leu Arg
       290                 295                 300
   gcc ggc gag ccg acc ctg gct gtg atc tgc gtc gcc ctg ttc ggg ttg      960
   Ala Gly Glu Pro Thr Leu Ala Val Ile Cys Val Ala Leu Phe Gly Leu
   305                 310                 315                 320
   gta gtt tcg ccg gtc tcg tgg tca cac cat tgg gtg tgg atg ctg ccg     1008
   Val Val Ser Pro Val Ser Trp Ser His His Trp Val Trp Met Leu Pro
                   325                 330                 335
   gcc gtg ctg gtg att ggg cta ctg ggt tgg cgt cgc cgc aac gtc gcg      1056
   Ala Val Leu Val Ile Gly Leu Leu Gly Trp Arg Arg Arg Asn Val Ala
               340                 345                 350
   ttg gcc atg ctc agc ctg gcc ggg gtg gtg ctg atg agg tgg aca ccg      1104
   Leu Ala Met Leu Ser Leu Ala Gly Val Val Leu Met Arg Trp Thr Pro
           355                 360                 365
   atc gac ctg ctt ccc caa cac cgg gag acg act gcg gtc tgg tgg cgt      1152
   Ile Asp Leu Leu Pro Gln His Arg Glu Thr Thr Ala Val Trp Trp Arg
       370                 375                 380
   caa ctc gcg ggg atg tcc tac gtg tgg tgg gcg ctg gcg gtc atc gtc      1200
   Gln Leu Ala Gly Met Ser Tyr Val Trp Trp Ala Leu Ala Val Ile Val
   385                 390                 395                 400
   gtt gcc gga ctc acc gtt acc gcc agg atg acg ccg cag cgc tcg ctt      1248
   Val Ala Gly Leu Thr Val Thr Ala Arg Met Thr Pro Gln Arg Ser Leu
                   405                 410                 415
   acg cgc gga ctg acc ccg gcg ccg acg gcc agc tga                      1284
   Thr Arg Gly Leu Thr Pro Ala Pro Thr Ala Ser
               420                 425
<210> 6
   <211> 427
   <212> PRT
   <213> Mycobacterium bovis
<400> 6
   Met Ser Ala Trp Arg Ala Pro Glu Val Gly Ser Arg Leu Gly Arg Arg
   1               5                   10                  15
   Val Leu Trp Cys Leu Leu Trp Leu Leu Ala Gly Val Ala Leu Gly Tyr
               20                  25                  30
   Val Ala Trp Arg Leu Phe Gly His Thr Pro Tyr Arg Ile Asp Ile Asp
           35                  40                  45
   Ile Tyr Gln Met Gly Ala Arg Ala Trp Leu Asp Gly Arg Pro Leu Tyr
       50                  55                  60
   Gly Gly Gly Val Leu Phe His Thr Pro Ile Gly Leu Asn Leu Pro Phe
   65                  70                  75                  80
   Thr Tyr Pro Pro Leu Ala Ala Val Leu Phe Ser Pro Phe Ala Trp Leu
                   85                  90                  95
   Gln Met Pro Ala Ala Ser Val Ala Ile Thr Val Leu Thr Leu Val Leu
               100                 105                 110
   Leu Ile Ala Ser Thr Ala Ile Val Leu Thr Gly Leu Asp Ala Trp Pro
           115                 120                 125
   Thr Ser Arg Leu Val Pro Ala Pro Ala Arg Leu Arg Arg Leu Trp Leu
       130                 135                 140
   Ala Val Leu Ile Val Ala Pro Ala Thr Ile Trp Leu Glu Pro Ile Ser
   145                 150                 155                 160
   Ser Asn Phe Ala Phe Gly Gln Ile Asn Val Val Leu Met Thr Leu Val
                   165                 170                 175
   Ile Val Asp Cys Phe Pro Arg Arg Thr Pro Trp Pro Arg Gly Leu Met
               180                 185                 190
   Leu Gly Leu Gly Ile Ala Leu Lys Leu Thr Pro Ala Val Phe Leu Leu
           195                 200                 205
   Tyr Phe Leu Leu Arg Arg Asp Gly Arg Ala Ala Leu Thr Ala Leu Ala
       210                 215                 220
   Ser Phe Ala Val Ala Thr Leu Leu Gly Phe Val Leu Ala Trp Arg Asp
   225                 230                 235                 240
   Ser Trp Glu Tyr Trp Thr His Thr Leu His His Thr Asp Arg Ile Gly
                   245                 250                 255
   Ala Ala Ala Leu Asn Thr Asp Gln Asn Ile Ala Gly Ala Leu Ala Arg
               260                 265                 270
   Leu Thr Ile Gly Asp Asp Glu Arg Phe Ala Leu Trp Val Ala Gly Ser
           275                 280                 285
   Leu Leu Val Leu Ala Ala Thr Ile Trp Ala Met Arg Arg Val Leu Arg
       290                 295                 300
   Ala Gly Glu Pro Thr Leu Ala Val Ile Cys Val Ala Leu Phe Gly Leu
   305                 310                 315                 320
   Val Val Ser Pro Val Ser Trp Ser His His Trp Val Trp Met Leu Pro
                   325                 330                 335
   Ala Val Leu Val Ile Gly Leu Leu Gly Trp Arg Arg Arg Asn Val Ala
               340                 345                 350
   Leu Ala Met Leu Ser Leu Ala Gly Val Val Leu Met Arg Trp Thr Pro
           355                 360                 365
   Ile Asp Leu Leu Pro Gln His Arg Glu Thr Thr Ala Val Trp Trp Arg
       370                 375                 380
   Gln Leu Ala Gly Met Ser Tyr Val Trp Trp Ala Leu Ala Val Ile Val
   385                 390                 395                 400
   Val Ala Gly Leu Thr Val Thr Ala Arg Met Thr Pro Gln Arg Ser Leu
                   405                 410                 415
   Thr Arg Gly Leu Thr Pro Ala Pro Thr Ala Ser
               420                 425
<210> 7
   <211> 1551
   <212> DNA
   <213> Mycobacterium bovis
<220>
   <221> CDS
   <222> (1)..(1551)
<400> 7
   atg act act ccg agc cat gct cca gcg gtt gat ttg gct aca gcg aaa      48
   Met Thr Thr Pro Ser His Ala Pro Ala Val Asp Leu Ala Thr Ala Lys
   1               5                   10                  15
   gat gct gtt gtc caa cac ctt tcg cga ctt ttc gag ttc act acc ggt      96
   Asp Ala Val Val Gln His Leu Ser Arg Leu Phe Glu Phe Thr Thr Gly
               20                  25                  30
   ccg cag ggc gga ccg gcg cgg ctg ggc ttc gcc ggc gcg gtg ctg atc     144
   Pro Gln Gly Gly Pro Ala Arg Leu Gly Phe Ala Gly Ala Val Leu Ile
           35                  40                  45
   acc gca ggc ggg ctg gga gcc ggc agc gtc cgc caa cat gac ccg ctg     192
   Thr Ala Gly Gly Leu Gly Ala Gly Ser Val Arg Gln His Asp Pro Leu
       50                  55                  60
   ctg gag tcg att cac atg tcc tgg ctg cgc ttc ggc cac gga ctc gtg     240
   Leu Glu Ser Ile His Met Ser Trp Leu Arg Phe Gly His Gly Leu Val
   65                  70                  75                  80
   ctg tcg tcg att ctg ttg tgg aca ggt gtg ggt gtg atg ctg ctt gcg     288
   Leu Ser Ser Ile Leu Leu Trp Thr Gly Val Gly Val Met Leu Leu Ala
                   85                  90                  95
   tgg ctg ggt cta ggc cga cgg gtc ctc gcc ggc gaa gcc acc gag ttc     336
   Trp Leu Gly Leu Gly Arg Arg Val Leu Ala Gly Glu Ala Thr Glu Phe
               100                 105                 110
   acc atg cgg gca acc acc gtt atc tgg ctg gcg ccg cta ctg ctg tcg     384
   Thr Met Arg Ala Thr Thr Val Ile Trp Leu Ala Pro Leu Leu Leu Ser
           115                 120                 125
   gtg ccc gtc ttc agc cgg gac act tac tcg tat ctg gcc caa ggg gcg     432
   Val Pro Val Phe Ser Arg Asp Thr Tyr Ser Tyr Leu Ala Gln Gly Ala
       130                 135                 140
   ctt ctg cgc gac ggt ctg gat cct tac gct gtt ggc ccg gtc ggt aat     480
   Leu Leu Arg Asp Gly Leu Asp Pro Tyr Ala Val Gly Pro Val Gly Asn
   145                 150                 155                 160
   ccc aat gcg ctg ctg gac gac gta agc ccg atc tgg acg atc acc acc     528
   Pro Asn Ala Leu Leu Asp Asp Val Ser Pro Ile Trp Thr Ile Thr Thr
                   165                 170                 175
   gcg ccc tac ggt cct gcg ttc att ctg gtt gcg aag ttc gtc acg gta      576
   Ala Pro Tyr Gly Pro Ala Phe Ile Leu Val Ala Lys Phe Val Thr Val
               180                 185                 190
   atc gtc ggc aac aat gtc gtc gcc gga acc atg ctg ttg cgt ttg tgc      624
   Ile Val Gly Asn Asn Val Val Ala Gly Thr Met Leu Leu Arg Leu Cys
           195                 200                 205
   atg ctg ccc ggg ctg gcg ttg ctg gtc tgg gcc act cca cgc ttg gcc      672
   Met Leu Pro Gly Leu Ala Leu Leu Val Trp Ala Thr Pro Arg Leu Ala
       210                 215                 220
   agc cat ctc ggc acc cac ggc ccg acc gcg ctg tgg atc tgc gtg ctg      720
   Ser His Leu Gly Thr His Gly Pro Thr Ala Leu Trp Ile Cys Val Leu
   225                 230                 235                 240
   aac cca ctg gtc ctc atc cat ctg atg ggc ggg gtg cac aac gag atg      768
   Asn Pro Leu Val Leu Ile His Leu Met Gly Gly Val His Asn Glu Met
                   245                 250                 255
   ctg atg gtg ggt ctg atg acc gcc ggt atc gcg ttg acc gtc cag ggc      816
   Leu Met Val Gly Leu Met Thr Ala Gly Ile Ala Leu Thr Val Gln Gly
               260                 265                 270
   cgt aat gtc gcg ggg atc atc ctg atc acc gtt gcg atc gcg gtg aag      864
   Arg Asn Val Ala Gly Ile Ile Leu Ile Thr Val Ala Ile Ala Val Lys
           275                 280                 285
   gcc acc gcc gga atc gcg ttg ccc ttc ttg gtc tgg gtt tgg ctg cgt      912
   Ala Thr Ala Gly Ile Ala Leu Pro Phe Leu Val Trp Val Trp Leu Arg
       290                 295                 300
   cat ctg cgt gag cga cgg ggg tac cgg ccg gtc cag gcg ttc ctg gca      960
   His Leu Arg Glu Arg Arg Gly Tyr Arg Pro Val Gln Ala Phe Leu Ala
   305                 310                 315                 320
   gcc gcc gcg ata tcg ctg ctg atc ttc gtc gcg gtg ttc gcg gtg ctg     1008
   Ala Ala Ala Ile Ser Leu Leu Ile Phe Val Ala Val Phe Ala Val Leu
                   325                 330                 335
   tct gcg gta gcc ggc gtt ggc cta ggg tgg ctg acc gcg ctg gcc ggc     1056
   Ser Ala Val Ala Gly Val Gly Leu Gly Trp Leu Thr Ala Leu Ala Gly
               340                 345                 350
   tcg gtg aaa atc atc aac tgg ctg acg gtg ccc acc ggg gcg gcc aac     1104
   Ser Val Lys Ile Ile Asn Trp Leu Thr Val Pro Thr Gly Ala Ala Asn
           355                 360                 365
   gtg atc cac gcg ctg ggc aga ggg ctc ttc acg gtc gac ttc tac acc     1152
   Val Ile His Ala Leu Gly Arg Gly Leu Phe Thr Val Asp Phe Tyr Thr
       370                 375                 380
   ttg ctg cgg atc acc cgg ctg atc gga atc gtg atc atc gcg gtg tcg     1200
   Leu Leu Arg Ile Thr Arg Leu Ile Gly Ile Val Ile Ile Ala Val Ser
   385                 390                 395                 400
   ctg ccg ctg ttg tgg tgg cgg ttc cgg cgc gac gac cgg gcc gcg ctg     1248
   Leu Pro Leu Leu Trp Trp Arg Phe Arg Arg Asp Asp Arg Ala Ala Leu
                   405                 410                 415
   acc ggg gtc gca tgg tcg atg ctg atc gtg gtg ctg ttc gta ccc gcc     1296
   Thr Gly Val Ala Trp Ser Met Leu Ile Val Val Leu Phe Val Pro Ala
               420                 425                 430
   gcc ctg ccg tgg tac tac tcc tgg ccg ctg gcg gtc gct gcc ccg ttg      1344
   Ala Leu Pro Trp Tyr Tyr Ser Trp Pro Leu Ala Val Ala Ala Pro Leu
           435                 440                 445
   gcc cag tca cga cgg gcg atc gcg gcc atc gcc ggg ctc tcg act tgg      1392
   Ala Gln Ser Arg Arg Ala Ile Ala Ala Ile Ala Gly Leu Ser Thr Trp
       450                 455                 460
   gtg atg gtg atc ttc aaa ccc gac gga tcg cac ggg atg tat tcg tgg      1440
   Val Met Val Ile Phe Lys Pro Asp Gly Ser His Gly Met Tyr Ser Trp
   465                 470                 475                 480
   ctg cac ttc tgg atc gcc acc gcc tgc gca ctg act gcg tgg tat gtc      1488
   Leu His Phe Trp Ile Ala Thr Ala Cys Ala Leu Thr Ala Trp Tyr Val
                   485                 490                 495
   ctg tat cgg tca ccg gac cgg cgc gga gtg cag gct gca acc ccg gtg      1536
   Leu Tyr Arg Ser Pro Asp Arg Arg Gly Val Gln Ala Ala Thr Pro Val
               500                 505                 510
   gtc aat acg cca tag                                                  1551
   Val Asn Thr Pro
           515
<210> 8
   <211> 516
   <212> PRT
   <213> Mycobacterium bovis
<400> 8
   Met Thr Thr Pro Ser His Ala Pro Ala Val Asp Leu Ala Thr Ala Lys
   1               5                   10                  15
   Asp Ala Val Val Gln His Leu Ser Arg Leu Phe Glu Phe Thr Thr Gly
               20                  25                  30
   Pro Gln Gly Gly Pro Ala Arg Leu Gly Phe Ala Gly Ala Val Leu Ile
           35                  40                  45
   Thr Ala Gly Gly Leu Gly Ala Gly Ser Val Arg Gln His Asp Pro Leu
       50                  55                  60
   Leu Glu Ser Ile His Met Ser Trp Leu Arg Phe Gly His Gly Leu Val
   65                  70                  75                  80
   Leu Ser Ser Ile Leu Leu Trp Thr Gly Val Gly Val Met Leu Leu Ala
                   85                  90                  95
   Trp Leu Gly Leu Gly Arg Arg Val Leu Ala Gly Glu Ala Thr Glu Phe
               100                 105                 110
   Thr Met Arg Ala Thr Thr Val Ile Trp Leu Ala Pro Leu Leu Leu Ser
           115                 120                 125
   Val Pro Val Phe Ser Arg Asp Thr Tyr Ser Tyr Leu Ala Gln Gly Ala
       130                 135                 140
   Leu Leu Arg Asp Gly Leu Asp Pro Tyr Ala Val Gly Pro Val Gly Asn
   145                 150                 155                 160
   Pro Asn Ala Leu Leu Asp Asp Val Ser Pro Ile Trp Thr Ile Thr Thr
                   165                 170                 175
   Ala Pro Tyr Gly Pro Ala Phe Ile Leu Val Ala Lys Phe Val Thr Val
               180                 185                 190
   Ile Val Gly Asn Asn Val Val Ala Gly Thr Met Leu Leu Arg Leu Cys
           195                 200                 205
   Met Leu Pro Gly Leu Ala Leu Leu Val Trp Ala Thr Pro Arg Leu Ala
       210                 215                 220
   Ser His Leu Gly Thr His Gly Pro Thr Ala Leu Trp Ile Cys Val Leu
   225                 230                 235                 240
   Asn Pro Leu Val Leu Ile His Leu Met Gly Gly Val His Asn Glu Met
                   245                 250                 255
   Leu Met Val Gly Leu Met Thr Ala Gly Ile Ala Leu Thr Val Gln Gly
               260                 265                 270
   Arg Asn Val Ala Gly Ile Ile Leu Ile Thr Val Ala Ile Ala Val Lys
           275                 280                 285
   Ala Thr Ala Gly Ile Ala Leu Pro Phe Leu Val Trp Val Trp Leu Arg
       290                 295                 300
   His Leu Arg Glu Arg Arg Gly Tyr Arg Pro Val Gln Ala Phe Leu Ala
   305                 310                 315                 320
   Ala Ala Ala Ile Ser Leu Leu Ile Phe Val Ala Val Phe Ala Val Leu
                   325                 330                 335
   Ser Ala Val Ala Gly Val Gly Leu Gly Trp Leu Thr Ala Leu Ala Gly
               340                 345                 350
   Ser Val Lys Ile Ile Asn Trp Leu Thr Val Pro Thr Gly Ala Ala Asn
           355                 360                 365
   Val Ile His Ala Leu Gly Arg Gly Leu Phe Thr Val Asp Phe Tyr Thr
       370                 375                 380
   Leu Leu Arg Ile Thr Arg Leu Ile Gly Ile Val Ile Ile Ala Val Ser
   385                 390                 395                 400
   Leu Pro Leu Leu Trp Trp Arg Phe Arg Arg Asp Asp Arg Ala Ala Leu
                   405                 410                 415
   Thr Gly Val Ala Trp Ser Met Leu Ile Val Val Leu Phe Val Pro Ala
               420                 425                 430
   Ala Leu Pro Trp Tyr Tyr Ser Trp Pro Leu Ala Val Ala Ala Pro Leu
           435                 440                 445
   Ala Gln Ser Arg Arg Ala Ile Ala Ala Ile Ala Gly Leu Ser Thr Trp
       450                 455                 460
   Val Met Val Ile Phe Lys Pro Asp Gly Ser His Gly Met Tyr Ser Trp
   465                 470                 475                 480
   Leu His Phe Trp Ile Ala Thr Ala Cys Ala Leu Thr Ala Trp Tyr Val
                   485                 490                 495
   Leu Tyr Arg Ser Pro Asp Arg Arg Gly Val Gln Ala Ala Thr Pro Val
               500                 505                 510
   Val Asn Thr Pro
           515
<210> 9
   <211> 22
   <212> DNA
   <213> Artificial
<220>
   <223> oligonucleotide
<400> 9
   gacgtgggcc ggaatcgaag ca 22
<210> 10
   <211> 24
   <212> DNA
   <213> Artificial
<220>
   <223> oligonucleotide
<400> 10
   gatccacccg acctgccaaa cctg 24
<210> 11
   <211> 21
   <212> DNA
   <213> Artificial
<220>
   <223> oligonucleotide
<400> 11
   aacgcttcgc actgtgggtg g 21
<210> 12
   <211> 24
   <212> DNA
   <213> Artificial
<220>
   <223> oligonucleotide
<400> 12
   cgtaggacat ccccgcgagt tgac 24
<210> 13
   <211> 21
   <212> DNA
   <213> Artificial
<220>
   <223> oligonucleotide
<400> 13
   acacacccga gcgaaccgaa c 21
<210> 14
   <211> 24
   <212> DNA
   <213> Artificial
<220>
   <223> oligonucleotide
<400> 14
   caagcggatg ggtacgaggt cagc 24
<210> 15
   <211> 24
   <212> DNA
   <213> Artificial
<220>
   <223> oligonucleotide
<400> 15
   gatgggcggg gtgcacaacg agat 24
<210> 16
   <211> 23
   <212> DNA
   <213> Artificial
<220>
   <223> oligonucleotide
<400> 16
   cacgcagatg acgcagccaa acc 23
<210> 17
   <211> 21
   <212> DNA
   <213> Artificial
<220>
   <223> oligonucleotide
<400> 17
   atgacggcct tcgggttgta a 21
<210> 18
   <211> 20
   <212> DNA
   <213> Artificial
<220>
   <223> oligonucleotide
<400> 18
   cggctgctgg cacgtagttg 20
<210> 19
   <211> 20
   <212> DNA
   <213> Artificial
<220>
   <223> oligonucleotide
<400> 19
   caccacggtc ttataggcgc 20
<210> 20
   <211> 16
   <212> DNA
   <213> Artificial
<220>
   <223> oligonucleotide
<400> 20
   ccgttcacct ccactg 16
<210> 21
   <211> 21
   <212> DNA
   <213> Artificial
<220>
   <223> oligonucleotide
<400> 21
   atcgtggctc ggtcccctaa a 21
<210> 22
   <211> 20
   <212> DNA
   <213> Artificial
<220>
   <223> oligonucleotide
<400> 22
   cggtgacgac ctaaagtcgg 20

## Description

### Field of the invention

The present invention relates to the field of vaccines, most particularly live attenuated *Mycobacterium-*based vaccines. Disclosed herein are genes which, when mutated in mycobacteria used for immunization of animals, particularly mammals, confer improved protective efficacy as a vaccine against a *Mycobacterium* infection, while not interfering with the beneficial properties of known vaccination strains such as *Mycobacterium bovis* BCG. Such vaccines are particularly suitable against *Mycobacterium tuberculosis* infection.

### Background

One-third of the world's population is infected with *Mycobacterium tuberculosis* (*M. tb*), the etiologic agent of TB. The World Health Organization estimates that about eight to ten million new TB cases occur annually worldwide and the incidence of TB is currently increasing. Together with malaria and HIV-AIDS, TB is one of the three leading causes of death from a single infectious agent, and approximately two million deaths are attributable to TB annually (WHO Report, 2008). The only currently licensed vaccine is *Mycobacterium bovis* Bacille Calmette-Guerin (*M. bovis* BCG), an attenuated strain of *M*. *bovis,* which has been administered to over 4 billion people over the years since 1921, when it was first used. When administered at birth, *M*. *bovis* BCG confers consistent and reliable protection against disseminated disease in the first 10 years of life (Rodrigues et al, 1993). However, the protection conferred against pulmonary disease in adolescents and adults is much more variable (Colditz et al, 1994). The main current approach in developing improved prophylactic TB vaccines is to use modified *M. bovis* BCG or *M*. *tb* to improve upon *M*. bovis BCG as a priming vaccine, possibly followed by boosting some time later with selected immunodominant antigens as a protein or viral vector vaccine (Barker et al, 2009; STOP-TBPartnership, 2009).

In the case of tuberculosis, alveolar macrophages successfully phagocytose *M*. *tb* and are the primary site of infection. However, the mycobacteria interfere with phagosomal maturation, thus enabling the mycobacteria to partially evade many of the immune mechanisms that would otherwise eliminate them (50). The bacterial genes and pathways that are essential for the intracellular growth and survival of *M*. *tb* are attractive drug targets and several of such bacterial virulence mechanisms have been suggested (13, 14). However, the functional validation of the bacterial components suspected to be involved in *M*. *tb-* host interactions requires the generation of mutants. These critical experiments are hampered by the slow growth and poor homologous recombination efficiency of *M*. *tb* (39) and by the requirement to work in costly high-biosafety laboratories. The combination of these features makes mutant generation a very lengthy, cumbersome and expensive process. Therefore, many researchers resort to non-pathogenic fast-growing mycobacterial species to generate mutants, with results that are not necessarily to be extrapolated to their slow-growing cousins (24). The attenuated vaccine strain *M*. *bovis* BCG is a more faithful, yet safer model organism for many aspects of *M*. *tb-* host interactions (8, 17). It is also slow-growing and is genomically extremely similar to *M. tuberculosis* (>99%), only lacking a small number of genomic regions, of which the RD1 locus has primarily resulted in its attenuation (2, 33). Each BCG strain has multiple deletions, of which only the RD1 locus is common to all.

It should be noted that the unusual cell wall of Mycobacteria is one of the factors that enable the mycobacteria to successfully colonize their host. The cell wall is composed of a complex of lipids, glycolipids and proteins which interact with macrophages (5). One such cell envelope component is lipoarabinomannan (LAM), which, along with its precursors lipomannan (LM) and phosphatidyl-myo-inositol mannosides (PIMs), is noncovalently anchored to the plasma membrane. LAM contains a mannosylphosphatidyl-myo-inositol anchor (MPI), a mannan core and long, substituted arabinan branches. The mannan backbone consists of α-1,6-linked mannose units with single residue α-1,2-Manp substitutions. The arabinan polymer is composed of α-1,5-linked Ara*f* units and is substituted with branched hexa-arabinofuranosides and linear tetra-arabinofuranosides. The non-reducing termini of these arabinan side-chains terminate in cap motifs. In slow-growing, pathogenic strains, these motifs consist of one, two or three α-1,2-linked Man*p* residues with the dimannoside being the most abundant. In contrast, in non-pathogenic strains, there are usually inositol phosphate caps (PILAM in *M*. *smegmatis*) or no caps at all (AraLAM in *M. chelonae*) (7). ManLAM (mannosylated lipoarabinomannan) has been reported to play a role in subverting host cell signaling pathways (18) and blocking phagosome maturation (15) in host macrophages.

LAM is built from phosphatidyl-*myo*-inositol (PI), the synthesis of which requires the activity of the diacylglycerol kinase Mb2276 (45). PI undergoes a series of sequential α-mannosylations, catalyzed by PimA (Mb2642c), PimB (Mb0572), PimC (Mb1785c) and PimF (Mb1538) (25, 26, 38, 57). The glycosyl donor substrate for all mannosylations in ManLAM synthesis from PI is polyprenol phosphomannose, which is synthesized from GDPmannose by the polyprenol phosphomannose synthase ppm1 (Mb2077c) (19). GDP-mannose synthesis in this process involves the phosphomannose mutase pmmB (Mb3336) (55). LM is synthesized on the PIM4 precursor by the addition of a linear α-1,6-mannose polymer of 21-34 residues. The mannosyltransferase Mb2196 is required for this modification (23). A previous study showed that Rv2181 (Mb2203) is the mannosyltransferase responsible for the addition of single α-1,2-Manp residues to the mannan core of LM/LAM (20). In this latter study, the inactivation of the corresponding homologue in *M. smegmatis* leads to the production of a truncated LAM and the absence of LM. However, more recent data reveal that a similar inactivation in *M. tb* leads to production of lower molecular weight LAM, but does not affect the synthesis of LM, revealing a role for this mannosyltransferase in the terminal capping of LAM (24). LAM is derived from LM through the addition of poly α-1,5-ara*f* chains, the synthesis of which requires embC (4, 65). The transcription of embC is regulated by the serine threonine kinase pknH via the phosphorylation of the transcriptional regulator EmbR (59). The linear arabinan branches are further substituted with hexa- and tetra- arabinofuranoside structures. These non-reducing arabinan termini of the LAM are capped in *M. tuberculosis* and *M. bovis* BCG, to varying degrees, with 1 to 3 (α-1,2)-Man*p* residues. The first mannosyltransferase involved in this α-1,2-mannose capping is encoded by Rv1635c and its homologue in *M. bovis* BCG, Mb 1661c (11). While the gene Mb1661c is likely responsible for the addition of the first mannose residue of the cap, the mannosyltransferase Mb2203 is thought to add the additional one or two mannose residues to generate di- and tri- Manp capped LAM (24). Other factors influencing the virulence of M. *tb* include the lipoprotein signal peptidase, LspA (Mb1566) (53), the lipid phosphatase SapM (Mb3338) (42) and the Zn metalloprotease Zmp1 (Mb0204c) (35). WO2004/033677 imparts that secreted acid phosphatase (sapM) is present only in pathogenic mycobacteria and expressed selectively at phagosomal pH. It discloses that an isolated DNA sequence comprising a promoter or a promoter fragment of a SapM gene is sufficient to control expression of a nucleotide sequence of interest and is inducible under low-pH conditions. Also taught are methods of diagnosing tuberculosis by detecting SapM, and vaccines comprising SapM or an anti-SapM antibody.

Examples of mycobacteria modifications for vaccination purposes include the following:
WO2009/009798 relates to allelic exchange methods to generate directed mutations within genes of slow-growing stains of mycobacteria (e.g., Mycobacterium avium subsp. paratuberculosis (Map). Deletion of gene regions from the genome of virulent slow-growing mycobacteria (such as Map, M. bovis and M. tuberculosis) attenuates the virulence of the mycobacteria without eliminating the ability of the mycobacteria to colonize susceptible mammals. These attenuated mycobacteria are capable of protecting the mammals from challenge by virulent mycobacteria (e.g., Map, M. bovis, M. tuberculosis).

WO2007/084353 discloses mycobacteria comprising (a) a mutation that is not in a SecA2 gene that attenuates the virulence of the mycobacteria in a mammalian host, and (b) a mutation in a SecA2 gene that eliminates SecA2 activity.

WO2006/078164 relates to Mycobacteria in which the lipoarabinomannan cap-specific mannosyl transferases have been inactivated and that therefore express mannose cap-deficient lipoarabinomannan. Such Mycobacteria with mannose cap-deficient lipoarabinomannan may be used as vaccines against mycobacterial diseases as they lack the immunosuppressive action of the mannose cap.

Most work in the tuberculosis vaccine field is directed by the belief that BCG is 'missing something of *M*. *tuberculosis'* and that this either has to be added back to BCG to improve vaccine-induced protection, or that, conversely, *M. tb* should be attenuated to the low virulence of BCG while keeping its immunodominant antigens. Examples of the former are recombinant BCG strains overexpressing immunodominant *M*. *tb* antigens (Castanon-Arreola et al, 2005; Horwitz & Harth, 2003; Pym et al, 2003) and examples of the latter are virulence factor (Copenhaver et al, 2004), auxotrophic (Sambandamurthy et al, 2005; Sambandamurthy et al, 2002) or signal transduction (Martin et al, 2006) mutants of *M*. *tb.* Moreover, improved induction of phagosome maturation and apoptosis in phagocytes (Grode et al, 2005; Hinchey et al, 2007; Sadagopal et al, 2009; Sun et al, 2009; Velmurugan et al, 2007) is pursued to increase crosspresentation and therewith, vaccine efficiency. However, of all the engineered live vaccines, in direct comparisons with BCG vaccine, only five have been demonstrated to be sufficiently promising in experimental animals to be moved forward to Phase I clinical trials (STOP-TBPartnership, 2009): BCG overexpressing antigen 85b (rBCG30 prolonged survival in guinea pigs) (Horwitz & Harth, 2003); the urease C-deficient listeriolysin-secreting recombinant BCG (Grode et al, 2005; Tchilian et al, 2009) (ΔureC hly+ rBCG decreased CFU counts in lungs of mice), the urease C-deficient perfringiolysin-secreting recombinant BCG (Sun et al, 2009) (safer than BCG in SCID mouse study), the phoP mutant of *M. tuberculosis* (Martin et al, 2006; Verreck et al, 2009) (prolonged survival in guinea pigs, under high-dose challenge, while equal protection against low-dose challenge, and equal protection in mice; decreased CFU counts in lungs of rhesus macaques) and the secA2 mutant of *M*. *tuberculosis* (Hinchey et al, 2007; Sadagopal et al, 2009) (prolonged survival in mice). As *M. bovis* BCG is a live vaccine, there are regulatory and safety concerns towards transgenic strains being implemented at the massive scale which is relevant for TB protection (e.g. transgene stability; risk for horizontal gene transfer of the transgene between the vaccine and *M. tb* itself (Krzywinska et al, 2004); different deliberate environmental release GMO regulations throughout the world) (Kamath et al, 2005; Walker et al, 2010). There are also potential safety issues with attenuated *M*. *tb* as a vaccine, and special precautions have to be taken (such as double mutations, with each single mutation resulting in sufficient attenuation) (Kamath et al, 2005; Walker et al, 2010). If a *M. bovis* BCG strain with better protective capability than the licensed strain BCG itself could be found in which the improved protection was brought about by targeted inactivation of endogenous genes rather than expressing heterologous transgenes, these concerns would be reduced and clinical implementation and public acceptance would be more straightforward.

### Summary

It was reasoned that mutation of mycobacterial components reportedly involved in phagosome maturation inhibition may yield better vaccines, as such mutations should result in better vaccine antigen processing and display. To this effect, an ordered *M. bovis* BCG transposon insertion mutant library was generated and mutants in 15 genes were identified, amongst which are 2 genes necessary for the alpha-1,2-oligomannosyl capping of cell wall ManLAM (Fratti et al, 2003; Hmama et al, 2004; Kang et al, 2005) and the PI3P phosphatase gene SapM[21]. Capping of LAM with α-1,2-oligomannosyl structures was until recently thought to be mainly mediated by the Rv1635c (Mb1661c)-encoded α-1,2-mannosyltransferase (Dinadayala et al, 2006). However, recent findings by Kaur and colleagues and in our laboratory demonstrate that another mannosyl-transferase (Rv2181 or Mb2203) is actually more selectively affecting the α-1,2-oligomannosyl cap structure synthesis, leaving the LAM levels in the cell wall intact (Kaur et al, 2006; Kaur et al, 2008). Therefore, this mutant is more suitable to address the specific role of α-1,2-oligomannosyl capping than the Mb1661c mutant (Appelmelk et al, 2008), which might have an altered cell wall composition compensating to the LAM deficiency. As ManLAM (mannosylated lipoarabinomannan) has been reported to play a role in subverting host cell signaling pathways (18) and blocking phagosome maturation (15) in host macrophages, mutants in the synthesis of ManLAM in slow-growing mycobacterium such as M. bovis BCG are of interest.

Both ManLAM capping and SapM were assigned important functions in phagosome maturation inhibition, but this has been disputed for ManLAM capping upon mutant studies[22] and has not yet been validated through mutant studies for SapM. In addition, the proposed LM α-1,6-mannosyltransferase Mb2196, which was not studied sofar in slow growing Mycobacteria, has now for the first time been biochemically characterized. These transposon insertion mutants of *M*. *bovis* BCG were evaluated as TB vaccines in a stringent model and it could be shown that they all have surprisingly improved vaccine efficacy, with the SapM::T mutant vaccinees showing the longest survival. The mutant BCG strains showed unaltered uptake by macrophages and dendritic cells and lysosome colocalization in macrophages *in vitro.* In addition, their replication and granuloma formation in mice was comparable to *M. bovis* BCG wild type, indicating BCG-equivalent safety of these vaccines. Vaccination with the SapM locus mutant significantly increases recruitment of CD11c⁺CD40⁺ MHC-II^{lo/med} CD8α⁻ DCs to the draining lymph nodes and their activation, explaining an improved immune response. Further exploration of the mechanism evoking the improved vaccine efficiency of the mutants showed that the latter induced similar oxidant activity and autophagy following infection of macrophages compared to wild type BCG.

Thus, according to a first aspect, a mycobacterium is provided comprising a genetically engineered mutation in at least one endogenous gene selected from SapM, the endogenous ManLAM α-1,2-mannosyltransferase corresponding to Mb1661c in *Mycobacterium bovis,* the endogenous ManLAM α-1,2-mannosyltransferase corresponding to Mb2203 in *Mycobacterium bovis* and the endogenous LM α-1,6-mannosyltransferase corresponding to Mb2196 in *Mycobacterium bovis.* These ManLAM α-1,2-mannosyltransferases are also equivalent to Rv1635c and Rv2181, respectively, in the *Mycobacterium tuberculosis* H37Rv strain. According to specific embodiments, the genetically engineered mutation (i.e., mutation obtained by genetic engineering methods) is a non-naturally occurring mutation. According to particular embodiments, combinations of the mentioned genes are mutated and/or an endogenous gene contains more than one mutation. According to specific embodiments, the genetically engineered mutation encodes a gene product that has a reduced (knock-down) or absent (knock-out) functionality.

This may be either because there is none or less of the gene product present than in the corresponding wild type strain, and/or because the gene product is not or only partially functional. According to particular embodiments, the genetically engineered mutation is created by insertion mutagenesis. According to specific embodiments, the mycobacterium may have further mutations as well. According to even more specific embodiments, the mycobacterium also comprises at least one mutation that decreases virulence. According to alternative, but not exclusive embodiments, the mycobacterium also comprises at least one mutation in an endogenous antioxidant gene. According to further embodiments, the endogenous antioxidant gene is selected from secA2, sigH and SodA (Sadagopal et al., 2009). According to particular embodiments, the endogenous SapM gene, the ManLAM α-1,2-mannosyltransferase corresponding to the Mb1661c gene, the ManLAM α-1,2-mannosyltransferase corresponding to the Mb2203 gene or the LM α-1,6-mannosyltransferase corresponding to the Mb2196 gene comprises SEQ ID NOs: 1, 3, 5, or 7, contiguous portions thereof, or sequences at least 95%, at least 98%, or at least 99% identical thereto, respectively; or comprises a sequence encoding SEQ ID NOs: 2, 4, 6, or 8, contiguous portions thereof, or encoding sequences at least 95%, at least 98%, or at least 99% identical thereto, respectively.

According to specific embodiments, the mycobacteria described herein are selected from Mycobacterium tuberculosis, Mycobacterium bovis or M. bovis Bacille Calmette-Guérin (BCG). According to very specific embodiments, the mycobacterium is selected from the deposited strains LMG P-25310, LMG P-25309, LMG P-25308, and LMG P-25441.

In a further aspect, uses of a mycobacterium as described herein, or of a portion thereof, are provided for the preparation of a recombinant vaccine, particularly a vaccine against tuberculosis. According to particular embodiments, the vaccine is a *M. bovis, M. bovis* Bacille Calmette-Guérin (BCG), or *M*. *tuberculosis*-based vaccine. According to alternative (but not exclusive) particular embodiments, the vaccine comprises a live attenuated vaccine.

Thus also methods are provided for preparing a vaccine (or vaccine composition), comprising the step of generating a vaccine, e.g. by providing a live attenuated mycobacterium strain in a pharmaceutically acceptable carrier.

According to yet a further aspect, vaccines are provided comprising a mycobacterium as described herein, thus with a genetically engineered mutation in at least one endogenous gene selected from SapM, the endogenous ManLAM α-1,2-mannosyltransferase corresponding to Mb1661c in Mycobacterium bovis, the endogenous ManLAM α-1,2-mannosyltransferase corresponding to Mb2203 in Mycobacterium bovis, and the endogenous LM α-1,6-mannosyltransferase corresponding to Mb2196 in *Mycobacterium bovis,* in a pharmaceutically acceptable carrier or excipient. According to an alternative embodiment, the vaccines comprise a portion of said mycobacterium.

The vaccine may be useful in the treatment of several diseases, as recombinant mycobacterium-based vaccines are known to be excellent carrier vaccines (Bastos et al., 2009, particularly the tables therein). According to most specific embodiments, the vaccine is a vaccine against tuberculosis. In other words, the vaccine is suitable to protect an animal, most particularly a mammal, from challenge by a virulent mycobacterium selected from M. bovis or M. tuberculosis. Mammals may for instance be selected from humans (in particular human children) or bovines, particularly cows.

According to particular embodiments, the vaccine is a live attenuated vaccine.

Thus, according to a particular aspect, the mycobacterium as described herein is provided for use in treatment of tuberculosis. According to further embodiments, the vaccine as described herein is provided for use in treatment of tuberculosis. Thus, methods are provided of protecting a mammal from a virulent Mycobacterium infection, particular from *Mycobacterium tuberculosis* or *M*. *bovis,* comprising treating the mammal with a vaccine as described herein (or possibly with a live attenuated mycobacterium as described herein). Alternatively, methods are provided of inducing an immune response in a mammal, the method comprising inoculating the mammal with the mycobacterium as described herein or the vaccine as described herein.

### Figure legends

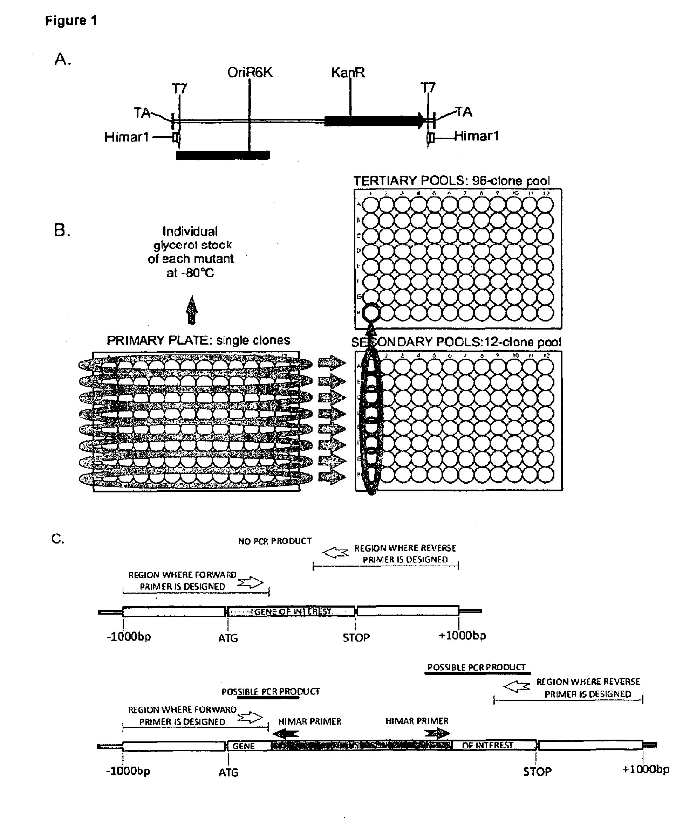

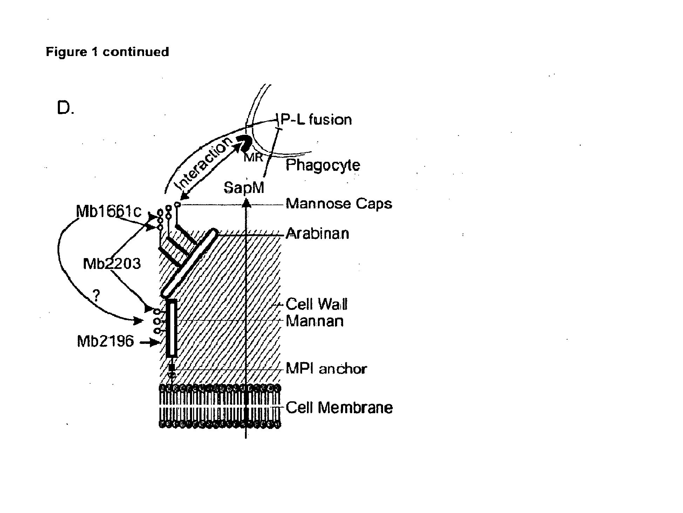

**Figure 1****. Generation of an ordered transposon insertion mutant library and schematic representation of the 4 selected mutants**
**A.** The transposon insertion mutant library was created using the donor phagemid ΦMycomarT7. This phagemid includes a transposon that encodes a kanamycin resistance gene, T7 promoters oriented so as to promote transcription into adjacent chromosomal DNA and terminal 29-bp inverted repeats. This construct integrates into the mycobacterial genome theoretically after every 'TA'. **B.** Individual mutants were grown in 96 well plates till mid log phase (21 days) and aliquots from one row of the 96 well plates were pooled into a single well of a secondary pool plate. Aliquots from each well in a column from the secondary PCR pool plate were pooled into a single well of a tertiary PCR pool plate. These pools were frozen at -20°C and used for genomic DNA isolation for PCR screening. **C.** We performed PCR screening for specific mutants in the ordered library using a primer hybridizing to the inverted repeats at either ends of the transposon (Himar primer), and gene-specific primers either upstream or downstream of (and occasionally in) the ORF of the target gene. **D.** The three mannosyltransferases and the lipid phosphatase biochemically characterized in this paper are shown in this panel with their site of action. Question marks indicate that the function of the genes in the indicated biosynthetic steps is either unclear at present (Mb1661c function in adding single α-1,2-mannose residues on the mannan backbone) or has not been confirmed in strains of the *M*. *tb* complex. Grey-filled circles represent single α-1,2-linked mannose residues.

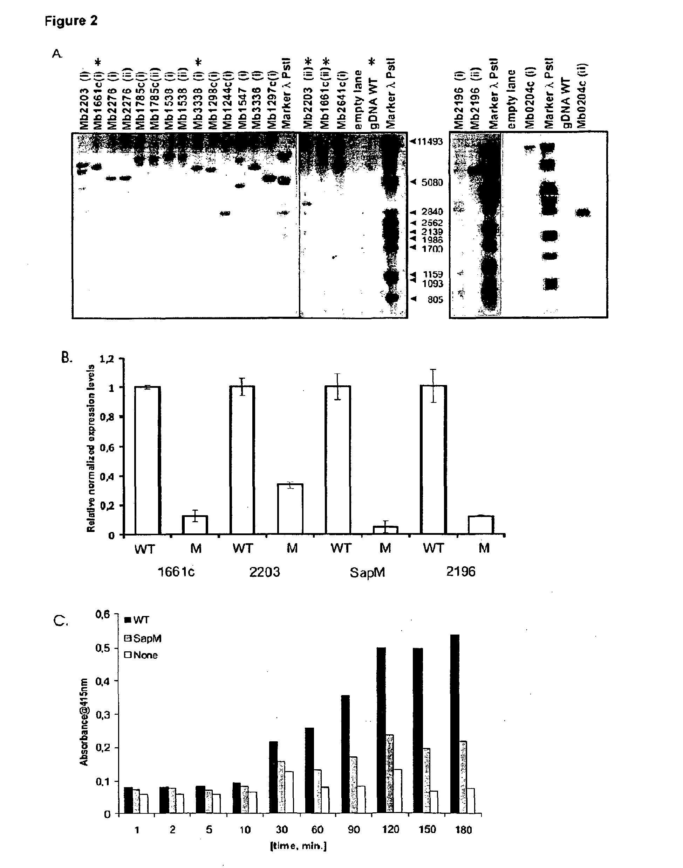

**Figure 2****. Southern blot analysis of the mutants, relative expression analysis of disrupted genes by quantitative PCR and phosphatase assay**
**A.** The genomic DNA of all the mutants was digested with *Bam*H1 and *Pst*1 (data not shown) and probed with ³²P dCTP labeled transposon. The number of bands corresponds to the number of insertions of the transposon in the mutant. Apart from three mutants, i.e. mutants in Mb2203 (i), Mb1547(i), and Mb2196 (i), all other mutants show single integration of the transposon. The markers used were the λPst1 marker prepared in-house by digesting 1µg of lambda DNA with 1U Pstl enzyme (Fermentas, Canada) at 37°C for 2 hours. **B.** Quantification of Mb1661c, Mb2203, SapM (Mb3338) and Mb2196 transcript in wild type (WT) and mutants in genes for Mb1661c, Mb2203, SapM, Mb2196 (M). Transcript levels were measured by real-time PCR on cDNA prepared using RNA extracted from log phase cultures of wild type and mutants. Primer sets are given in the table below. All values were normalized against the expression level of the 16S rRNA encoding housekeeping gene. The wild type to mutant transcript ratio was arbitrarily set to 1. Data represent average ± SD (n=3). Similar results were obtained in an independent quantitative PCR performed using a second set of primers for each transcript (data not shown). **C.** Phosphatase activity in the culture supernatant of *M*. *bovis* BCG wild type (black bars) versus *M*. *bovis BCG* SapM mutant (grey bars) were determined by measuring the hydrolysis of pNPP at A415. Culture medium was used as a control (white bars).

| Primer Name | Primer Sequence |
|---|---|
| 1661c FP | 5' GACGTGGGCCGGAATCGAAGCA 3' (SEQ ID NO: 9) |
| 1661c RP | 5' GATCCACCCGACCTGCCAAACCTG 3'(SEQ ID NO: 10) |
| 2203 FP | 5' AACGCTTCGCACTGTGGGTGG 3'(SEQ ID NO: 11) |
| 2203 RP | 5'CGTAGGACATCCCCGCGAGTTGAC3'(SEQ ID NO: 12) |
| SapM FP | 5' ACACACCCGAGCGAACCGAAC 3'(SEQ ID NO: 13) |
| SapM RP | 5' CAAGCGGATGGGTACGAGGTCAGC 3'(SEQ ID NO: 14) |
| 2196 FP | 5' GATGGGCGGGGTGCACAACGAGAT 3'(SEQ ID NO: 15) |
| 2196 RP | 5' CACGCAGATGACGCAGCCAAACC 3'(SEQ ID NO: 16) |
| 16S FP | 5' ATGACGGCCTTCGGGTTGTAA 3'(SEQ ID NO: 17) |
| 16S RP | 5' CGGCTGCTGGCACGTAGTTG 3'(SEQ ID NO: 18) |

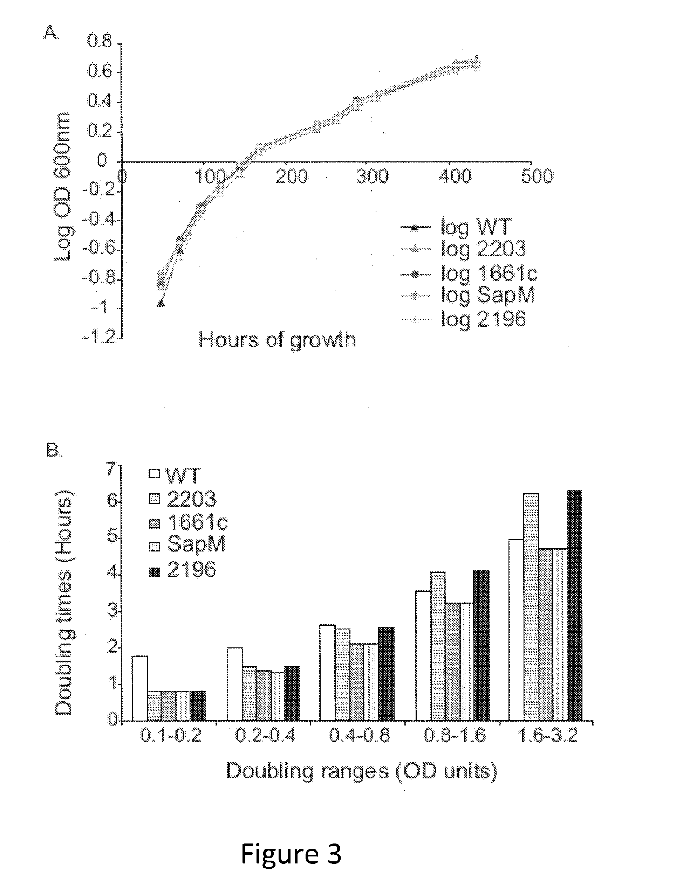

**Figure 3****. Growth Rate and doubling times**
**A.** Wild type and mutants are cultured in Middlebrook 7H9 broth (with glycerol, OADC, 0.05% tween-80 and antibiotics) for 3 weeks (504 hrs), no stirring, at 37°C. ODs are taken once every 24 hrs and doubling times calculated. No significant differences in growth rate are observed. **B.** Doubling times were calculated from the growth curves and it was observed that as the density of the cultures increases, the doubling time also increases.

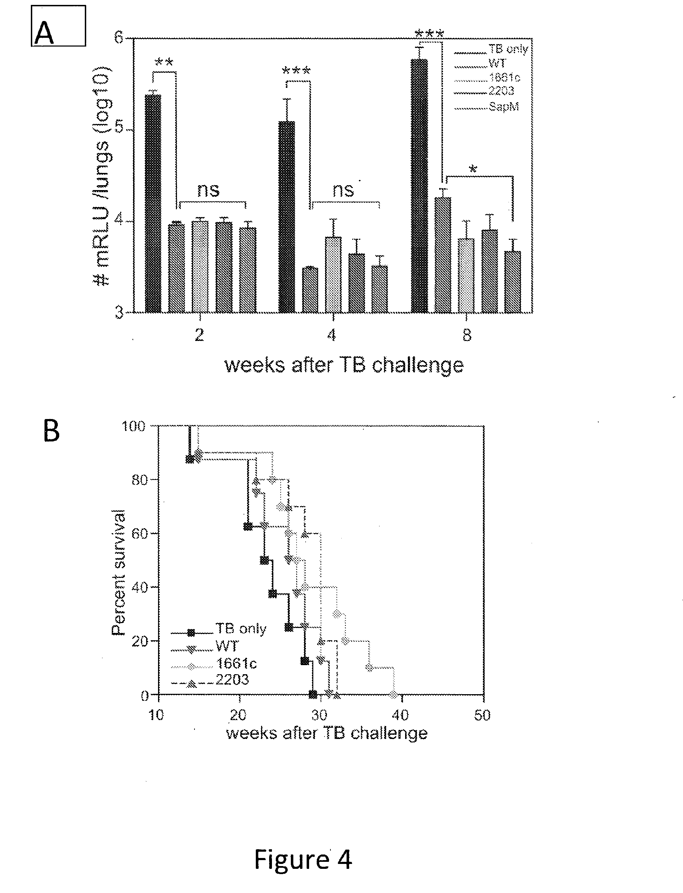

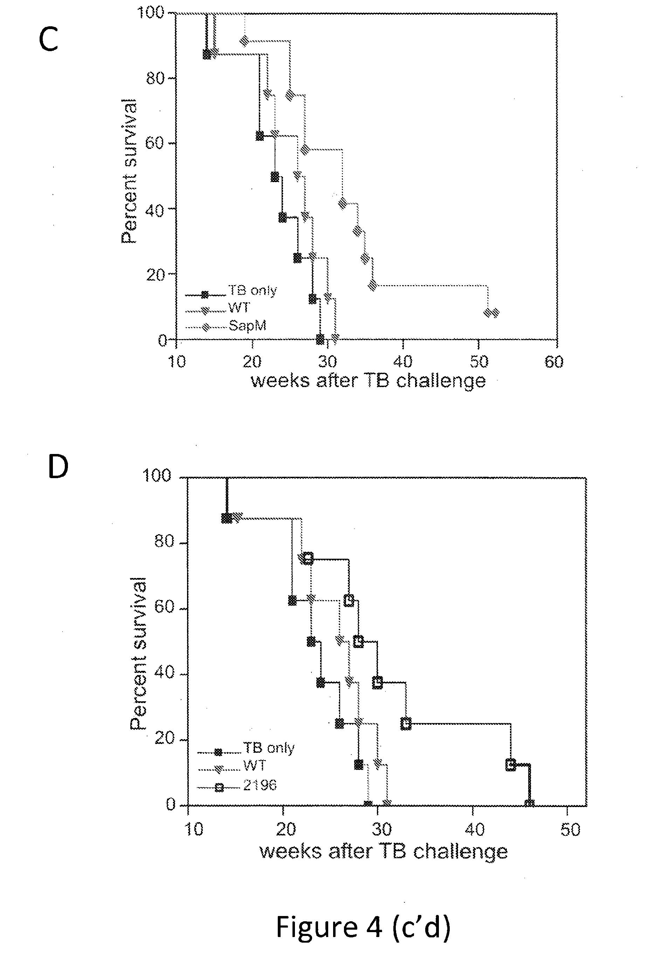

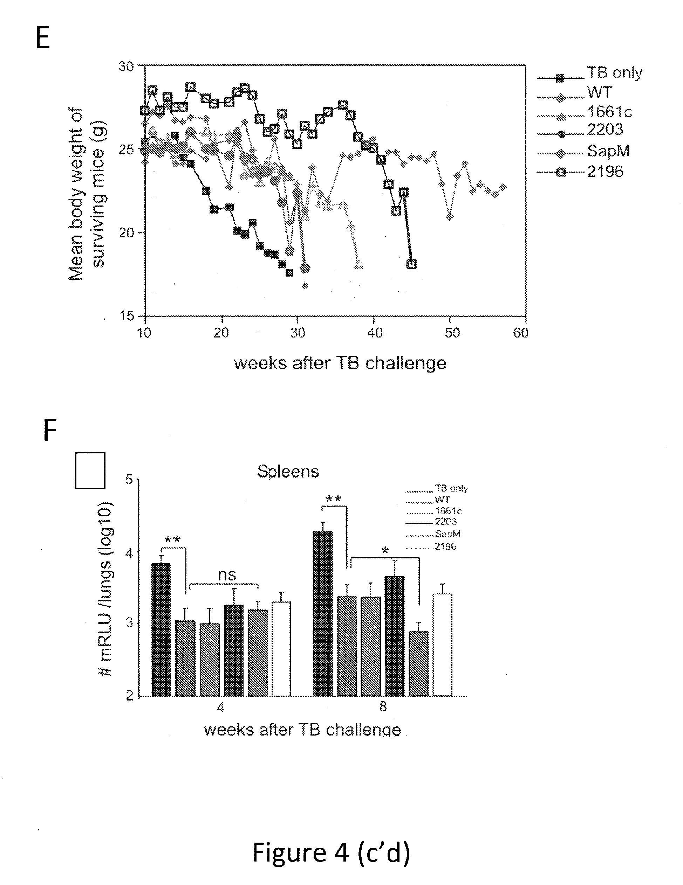

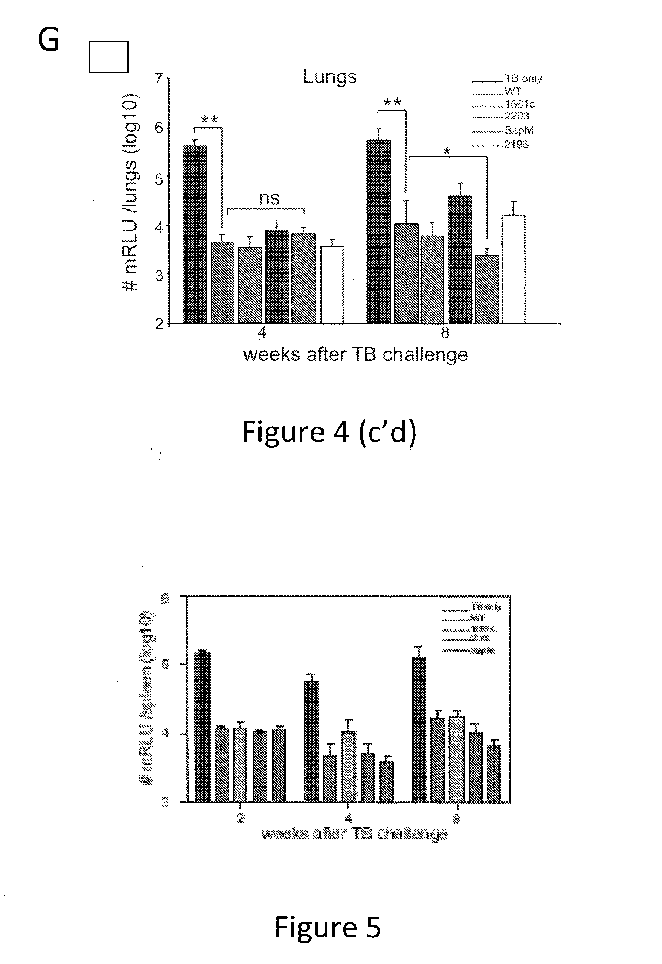

**Figure 4****. Protective efficacy of M. bovis BCG wild type versus mutant SapM::T (Mb3338), Mb2203::T, Mb1661c::T and Mb2196::T in Balb/c mice**
**A.** BALB/c mice were vaccinated subcutaneously with the parental or mutant M. bovis BCG strains. Three months post-vaccination, animals were intravenously infected with luminescent M. tuberculosis H37Rv. Mice were sacrificed 2, 4 and 8 weeks post-infection and the number of bacteria in lungs was determined by luminometry. (One-way ANOVA, Bonferroni's Multiple Comparison Test; ***P<0,0001; ^{**}P<0,0012; *P<0,05; ns, not significant) **B**-**E**. Mice were also followed for 11 months in a long term survival study and monitored weekly for mortality and weight loss. The percentage of survival is shown in the figure. The median survival time for unvaccinated mice is 23.5 weeks, and for mice vaccinated with BCG wild type, 1661c::T, 2203::T, 2196::T and SapM::T respectively 26,5 weeks, 27,5 weeks, 29 weeks, 30 weeks and 32 weeks (P=0.0145) (Kaplan-Meier, Bonferroni) **F-G.** BALB/c mice were vaccinated subcutaneously with the parental or mutant M. *bovis* BCG strains. Three months postvaccination, animals were intratracheally infected with luminescent M. *tuberculosis* H37Rv. Mice were sacrificed 4 and 8 weeks post-infection and the number of bacteria in spleens and lungs was determined by luminometry. (One-way ANOVA, Bonferroni's Multiple Comparison Test; ***P<0,0001; ^{**}P<0,0012; *P<0,05; ns, not significant)

**Figure 5****. Protective efficacy of M. *bovis* BCG wild type versus mutant SapM::T (Mb3338::T), Mb2203::T and Mb1661c::T in Balb/c mice**
BALB/c mice were vaccinated subcutaneously with 105 CFU of the parental or mutant *M*. *bovis* BCG strains. Three months post-vaccination, animals were intravenously infected with 5x10⁴ CFU of luminescent *M*. *tuberculosis* H37Rv. Mice were sacrificed 2, 4 and 8 weeks post-infection and the number of bacteria in spleens was determined by luminometry. No significant differences could be detected (One-way ANOVA, Bonferroni's Multiple Comparison Test).

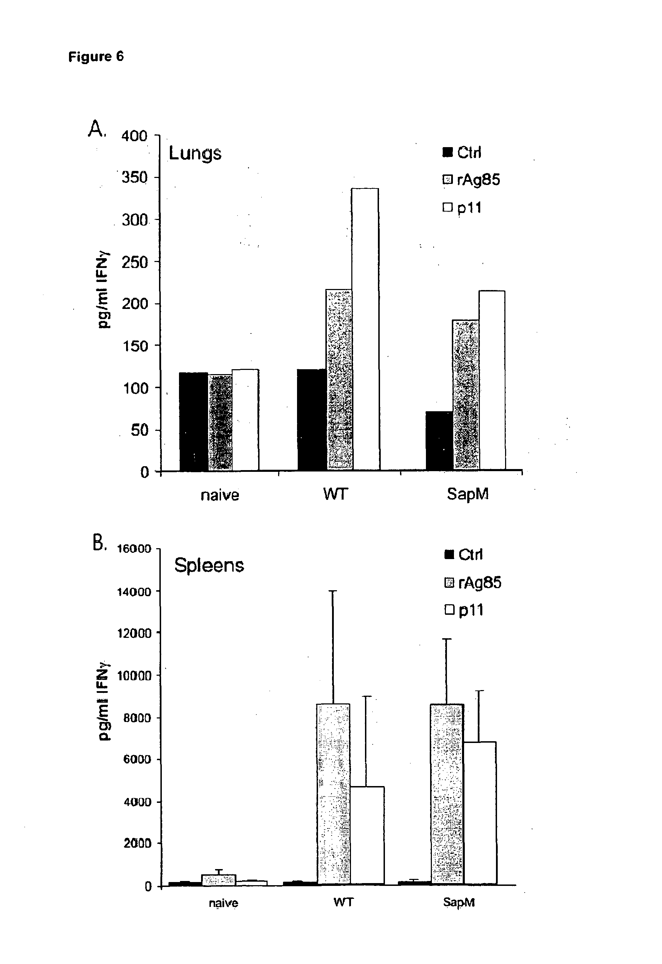

**Figure 6** **IFN-γ production following restimulation of spleen and lung cells from vaccinated mice**
BALB/c mice were vaccinated intravenously with the parental *M. bovis* BCG or the SapM locus mutant. Four weeks after infection, mice were killed and lungs and spleens were removed aseptically. **IFN-γ** production following restimulation of lungs **(A)** and splenocytes **(B)** was determined (72h post treatment) in response to recAg85A (Rv3804c) and the synthetic 20-mer peptide p11 (Ag85A99-118aa, dominant CD4 T cell epitope).

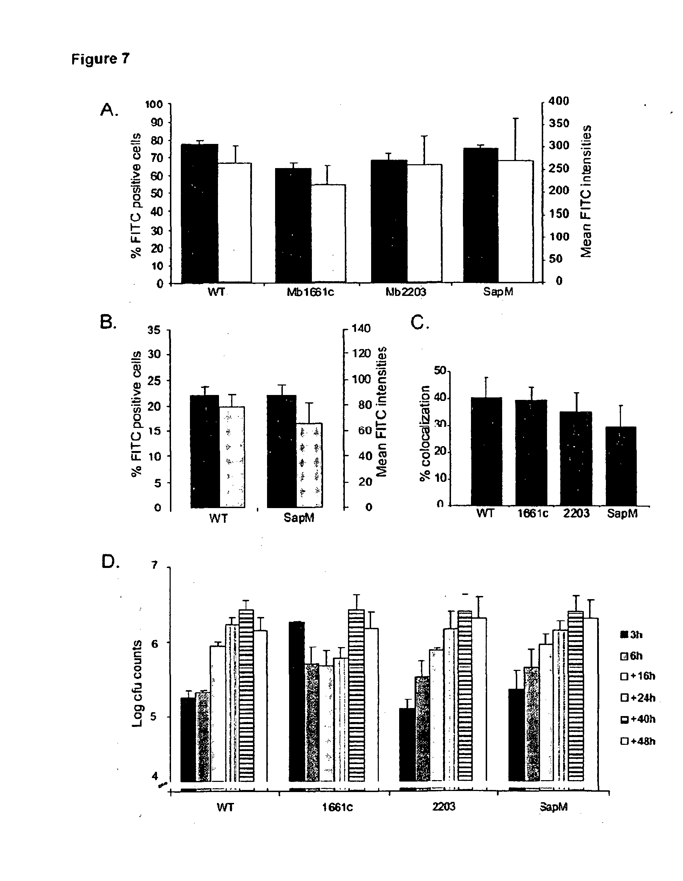

**Figure 7****. Binding of M. *bovis* BCG wild type and mutants to murine macrophages and BM-DCs and clearance following infection of macrophages**
**A.** Macrophages were infected for 6h with FITC-labeled mycobacteria (*M. bovis* BCG wild type versus mutants as indicated in the figure) at a MOI of 10. The amount of FITC-positive macrophages and mean FITC intensities are shown as averages of 6 independent experiments with indicated SEM. **B.** BM-DCs were infected for 24h with FITC-labeled mycobacteria (*M. bovis* BCG wild type versus SapM::T mutant as indicated in the figure) at a MOI of 2. The amount of FITC-positive DCs and mean FITC intensities are shown as averages of 5 independent experiments with indicated SEM. **C.** Colocalization of FITC-labeled bacteria and lysotracker-stained lysosomes in infected murine macrophages (3h infection) was determined by confocal microscopy. Shown is an average of 3 independent experiments, with indicated SEM. **D.** Mf4/4 cells were infected with *M*. *bovis* BCG wild type versus mutants (MOI 10) for the indicated time points. Infected cells were lysed and intracellular mycobacteria counted by plating on 7H10 agar. In a parallel experiment, the cell death of Mf4/4 cells following infection was checked by PI uptake. Macrophages survived at least 48h post-infection (data not shown). This experiment is an average of a duplicate experiment (with indicated SEM) and is representative for 3 other independent experiments. Y-axis is represented in log scale. No statistically significant differences were found in the experiments shown in A, B, C and D between any of the mutants and wild type BCG (P>0.1, Mann-Whitney U-test).

**Figure 8****. Intracellular oxidative activity within macrophages and autophagy induction by macrophages** and **BM-DCs infected with *M. bovis* BCG wild type versus mutants.**
Mf4/4 cells were infected with *M*. *bovis* BCG wild type versus mutants at MOI 10 and incubated at 37°C. At the indicated time points post-infection, cells were treated with CMH2DCFDA, washed and analyzed on a FACSCalibur where mean fluorescence intensity (MFI) was determined.
**Figure 9****. LN DC trafficking and activation**
Balb/cJ mice were infected subcutaneously with *M*. *bovis* BCG wild type and *M*. *bovis* BCG SapM mutant. At day 1 and day 7 post-infection, five mice per group were sacrificed and inguinal lymph nodes (ILN) were removed. Cells were prepared, labeled with the different antibodies and analyzed by flowcytometry. **A.** Determination of CD11c+ cells. **B.** Percentage of CD11c+ cells on the total ILN population. **C.** Percentage of CD11c+CD40+ MHC-IIlo/med CD8α- DCs. **D.** Representation of MHCIIlo CD40- (P3), MHCIImed CD40+ (P1), MHCIIhi CD40+ (P2) **(left column),** representation of expression levels of CD8a, MHCII, CD80 and CD86 in on cells in P1. (Kruskal-Wallis, Bonferroni's Multiple Comparison Test; ***P<0,005; **P<0,01; *P<0,05; ns, not significant)

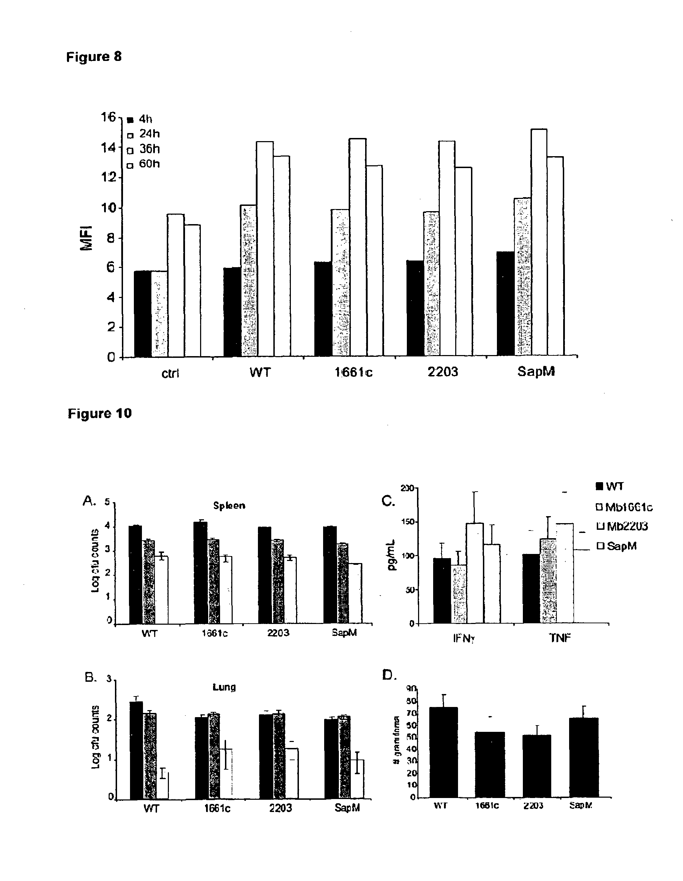

**Figure 10****. Bacterial replication of M. *bovis* BCG wild type versus mutants in spleen and lungs of mice, cytokine production and granuloma formation following infection of mice**
**A-B.** Balb/c mice were intravenously infected with *M*. *bovis* BCG wild type versus mutants and sacrificed 2 weeks (black bars), 4 weeks (dark gray bars) and 12 weeks (light gray bars) post-infection (5 per group). Lung and spleen homogenates were plated for CFU counting on 7H10 agar in duplicate. The mean and standard errors (SEM) of each group are shown in the figure. No difference was detected between the mutants and wild type (P>0.1, Mann-Whitney U-test). **C.** C57BL/6 mice were infected intratracheally with *M. bovis* BCG wild type and mutants. 4 weeks post-infection, five mice per group were killed and bronchoalveolar lavage (BAL) was performed. The mean levels of TNF and **IFN-γ** in the BALF and standard errors (SEM) of each group are shown and representative for 2 independent experiments. (P>0.05, Mann Whitney U test) **D.** C57BL/6 mice were infected intravenously with *M*. *bovis* BCG wild type and mutants. After 3 weeks, five mice per group were killed and livers were removed aseptically. Quantitative studies on hematoxylin/eosin (H&E)-stained paraffin sections to identify granuloma were performed by direct microscopic examination (3,5x magnification) and analysis with ImageJ software. The mean and standard errors (SEM) of each group are shown in the figure. Mb1661c::T and Mb2203::T induce less granuloma than *M. bovis* BCG wild type following infection of mice (P<0.01, Mann-Whitney U-test).

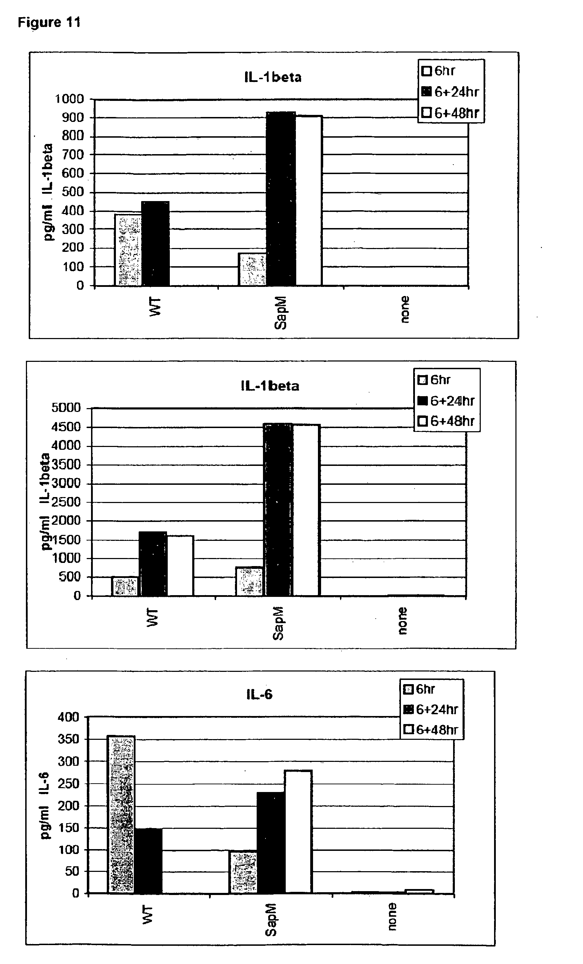

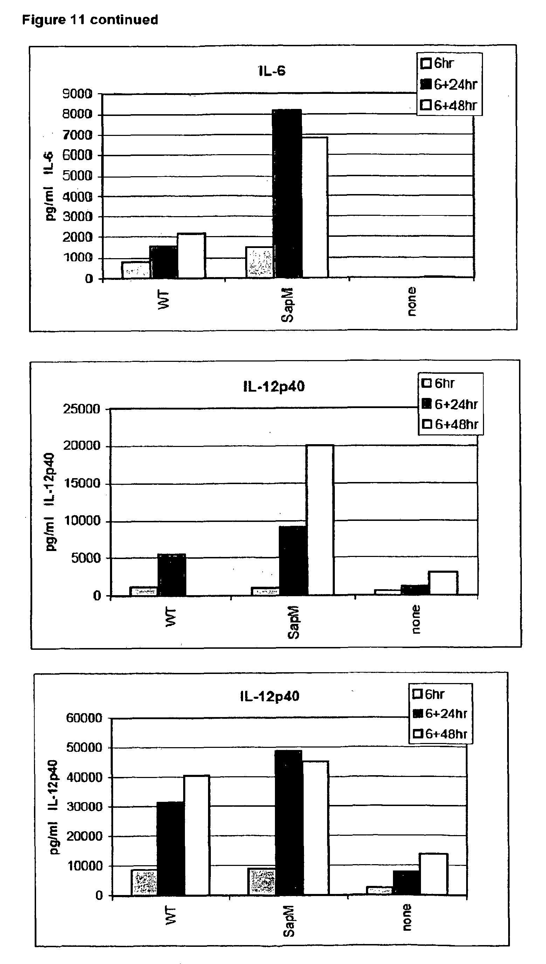

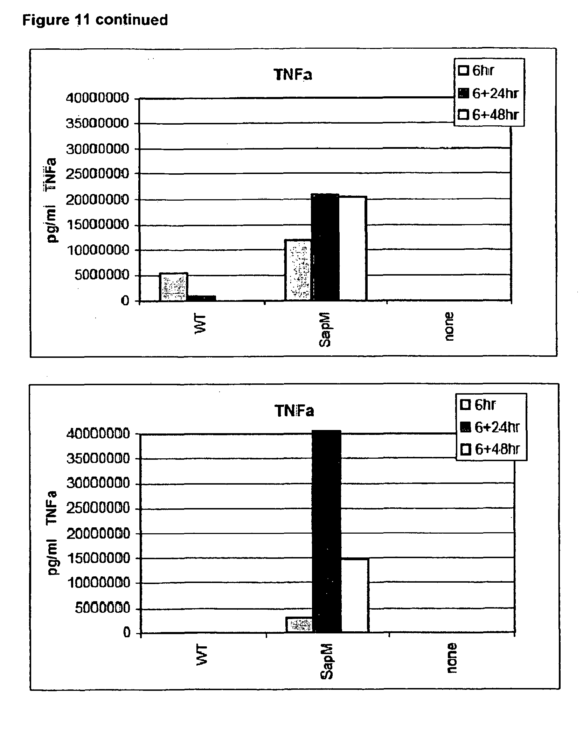

**Figure 11****. Cytokine production following infection of BM-DCs with *M*. *bovis* BCG wild type versus SapM::T**
BM-DCs were infected for 6h with *M. bovis* BCG wild type versus mutants (MOI 2). At the indicated time points, supernatant was removed and analyzed for the presence of cytokines by flow cytometry.

### Detailed description

### Definitions

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. Unless specifically defined herein, all terms used herein have the same meaning as they would to one skilled in the art of the present invention. Practitioners are particularly directed to Sambrook et al., Molecular Cloning: A Laboratory Manual, 2nd ed., Cold Spring Harbor Press, Plainsview, New York (1989); and Ausubel et al., Current Protocols in Molecular Biology (Supplement 47), John Wiley & Sons, New York (1999), for definitions and terms of the art. The definitions provided herein should not be construed to have a scope less than understood by a person of ordinary skill in the art.

A "mycobacterium" as used herein refers to micro-organisms of the genus *Mycobacterium* (sometimes abbreviated as *M*. herein), from the family of Mycobacteriaceae. Particularly envisaged mycobacteria include members of the *Mycobacterium tuberculosis* complex (Taxon identifier 77643 in the UniProt or NCBI taxonomy database), which includes the species *M*. *tuberculosis* (the major cause of human tuberculosis), *M*. *bovis, M. bovis BCG* (the strain most often used for vaccination purposes), M. *africanum, M. microti, M. canetti,* and *M. pinnipedii.*

A "genetically engineered mutation" as used throughout the application refers to a mutation in the genetic material (i.e. typically DNA), in particular a non-naturally occurring mutation, obtained via genetic engineering techniques. Such mutation can be a point mutation, a substitution, a deletion or an insertion. Typically, a genetically engineered mutation as envisaged herein will result in reduced or absent levels of the functional gene product (i.e., a loss-of-function mutation). The mechanism thereof is not vital to the invention, but typically may involve reduction or loss of transcription and/or translation of the affected gene, or only transcription/translation of a dysfunctional gene product. Genetically engineered mutations in a gene need not necessarily be in an exon or open reading frame, they can be in an intron or even be upstream of the start codon, as long as it affects the levels of the functional gene product. This can easily be checked, e.g. by Q-PCR for gene transcription levels. Functionality of a gene product can also be checked, as one of skill in the art will know, e.g. by providing a natural substrate to the affected enzyme.

An "endogenous gene" as used herein is a gene naturally occurring in the organism, in particularly in the species.

The term "SapM", as used herein refers to a gene which encodes a PI3P phosphatase (enzyme class 3.1.3.2), or to the encoded protein product. The gene is also known under other names, e.g. Rv3310 (from the *Mycobacterium tuberculosis H37Rv* strain; GeneID 887988), Mb3338 (from the *Mycobacterium bovis AF2122*/*97* strain, GeneID 1093156), SapM putative acid phosphatase or BCG_3375, SapM_1 or JTY_3335, SapM_2 or JTY_3355, SapM_3 or JTY_3375 (respective GenelDs 4696502 from *Mycobacterium bovis BCG str. Pasteur 1173P2,* 7561659, 7561660, and 7561661 from *Mycobacterium bovis BCG str. Tokyo 172.* The protein product of these differs in one amino acid (A171T) from Rv3310). Other GenelDs are known as well (typically as hypothetical or provisional protein and not yet validated), but all of these can be mapped to Rv3310 of the *H37Rv* strain and are thus readily identifiable to the skilled person. Examples include GenelDs 6698178, 6820173, and 5224028. The Rv3310 and Mb3338 genes and their encoded proteins show 100% sequence identity, the DNA and protein sequence are SEQ ID NO: 1 and 2, respectively. According to particular embodiments, the SapM gene is from a member of the Mycobacterium tuberculosis complex (Taxon identifier 77643). According to alternative very particular embodiments, the SapM gene does not encode the hypothetical protein MAP3432 (GeneID 2719192)

As used herein, the "endogenous ManLAM α-1,2-mannosyltransferase corresponding to Mb1661c in *Mycobacterium bovis"* is a gene that corresponds (can be mapped) to Mb1661c (Gene ID 1092601 from the *Mycobacterium bovis AF2122*/*97* strain) or Rv1635c (GeneID 885100 from the *Mycobacterium tuberculosis H37Rv* strain), or to the encoded protein product. These two genes are equivalent to each other and show 100% sequence identity in the encoded 556 aa gene product (SEQ ID NO: 4), as well as in the primary DNA sequence (SEQ ID NO: 3). As with the SapM gene, other GenelDs are known as well that can easily be (or are already) mapped to the H37RV Rv1635c gene. Examples include GenelDs 5222331,6822253 and 6701614.

The term "the endogenous ManLAM α-1,2-mannosyltransferase corresponding to Mb2203 in *Mycobacterium bovis"* as used herein is a gene that corresponds (can be mapped) to Mb2203 (GeneID 1091304 from the *Mycobacterium bovis AF2122*/*97* strain) or Rv2181 (GeneID 888269 from the *Mycobacterium tuberculosis H37Rv* strain), or to the encoded protein product. These two genes are equivalent to each other and show 100% sequence identity in the encoded 427 aa gene product (SEQ ID NO: 6), as well as in the primary DNA sequence (SEQ ID NO: 5). As with the SapM gene or Rv1635c gene, other GenelDs are known as well that can easily be (or are already) mapped to the H37RV Rv2181 gene. Examples include GenelDs 6701835, 5222885 and 6822792.

The term "the endogenous LM α-1,6-mannosyltransferase corresponding to Mb2196 in *Mycobacterium bovis"* as used herein is a gene that corresponds (can be mapped) to Mb2196 (GeneID 1091287 from the *Mycobacterium bovis AF2122*/*97* strain) or Rv2174 (GeneID 887528 from the *Mycobacterium tuberculosis H37Rv* strain), or to the encoded protein product. These two genes are equivalent to each other and show 99.8% sequence identity in the encoded 516 aa gene product (SEQ ID NO: 8) - the only difference being a Ser → Ala substitution at position 451 - as well as in the primary DNA sequence (SEQ ID NO: 7). As with the SapM gene or Rv1635c gene, other GeneIDs are known as well that can easily be (or are already) mapped to the H37RV Rv2174 gene. Examples include GeneID 909900.

The term 'contiguous portions of a sequence' as used herein refers to a non-interrupted sequence of nucleic acids or amino acids also occurring in the same order in the sequence referred to. Particularly envisaged are contiguous portions having a length of at least 25%, 50%, 70%, 75%, 80% or 90% of the length of the reference sequence, and contiguous portions are typically at least 25 nucleic acids or at least 8 amino acids.

A 'live attenuated vaccine' as used herein is a vaccine containing live or viable micro-organisms with reduced virulence (attenuated); as opposed to an inactivated vaccine.

The term "sequence identity" as used herein refers to the extent that sequences are identical on a nucleotide-by-nucleotide basis or an amino acid-by-amino acid basis over a window of comparison. Thus, a "percentage of sequence identity" is calculated by comparing two optimally aligned sequences over the window of comparison, determining the number of positions at which the identical nucleic acid base (e.g., A, T, C, G) or the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity. For the purposes of the present invention, "sequence identity" will be understood to mean the "match percentage" calculated by the DNASIS computer program (Version 2.5 for windows; available from Hitachi Software engineering Co., Ltd., South San Francisco, Calif., USA; or accessible via the internet) using standard defaults as used in the reference manual accompanying the software. "Similarity" refers to the percentage number of amino acids that are identical or constitute conservative substitutions (e.g. replacing one hydrophobic amino acid residue with another, or one positively charged amino acid residue with another). Similarity may be determined using sequence comparison programs such as GAP (Deveraux et al. 1984, Nucleic Acids Research 12, 387-395) or BLAST. In this way, sequences of a similar or substantially different length to those cited herein might be compared by insertion of gaps into the alignment, such gaps being determined, for example, by the comparison algorithm used by GAP.

The present invention relates inter alia to mycobacteria that are mutated in a gene encoding mycobacterial components reportedly involved in phagosome maturation inhibition, and their use in vaccine preparation. Such mutations were found to result in better vaccine antigen processing and presentation. This results in more effective vaccines (offering prolonged survival and/or increased chance of survival in a mouse model of *M*. *tuberculosis* infection), while maintaining the other properties of the strain used for vaccination (e.g. relating to biosafety measures).

Thus, according to a first aspect, a mycobacterium is provided comprising a genetically engineered mutation in at least one endogenous gene selected from SapM, the endogenous ManLAM α-1,2-mannosyltransferase corresponding to Mb1661c in *Mycobacterium bovis,* the endogenous ManLAM α-1,2-mannosyltransferase corresponding to Mb2203 in *Mycobacterium bovis,* and the endogenous LM α-1,6-mannosyltransferase corresponding to Mb2196 in *Mycobacterium bovis.* The nomenclature of genes across different species of mycobacteria is incomplete, and not all genes in each species have been attributed a function yet. In order to avoid confusion, it was chosen to indicate the genes in this way. Based on sequence homology, the skilled person will be able to readily identify the corresponding (or equivalent) gene in other species. For instance, in the *Mycobacterium tuberculosis* H37Rv reference strain, the gene corresponding to Mb1661c is Rv1635c, both having the same sequence (SEQ ID NO: 3), and the gene corresponding to Mb2203 is indicated as Rv2181, also having an identical sequence (SEQ ID NO: 5) (see Table 1). Note that this also applies to the PI3P phosphatase SapM: indicated as Mb3338 and Rv3310 in *Mycobacterium bovis* and *Mycobacterium tuberculosis* respectively, but having an identical sequence, corresponding to SEQ ID NO: 1.

Thus, according to particular embodiments, the endogenous SapM gene comprises SEQ ID NO: 1, contiguous portions thereof, or sequences at least 90%, at least 95%, at least 98%, or at least 99% identical to SEQ ID NO: 1 or the contiguous portions thereof. Similarly, the ManLAM α-1,2-mannosyltransferase equivalent to the Mb1661c gene may comprise SEQ ID NO: 3, contiguous portions of this sequence, or sequences at least 90%, at least 95%, at least 98%, or at least 99% identical to SEQ ID NO: 3 or the contiguous portions thereof. Likewise, the ManLAM α-1,2-mannosyltransferase corresponding to Mb2203 comprises in particular embodiments SEQ ID NO: 5, contiguous portions thereof, or sequences at least 90%, at least 95%, at least 98%, or at least 99% identical thereto. Or the LM α-1,6-mannosyltransferase corresponding to Mb2196 comprises in particular embodiments SEQ ID NO: 7, contiguous portions thereof, or sequences at least 90%, at least 95%, at least 98%, or at least 99% identical thereto.

Alternatively, the percentage sequence identity or similarity may be defined at the protein (amino acid) level. Thus, the endogenous SapM gene may be defined as a gene encoding a sequence comprising SEQ ID NO: 2, contiguous portions of this sequence, or encoding sequences at least 90%, at least 95%, at least 98%, or at least 99% identical to SEQ ID NO: 2 or a contiguous portion thereof. Similarly, the ManLAM α-1,2-mannosyltransferase equivalent to the Mb1661c gene may encode sequences comprising SEQ ID NO: 4, contiguous portions thereof, or sequences at least 90%, at least 95%, at least 98%, or at least 99% identical to SEQ ID NO: 4 or the contiguous portions thereof. Likewise, the ManLAM α-1,2-mannosyltransferase corresponding to Mb2203 encodes in particular embodiments sequences comprising SEQ ID NO: 6, contiguous portions thereof, or sequences at least 90%, at least 95%, at least 98%, or at least 99% identical thereto. Or the LM α-1,6-mannosyltransferase corresponding to Mb2196 encodes in particular embodiments sequences comprising SEQ ID NO: 8, contiguous portions thereof, or sequences at least 90%, at least 95%, at least 98%, or at least 99% identical thereto.

According to particular embodiments, combinations of these genes are mutated, for instance both ManLAM α-1,2-mannosyltransferase genes are mutated, or the endogenous SapM gene and one or two of the ManLAM α-1,2-mannosyltransferase genes or the LM α-1,6-mannosyltransferase gene. Additionally and/or alternatively, one or more of these endogenous genes may contain more than one mutation.

According to specific embodiments, the gene with the genetically engineered mutation encodes a gene product that has a reduced (knock-down) or absent (knock-out) functionality. This may be either because there is none or less of the gene product present than in the corresponding wild type strain, and/or because the gene product is not or only partially functional. Thus, the genetically engineered mutations may influence expression levels of the gene product, stability of the gene product, encode defunct or nonsense gene products (e.g. by insertion of a stop codon), encode a different gene product, or any combination of these. According to specific embodiments, the expression levels of the gene product are reduced by 25%, 50%, 75% or more. Expression of the gene product can e.g. be evaluated by QPCR. Mutations may be insertions, substitutions or deletions, and may vary in size from one base pair (or one amino acid) to the whole gene (e.g. complete gene deletion or substitution). Of note, mutations also include mutations upstream of the start codon, such as in the promoter region, particularly mutations within 30 nucleotides upstream of the start codon, as long as these mutations affect the levels and/or function of the gene product. According to particular embodiments, the genetically engineered mutation is created by insertion mutagenesis. According to specific embodiments, the mutation is not generated using an allelic exchange mutagenesis method.

According to specific embodiments, the mycobacterium may have further mutations as well. According to even more specific embodiments, the mycobacterium also comprises at least one mutation that decreases virulence (note that this is for instance the case in the *Mycobacterium bovis* BCG strain). Another example of these further mutations, are mutations in antioxidant genes. Indeed, Sadagopal et al. have shown that reducing the activity and secretion of microbial antioxidants enhances the immunogenicity of BCG (Sadagopal et al., 2009). This may be of particular interest for pulmonary TB, as the traditional BCG strains are less reliable in protecting against this form of TB. As these antioxidants function independently from phagosome maturation, it can be expected that a mycobacterium with at least one mutation in a phagosome maturation gene (such as in SapM, Mb1661c, Mb2203 or Mb2196) and at least one mutation in an antioxidant gene will be even more suitable as basis for an improved vaccine composition. Particularly envisaged as antioxidant genes to be downregulated or knocked out are one or more (or all) of the group selected from secA2, sigH and SodA. How mutants in these antioxidant genes can be generated is described in Sadagopal et al. (Sadagopal et al., 2009).

According to specific embodiments, the mycobacteria described herein are selected from *Mycobacterium tuberculosis, Mycobacterium bovis* or *Mycobacterium bovis* Bacille Calmette-Guérin (BCG). Note that many strains for BCG are known (including, but not limited to, BCG-Russia, -Japan, - Moreau, -Sweden, -Birkhaug, -China, -Prague, -Glaxo, -Danish, Mérieux, -Tice, -Phipps, -Frappier, - Connaught and -Pasteur), and all of them are envisaged for the applications described herein.

Brosch et al. (Brosch et al., 2007) have demonstrated that the different BCG strains harbor different duplicated regions. This is of interest, as some BCG strains (e.g. BCG-Pasteur) do not have a duplication of regions harbouring the phagosome maturation genes and antioxidant genes discussed herein, whereas others (e.g. BCG-Danish) do. As it is easier to ensure non-functional genes when they are not duplicated, it is envisaged in particular embodiments to use strains not harbouring a duplication of regions harbouring a phagosome maturation gene (such as e.g. SapM). Alternatively or additionally, it is envisaged that the strain does not have a duplication of an antioxidant gene.

According to very specific embodiments, the mycobacterium is selected from the deposited strains LMG P-25310 (*Mycobacterium bovis* BCG carrying a mutation in the SapM gene - Mb 3338), LMG P-25309 (M. bovis BCG carrying a mutation in the Mb1661c gene), LMG P-25308 (M. bovis BCG carrying a mutation in the Mb2203 gene) and LMG P-25441 (M. bovis BCG carrying a mutation in the Mb2196 gene). Deposits have been made at the BCCM/LMG Bacteria Collection, University of Gent, Belgium. Also deposited was the wild-type M. bovis BCG strain used for generation of the mutants, as LMG P-25307.

In a further aspect, uses of a mycobacterium as described herein, or of a portion thereof, are provided for the preparation of a recombinant vaccine, particularly a vaccine against tuberculosis. Whereas typically, a whole (attenuated) mycobacterium will be used in a vaccine, it is envisaged that portions thereof may be used (a so-called 'sub-unit vaccine'), e.g. containing specific proteins from the Mycobacterium. Very particular subsets of sub-unit vaccines are recombinant vaccines, in which at least one protein from a mycobacterium is non-endogenously present in micro-organisms used for vaccination (e.g. through recombinant overexpression). A recombinant vaccine can also be used as a vaccine against other diseases than tuberculosis, when other non-endogenous proteins are expressed as antigens in the mycobacterium.

The development of mycobacterial genetic systems to express foreign antigens and the adjuvanticity of BCG are the basis of the potential use of this attenuated mycobacterium as a recombinant vaccine (Stover et al., 1991). Over the years, a range of strategies has been developed to allow controlled and stable expression of viral, bacterial and parasite antigens in BCG, resulting in both cellular and humoral immune responses against these heterologous antigens (Dennehy et al., 2005). Moreover, it has been demonstrated that recombinant BCG overexpressing antigens of *M*. *tuberculosis* is more efficient in conferring protection against tuberculosis than the wild type BCG strain (Castanon-Arreola et al., 2005; Horwitz, 2005).

A good overview of heterologous bacterial, parasite and viral antigens that may successfully be used in recombinant BCG as a vaccine vector is given in Bastos et al. (Bastos et al., 2009), particularly in tables 1-3. Examples include, but are not limited to, bacterial antigens to provide a vaccine against bacterial infection (e.g. of *Escherichia coli, Borrelia burgdorferi, Streptococcus pneumonia, Listeria monocytogenes, Bordetella pertussis, Mycobacterium tuberculosis, Mycobacterium leprae, Anaplasma marginale, Leptospira interrogans, Mycoplasma hyopneumoniae, Clostridium perfringens*), bacterial toxins (e.g. Pertusis toxin, tetanus toxin, cholera toxin), parasitic antigens to provide protection against parasitic disease (e.g. of *Leishmania* species, *Schistosoma mansoni, Schistosoma haematobium, Schistosoma japonicum, Plasmodium yoelii, Plasmodium falciparum, Toxoplasma gondii, Leishmania chagasi, Echinococus multilocularis, Eimeria tenella*), and viral antigens to protect against viral disease (e.g. of HIV-1, FMDV (foot-and-mouth disease virus), SIV, measles virus, papillomavirus, rabies virus, PRRSV (Porcine reproductive and respiratory syndrome virus), HCV (Hepatitis C virus), HBV (Hepatitis B virus), CRPV (cottontail rabbit papillomavirus), rotavirus, infectious bronchitis virus, RSV (respiratory syncytial virus)).

The skilled person will readily appreciate the potential of recombinant BCG and realize that this list is non-exhaustive and that other antigens can be readily tested to provide a vaccine for other (or even the same) diseases (for instance, a BCG equipped with the membraneperforating listeriolysin (Hly) of *Listeria monocytogenes* showed a significantly improved protection in mouse model when compared to the parental BCG strain following aerosol challenge with M. tuberculosis (Grode et al., 2005)).

Moreover, recombinant BCG may also be used as an immunomodulator. Immunomodulators are substances or live organisms that accelerate, prolong, or enhance the quality of a specificimmuneresponse to antigens. Live, attenuated, recombinant bacterial vaccines have been largely used to modulate the immune system to respond in a specific profile to a specific antigen. The immunomodulator effect of wild type BCG has been well described, and recently the expression of cytokines has even improved this effect. This approach has allowed modulation of the immune system to respond with a specific and desired pattern of cytokines. Examples of cytokines that can be expressed in recombinant BCG include, but are not limited to, IFN-γ, IL-2, IL-18, IFN-α-2b (see table 4 of Bastos et al., 2009).

Through immunomodulation, BCG may also be a useful therapy in the treatment of cancer, e.g. by strengthening particular immune responses. Even non-recombinant BCG can be used in the treatment of cancer, as promising results have been shown in e.g. leukemia, melanoma, colon cancer, lung cancer, skin cancer and bladder cancer. It is envisaged that the mycobacteria described herein can be used to develop vaccines (either recombinant or non-recombinant) against these diseases. Most particularly, it is envisaged that the mycobacteria provided herein can be used for a vaccine against bladder cancer. Indeed, BCG has been FDA-approved for treatment of bladder cancer in 1990. Most particularly, it is envisaged to treat superficial bladder cancer (i.e., bladder cancer not invading the muscular wall of the bladder), as BCG is recognized as the most effective treatment for this disease. This could even further be increased by providing BCG that recombinantly express IL-18 (Luo et al., 2004).

According to particular embodiments, the vaccine is a M. bovis, M. bovis Bacille Calmette-Guérin (BCG), or M. tuberculosis-based vaccine. According to alternative (but not exclusive) particular embodiments, the vaccine comprises a live attenuated vaccine.

Thus also methods are provided for preparing a vaccine (or vaccine composition), comprising the step of generating a vaccine, e.g. by providing a live attenuated mycobacterium strain in a pharmaceutically acceptable carrier.

According to yet a further aspect, vaccines are provided comprising a mycobacterium as described herein, thus with a non-naturally occurring mutation in at least one endogenous gene selected from SapM, the endogenous ManLAM α-1,2-mannosyltransferase corresponding to Mb1661c in Mycobacterium bovis, the endogenous ManLAM α-1,2-mannosyltransferase corresponding to Mb2203 in Mycobacterium bovis, and the endogenous LM α-1,6-mannosyltransferase corresponding to Mb2196 in Mycobacterium bovis, in a pharmaceutically acceptable carrier or excipient. According to an alternative embodiment, the vaccines comprise a portion of said mycobacterium.

According to most specific embodiments, the vaccine is a vaccine against tuberculosis. In other words, the vaccine is suitable to protect an animal, in particular a mammal, from challenge by a virulent mycobacterium selected from M. bovis or M. tuberculosis. Many mammals are known to suffer from tuberculosis, in particular from M. tuberculosis or M. bovis infection, and these animals may benefit from the vaccine. Such mammals include pets (such as, but not limited to, cats, dogs, rabbits, hamsters, guinea pigs and the like), livestock (such as, but not limited to, horses, donkeys, mules, bovines, cows or other cattle, Ilamas, camels or other camelids, pigs, sheep, goats, deer, elk, reindeer), non-domestic or zoo animals (such as, but not limited to, elephants, non-human primates (e.g. mandrills, baboons, macaques, ...), wild felines (tigers, lions, ...), bears, antelopes, sea lions, badgers, possums, and the like) and humans. Tuberculosis may behave as a zoonotic disease. Mammals that are particularly envisaged for the vaccines described herein are bovines, most particularly cows. Also particularly envisaged are humans, in particular children. Generally, young animals are particularly envisaged for the vaccines described herein. With young animals, it is typically meant that they are not yet of reproductive age.

According to particular embodiments, the vaccine is a live attenuated vaccine.

Thus, the mycobacterium as described herein is provided for use in treatment of tuberculosis. According to further embodiments, the vaccine as described herein is provided for use in treatment of tuberculosis. Thus, methods are provided of protecting an animal, in particular a mammal, in particular an animal in need thereof, from a virulent *Mycobacterium tuberculosis* or *M*. *bovis,* comprising treating the mammal with a vaccine as described herein (or possibly with a live attenuated mycobacterium as described herein). Alternatively, methods are provided of inducing an immune response in a mammal, the method comprising inoculating the mammal with the mycobacterium as described herein or the vaccine as described herein.

The way of administration of the vaccine can be determined by the skilled person, in accordance with the administration routes known in the art. These include, but are not limited to, subcutaneous administration, intradermal administration, intranasal administration, oral administration, parenteral administration, intramuscular administration, injection or the like.

The dosage regimen may also be determined by the skilled person using his expertise (e.g. single administration, repeated administration (twice or more at regular or irregular intervals), etc. This will typically also depend on the disease to be treated and the individual receiving the treatment (in bladder cancer in humans, for instance, a low-dose BCG regimen has been described as 75 mg, while a standard dose is 150 mg. However, doses doses of BCG as low as 1 mg have been documented to effectively support an immune response for a long period of time (5 years). In tuberculosis, an example of a typical dose is much lower: 0,075 mg, corresponding to 0,3-1,2 million living mycobacteria). Roughly speaking, a typical dose may fall between 0,01 µg/kg body weight and 10 mg/kg body weight. In treatment of tuberculosis, one treatment typically protects for a number of years. However, it is also envisaged that repeat doses are given (as is e.g. typically the case in treatment of bladder cancer).

As the vaccines described herein are more effective than standard vaccines, it is also envisaged that the treatment regimen may be shorter than for a treatment regimen with the equivalent WT mycobacterium strain; or that a suboptimal dose can be used (i.e., a dose that is lower than that typically used with the equivalent WT mycobacterium or WT mycobacterium-based vaccine).

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, useful methods and materials are described below. The materials, methods, and examples are illustrative only and not intended to be limiting. Other features and advantages of the invention will be apparent from the detailed description and from the claims.

### Examples

### Materials and methods

### Mycobacterial strains and media

The streptomycin resistant M. bovis BCG strain 1721 (RpsL, 42 Lys→Arg; a gift of Prof. P. Sander, Institute for Medical Microbiology, Zurich) and its mutants were grown in Middlebrook 7H9 broth (Difco) supplemented with 0.05% Tween80, Middlebrook OADC (Becton Dickinson) and appropriate antibiotic selection (100µg/ml streptomycin for wild type and additionally 50µg/ml kanamycin for the mutants) when grown in liquid culture. Difco Middlebrook 7H10 agar (similarly supplemented) was used for growth on solid culture. We selected the mutants on 7H10 plates supplemented with 20(µg/ml kanamycin. For the propagation of the phage, a streptomycin resistant M. smegmatis strain (also obtained from Prof. P. Sander) was used and grown in the Middlebrook 7H9 broth (Difco) supplemented with Middlebrook ADC (Becton Dickinson) without Tween80 or antibiotics. Throughout this study, M. bovis BCG was cultivated in static culture (i.e. without shaking) in tissue culture flasks equipped with a gas-permeable filter cap. The flasks were incubated upright with 10mL culture in a T25 flask (Falcon).

### Construction of an ordered transposon mutant library and generation of the mutants

The transposon donor phagemid, ΦmycomarT7, (received as a gift from Prof. Dr. Eric Rubin, Harvard School of Public Health, Boston) was propagated in *M. smegmatis* to generate phage stocks as described (43). Transduction was performed in *M. bovis* BCG, grown to OD600 1.0 as previously described (54). The library was plated on 7H10 agar plates containing 20µg/ml kanamycin, as the transposon contains the kanamycin resistance gene. The plating was performed at the appropriate dilution to obtain well-separated single colonies. Once the colonies were well grown on the 7H10 agar plates, they were manually picked using sterile toothpicks into 96 deep well plates, each plate containing 96 individual mutants. Great care was taken to only pick those colonies that were clearly clonal to avoid contamination in the subsequent ordering of the mutants.

### Ordering of the library into pools

Individual mutants were grown in 96 well plates in 200µl of Middlebrook 7H9 broth supplemented with 10% OADC and 50µg/ml kanamycin and 100µg/ml streptomycin till mid log phase (21 days) and aliquots frozen in 50% glycerol at -80°C. The remaining cultures were duplicated and grown a further 3 weeks. 10 µl culture aliquots from each row of a 96 deep well plate (12 individual mutants from the primary plate) were pooled into a single well to form a 'secondary pool' (Fig. 1B) and 10 µl culture aliquots from each column of a secondary pool plate were pooled into a single well to form a 'tertiary pool'. The cultures in the 8 secondary pool plates and the 1 tertiary pool plate were pelleted and stored at -20°C. The remaining cultures in the two sets of 96 deep well plates were stored in 50% glycerol at -80°C (one working library with the second as back-up).

### PCR Screening

For each gene of interest, a primer that hybridizes anywhere in the 1000 bases upstream or downstream of the coding sequence was designed using one of the following software packages: Primer Excel, Oligo or DNA Star. The primers were aligned against the *M. bovis* BCG genome (http://genolist.pasteur.fr/BoviList/) to ensure that the primer was as gene specific as possible. PCR was performed using this primer and a second primer that hybridized within the inverted repeats flanking the transposon (Fig. 1C). This primer reads away from the transposon and into the adjacent genomic sequence of the insertion, irrespective of its orientation with respect to the target sequence. We first screened the tertiary pools for the presence of transposon insertions in each target locus and then the secondary pools. The final 12 mutants (one row in the primary library plates containing 1 mutant per well) were then grown up from glycerol stocks and screened again to identify the individual mutant with the insertion (Fig. 1B). The region of the genome amplified by the primers was sequenced to confirm insertion in the gene of interest. The genomic DNA of the mutants was prepared as described (37) and approximately 30ng was used per PCR reaction. PCR was set-up using the Amplitaq Gold (Amersham, Uppsala) polymerase and buffer set.

### Characterization of the mutants by Southern blotting

Genomic DNA of the mutants was digested with BamHI and Pstl (restriction sites absent in the transposon) in sequence. The digested samples were blotted to Hybond N+ membranes (Amersham, Uppsala) by the neutral denaturing procedure (as per manufacturer's instructions). We hybridized the membranes with the full length transposon sequence, labeled with 250µCi [α-³²P] dCTP (Amersham, Uppsala) by random priming (Random Prime Kit, Amersham, Uppsala).

### Quantitative real time PCR

Log phase cultures of wild type *M*. *bovis* BCG and mutants (OD600 0.8-1.0), were pelleted and resuspended in TRI reagent (Molecular Research Center, Inc, USA), in a ratio of 1ml reagent per 50mg pellet. Cm baked glass beads were added and the pellet disrupted in a Retsch MM2000 bead beater (Retsch GmbH, Germany) at maximum amplitude for 3min at 4°C. The supernatant was collected by centrifugation at 13 000 rpm, 2 min, at 4°C and extracted with equal volumes of chloroform. The aqueous layer was collected and the RNA precipitated with 0.6 volumes of isopropanol. This solution was loaded on an RNeasy mini column (RNeasy Mini kit, Qiagen, USA) and further steps followed according to the Qiagen protocol for RNA extraction with the exception that, after DNase treatment, RNA was eluted in a final volume of 50µl of DEPC water. 10µl of the RNA obtained was used as template for cDNA synthesis using the iScript cDNA Synthesis Kit (Bio-Rad, USA). The cDNA obtained was diluted 1:5 and 2 µl used in a Q-PCR reaction using the Fast SYBR Green Master Mix (Applied Biosystems, USA). The samples were analyzed on the LightCycler Real Time PCR System (Roche Applied Science, Switzerland). The primers used for the analysis were designed downstream of the transposon insertion site in the mutants (as determined by sequencing). The PCR was done a total of three times for each sample and the mean results were used as the value for each sample.

### Analysis of phosphatase activity

Late log phase cultures of wild type *M. bovis* BCG and *M. bovis* BCG SapM mutant (OD₆₀₀ 4), were pelleted and the supernatans was evaluated for phosphatase activity, using pnitrophenyl phosphate (pNPP) as the substrate (52). 15 µl pNPP (5mM final) was added to 50 µL culture supernatant in a total volume of 150 µl (adjusted with 0,1 M sodium acetate pH6). Samples were incubated at 37°C for the indicated time points before stopping the reaction with 50 µL 1M Na2CO3. The relative rate of hydrolysis of pNPP was determined by quantification of released pNP and Pi at 415 nm.

### Growth curves and ultra-structure

We cultivated all strains in 10 ml of Middlebrook 7H9 broth (no antibiotic selection) at 37°C and recorded absorbance at 600nm once every 24 hrs for a period of 4 weeks. For electron microscopy, mycobacterial cells were frozen in membrane carriers in a high pressure freezer (EM Pact, Leica Microsystems, Vienna, Austria). Freeze substitution was carried out in a Leica EM Automatic Freeze Substitution (AFS). Over a period of 4 days the cells were substituted in dry acetone with 1% osmium, 0.2% glutaraldehyde and 0.1% uranyl acetate (UrAc) as follows: -90°C for 26h, 2°C /h temperature increase over 15 h, -60°C for 8 h, 2°C /h temperature increase over 15h, -30°C for 8 h. At -30°C the carriers were rinsed 3 times with acetone for 20 min each time. At the end cells are warmed up slowly to 4° C. Subsequently, we impregnated cells stepwise in Spurr's resin, which we polymerized at 70°C for 9 h. We cut ultra-thin sections of gold interference color using an ultra-microtome (ultracut E, Reichert-Jung) and collected them on formvar-coated copper slot grids. We stained the sections for 1 h with 50:50 (v/v) glutaraldehyde 4% and a 0.2M cacodylate buffer containing 1,5g/L ruthenium red followed by rinsing in 0,2M cacodylate buffer. We subsequently stained the sections for 4 h with a freshly prepared solution of 50:50 (v/v) osmium tetroxide 4% in ddH2O and 0,2M cacodylate buffer containing 1,5 g/L ruthenium red, followed by rinsing in the cacodylate buffer and then ddH2O. We visualized the grids with a JEOL 1010 TEM operating at 90 kV.

### Cyanovirin-N binding and mannosidase treatment

1 mg (wet weight) of *M*. *bovis* BCG wild type or mutant cells (culture OD600 ∼ 1.0) were incubated with 1µl of Cyanovirin-N (702.9 µg/ml, received as a gift from Dr. B O'Keefe, NIH, Bethesda) coupled to Alexa-488 (according to manufacturer's instructions, Molecular Probes) in 100µl of lectin buffer (50mM Tris/Cl pH 7.5, 150mM NaCl, 1mM MgCl2, 1mM CaCl2, 1mM MnCl2) for 1 hr on ice and assessed via flow cytometry on a FACS Calibur (Becton Dickinson) flowcytometer. Just before analysis, 15µM of propidium iodide (PI) was added into the sample. For the α-1,2-mannosidase treatment, 1mg of cells were incubated overnight at 37°C in 100mM ammonium acetate buffer (pH 5.0) with 33ng of recombinant *Trichoderma reesei* α-1,2-mannosidase in 50 uL (LPS-free, produced in *Pichia pastoris* inhouse) (34). The cells were then washed once with the buffer and the Cyanovirin-N staining performed as given above, followed by flow cytometric analysis.

### CS-35 labeling

1 mg (wet weight) *M*. *bovis* BCG wild type or mutant cells (culture OD600 ∼ 1.0) were incubated with 1:100 diluted CS-35 monoclonal antibody (TBVTRM, Colorado State University) (22) for 15 min in 1x PBS pH 7.4, in a reaction volume of 100µl, following which anti-mouse IgG-PE (Phycoerythrin, Molecular Probes, USA) was added and additionally incubated for 15 min. The cells were briefly washed in 1x PBS and analyzed via flow cytometry.

### Isolation of LM and LAM

We isolated lipoglycans on a small scale following the protocol described (23). Briefly, 50 mg of cells (wet-weight) from a culture at OD 1.0 (or 0.4-0.5 in case of low OD cultures), were extracted in chloroform: methanol: water (10: 10: 3) for 30 min at 50°C. The pellet was then incubated with buffer-saturated phenol for 2 hrs at 80°C and extracted with equal volumes of chloroform. The aqueous phase was collected and dialyzed against water.

### SDS-PAGE and in-gel lectin blot (In-gel Western)

The lipoglycan samples (derived from 10 mg of cells) were analyzed on 12% SDS-PAGE gels without stacking gels followed by Periodic acid-Schiff staining (48). The in-gel western was performed by incubating the gels for 1 hour in blocking buffer (In-gel western kit, Westburg, Leusden, The Netherlands) followed by an overnight incubation in lectin buffer with Cyanovirin-N (received as a gift from Dr. B O'Keefe, NIH, Bethesda) coupled to Alexa-633 (according to manufacturer's instructions, Molecular Probes) at 4°C. Shorter incubations times were tried but resulted in very low signal strength. The gel was washed thoroughly for 1 hr in lectin buffer and visualized at 700nm using the Odyssey Infrared Imaging System (Westburg, Leusden, The Netherlands) and read-outs made in grey-scale.

### DSA-FACE analysis of M. bovis BCG and isolated LM and LAM samples

Lipoglycan samples were subjected to mild acid hydrolysis by boiling them for 5 min in 10 µl of 10mM HCl, followed by neutralization with an equal volume of 10mM NaOH. We desalted the samples using porous-graphitized-carbon (Nu-tip) tips (SunChrom GmbH, Friedrichsdorf, Germany) as described previously (46). We vacuum evaporated the desalted sample and labeled then with APTS, followed by analysis by capillary electrophoresis on the ABI 3130 DNA sequencer (Applied Biosystems, Foster City, USA) as described (30). 1.0 µl of the APTS labeled samples were then digested overnight at 37°C in 0.1 µl of 100 mM ammonium acetate buffer (pH 5.0), with 6 ng of recombinant *T. reesei* α-1,2-mannosidase and the volume made up to 2 µl with milli-Q water. The samples were then analyzed by capillary electrophoresis as above.

### Lipid extraction and mycolic acid analyses by thin layer chromatography.

We grow the mycobacterial strains (5 ml to an OD600nm of 0.4 in the presence of 0.05 % Tween 80 in Middlebrook 7H9 broth (Difco) and 10% ADC (Difco) with appropriate antibiotics (wild type: 100 µg/ml Streptomycin, and mutants: 100 µg/ml Streptomycin and 50 µg/ml Kanamycin) at 37ºC. At this point 1 µCi/ml [1,2-14C]acetate (57 mCi/mmol, GE Healthcare, Amersham Bioscience) was added to the cultures, which were further incubated at 37°C for 24 h. The [14C]-labelled cells were harvested by centrifugation at 2000 x g, washed with PBS and processed as described below.

We initially resuspended the [14C] labelled cells in CH3OH/0.3 % NaCl (2 ml, 100:10, v/v) and mixed them with 1 ml of petroleum ether (60-80oC) for 15 min. The upper petroleum ether layer was removed and a further 1 ml of petroleum ether added, followed by further mixing for 15 min. The petroleum ether extracts were combined and evaporated under nitrogen using a heating block. The dried apolar lipid extract was resuspended in 200 µl of CH2Cl2 prior to thin-layer chromatography (TLC) and autoradiography. Polar lipids were extracted by the addition of CHCl3/CH3OH/0.3% NaCl (2.3 ml, 9:10:3, v/v/v) to the lower methanolic saline phase and mixed for 1 h. The mixture was centrifuged and the pellet reextracted twice with CHCl3/CH3OH/0.3% NaCl (750 µl, 5:10:4, v/v/v). CHCl3 (1.3 ml) and 0.3% NaCl (1.3 ml) were added to the combined extracts and the mixture centrifuged. The lower layer containing the polar lipids recovered and dried. The polar lipid extract was resuspended in CHCl3/CH3OH (2:1, v/v). We applied the apolar and polar lipid extracts (50,000 cpm each) to the corners of 6.6 x 6.6 cm plates of silica gel 60 F254 (Merck 5554) TLC plates. The plates were developed in a series of solvent systems, designed to cover the whole range of lipid polarities as described. For apolar lipid extracts these systems were named systems A-D and for polar lipid extracts; system E. System A TLCs were run thrice in direction 1 (petroleum ether 60-80: ethyl acetate 98:2) and once in direction 2 (petroleum ether 60-80: acetone 98:2). System B TLCs were run thrice in direction 1 (petroleum ether 60-80: acetone 92:8) and once in direction 2 (toluene: acetone 95:5). Systems C, D and E TLCs were run once in each direction using, for system C CHCl3:CH3OH (96:4) in the first direction and toluene: acetone (80:20) in the second, for system D CHCl3:CH3OH:H2O (100:14:0.8) in the first direction and chloroform: acetone:CH3OH:H2O (50:60:2.5:3) in the second and for system E CHCl3:CH3OH:H2O (60:30:6) in the first direction and CHCl3:acetic acid:CH3OH:H2O (40:25:3:6) in the second. Autoradiograms were produced by overnight exposure of Kodak X-Omat AR film to the plates to reveal [14C]-labeled apolar and polar lipids. For extraction of mycolic acid methyl esters (MAMEs), the delipidated cells from the 5ml cultures were subjected to alkaline hydrolysis with 5% aqueous TBAH at 100°C overnight, followed by the addition of 4 ml CH2CI2, 500 µl CH3I, 2 ml water, followed by mixing for 30 min. The upper aqueous phase was discarded following centrifugation, and the lower organic phase washed thrice with water and evaporated to dryness. The resulting MAMEs were dissolved in diethyl ether, insoluble residues were removed by centrifugation, and the ether solution evaporated to dryness and redissolved in 200 µl of CH2CI2. Equivalent volumes (5 µl) of the resulting solution of MAMEs was subjected to TLC with silica gel plates (5735 silica gel 60F254; Merck), developed in petroleum ether-acetone (95:5). Autoradiograms were produced by overnight exposure of Kodak X-Omat AR film to the plates to reveal [¹⁴C]-labeled MAMEs.

### Macrophage cells

Isolation and routine culture of the murine macrophage clone Mf4/4 has been described previously (Desmedt et al, 1998). The cells were cultured in RPMI 1640 medium supplemented with 10% fetal calf serum, L-glutamine (0.03%), 0,4mM sodiumpyruvate, 0,1mM non-essential amino acids and 50µM β-mercaptoethanol.

### Generation of bone marrow-derived dendritic cells

Bone marrow cells were cultured for 8 days in DC culture medium (RPMI 1640 containing GlutaMAX-I, supplemented with 5% (vol/vol) FCS, 50µM β-mercaptoethanol and 20 ng/mL GM-CSF (produced in house).

### Laboratory animals

Female C57BL/6 mice and BALB/c mice were purchased from Janvier (France) and were housed under specific pathogen-free conditions in microisolator units. Animals were used at 7-8 weeks at the beginning of the experiment. All experiments were approved by and performed according to the guidelines of the animal ethical committee of Ghent University and SIPH, Belgium.

### M. bovis BCG vaccination and M. tb challenge

Female BALB/c mice were vaccinated subcutaneously with 10⁵ CFU of the parental or mutant *M*. *bovis* BCG strains grown on 7H9-OADC medium (30 mice/group). Mice were challenged intravenously three months after BCG vaccination with 5.10⁴ CFU luminescent *M*. *tb* H37Rv, grown for two weeks on liquid 7H9-OADC medium (Tanghe et al, 2001). Bacterial replication in spleen and lungs was monitored by luminometry. Mice were sacrificed on week 2, 4 and 8 post-infection (4-5 mice/group), as indicated in the text. Luminescence was measured in a Turner Design Luminometer as flash emission (15 sec. integration time) using 1% n-decanal (Sigma) in ethanol as substrate. In this assay, only live bacteria are enumerated, because emission of light is dependent on the presence of reduced flavin mononucleotide (FMNH2), co-factor which is only found in living cells. At least 10 additional mice/group were monitored for weight loss and mortality in a long term survival experiment as reported before (Romano et al, 2006).

### Cytokine production following splenocyte and lung cell stimulations

BALB/c mice were infected intravenously (106 cells/mouse in 200µl in tail vain) with the *M*. *bovis* BCG wild type or mutant. Four weeks after infection, mice were killed by cervical dislocation and lungs and spleens were removed aseptically. Cells were isolated by use of a loosely fitting Dounce homogenizer, washed and adjusted to a concentration of 2.106 cells/mL, and grown in round-bottom microwell plates (Nunc), in RPMI 1640 medium, supplemented with 10% fetal calf serum, L-glutamine (0.03%), 0,4mM sodiumpyruvate, 0,1mM non-essential amino acids and 50µM β-mercaptoethanol.. Recombinant Ag85a (5µg/mL; Colorado State University) and the synthetic 20-mer peptide p11 (Ag85A99-118aa, dominant CD4 T cell epitope (Denis et al, 1998) were used to stimulate the cells in a volume of 100µL to 100µL of cell suspension (in triplicate). Cells were incubated at 37°C in a humidified CO2 incubator, and supernatants were harvested after 72h. Supernatants were frozen at -20°C until assay. The IFNγ concentration was determined by ELISA.

### Flowcytometric analysis of binding/uptake of mycobacteria by macrophages and BMDCs

Mf4/4 macrophages were kept in 24-well plates at 37°C to reach 2 x 10⁵ cells/well at the time of infection. Log phase *M. bovis* BCG wild type and mutants (OD∼0,8-1,2) were FITClabeled by resuspending the bacterial pellet (10 mg wet weight) in 200µL of a FITC solution (0,1 mg/mL FITC isomer 1 (Sigma Aldrich) diluted in 0,1 M NaHCO3, pH 9) and incubating this bacterial suspension at 25°C for 1h with gently shaking. Following this incubation period, bacteria are washed in PBS until the supernatant is clear of residual FITC. The bacteria were used for infection of Mf4/4 cell monolayers or BM-DCs at an MOI of 10 or 2 respectively and incubated for the indicated time points at 37°C. Macrophages were washed after infection, fresh medium was added and the infected cells were further incubated at 37°C. Before measurement, macrophages were washed 2 times with PBS and detached in non-enzymatic dissociation buffer (Gibco, Invitrogen NV). Infected BM-DCs were centrifuged for 10 min at 150xg and washed in PBS. The amount of FITC-positive macrophages/BM-DCs was determined in FL1 on a FACScalibur flow fluorocytometer (Becton Dickinson) equipped with a 488 nm argon ion laser.

### Infection of macrophages with M. bovis BCG and evaluation of survival and phagolysosome fusion

Macrophages were infected as described above. The number of CFU after the indicated time points was determined. For evaluation of phagolysosome formation by confocal microscopy, the cells were infected with FITC-labeled BCG. At 3h pi, cells were incubated with Lysotracker Red DND-99 (200nM) for 30 min. Cells were washed, and subsequently placed in medium without Lysotracker. Image fields were picked at random under transmission light (non-fluorescent) and each image field contained at least 20 macrophages. For experimental details, see below.

### Measurement of oxidant activity

Log phase M. *bovis* BCG wild type and mutants were used to infect Mf4/4 cells (MOI 10). At the indicated time points, cells were washed with PBS and incubated for 30 min with 100 nM CM-H2DCFDA (Molecular Probes). Afterwards, cells were washed in PBS again and analyzed for intracellular fluorescence on a FACScalibur flow cytometer equipped with a 488 nm argon ion laser.

### Induction of autophagy

To analyze autophagy following *M*. *bovis* BCG infection of macrophages or BM-DCs, the latter were infected with *M*. *bovis* BCG wild type versus mutants at respectively MOI 10 or MOI2 for 4h or 24h. Autophagy was also induced with rapamycin as previously described (Gutierrez et al, 2004). Cells were lysed in NP-40 lysis buffer (1% NP-40, 200mM NaCl, 5mM EDTA, 10% glycerol, 10mM Tris-HCl pH 7.5, protease inhibitors). We analyzed 40µg total protein on western blot (17,5% SDS-PAGE), which was revealed with monoclonal anti-LC3 antibody clone 5f10 (Nanotools, Enzo Life Sciences) and anti-mouse-HRP (Amersham).

LC3-I (18kD), LC3-II (16kD).

### Lymph node DC trafficking and activation

8-week-old female Balb/cJ mice (Janvier, France) were infected subcutaneously at the base of the tail with 1.106 CFU *M. bovis* BCG wild type and *M*. *bovis* BCG SapM mutant in 100µL PBS (5 mice/group). Day 1 and day 7 post-infection, five mice per group were sacrificed; inguinal and brachial/axillary lymph nodes were removed aseptically, and homogenized in RMPI with 1% collagenase (shaking 45 min at RT). Cells were prepared and labeled with CD11c-APC, MHCII-FITC, CD80-V450, CD40-PE, CD8a-PerCP, CD86-PE-Cy7 and analyzed on a LSRII flowcytometer (BDTM). Analysis was performed using FACSdiva software.

### Statistical analyses

For statistical analysis, results obtained in milliRelative Light Units (mRLU) were converted to mean log10 mRLU/organ. Survival data were analyzed using the method of Kaplan-Meier and Logrank test in Prism version 4.0 (GraphPad). The other data were analyzed with oneway ANOVA or Kruskal-Wallis followed by Bonferroni's Multiple Comparison Test or Mann-Whitney U test as indicated. P values < 0.05 were considered statistically significant.

### Cells

The macrophages used in these experiments are derived from a clonal, immortalized C57BL/6-derived population that both functionally and phenotypically expresses features characteristic of mature macrophages. Thus, these Mf4/4 cells express the surface molecules BM-8, F4/80, Mac-1, Mac-2, and CD14 that have been described for mature macrophages; they can exert receptor-mediated phagocytosis and produce IL-1, IL-6, IL-12, and TNF in response to LPS, but not to IFN-γ. Moreover, the Mf4/4 cells express increased levels of MHC class II Ags after treatment with **IFN-γ** and concomitantly acquire the capacity to present exogenous Ag to CD4+T cells. These features demonstrate that, despite their transformed state, the Mf4/4 cells retain their macrophage-specific constitutive and inducible functions. We could also demonstrate the expression of mannose receptor by competing the binding of FITC-mannose BSA with α-methyl-D-mannopyranoside (data not shown).

### Analysis of uptake of M. bovis BCG by confocal microscopy

Mf4/4 cells, grown on 1-well glass bottom dishes (Willco Wells B.V., Amsterdam, The Netherlands) and labeled with Cell tracker red (Molecular Probes, 10µM final, pre-incubated for 30 min in serum-free medium), are infected with FITC-labeled *M*. *bovis* BCG (MOI 10). After 3h at 37°C, confocal images were acquired with a 63 x HCX PL APO 1,4 NA OIL UVcorrected objective on a Leica TCS sp5 AOBS laser scanning inverted DMi6000 microscope. FITC was excited with the 488nm line of a Multi Argon laser and Cell tracker red with a 543nm HeNe. Sequential scanning was performed to exclude cross excitation bleed through. FITC and Cell tracker red were detected with the respective emission bandwidths: 500nm till 540nm and 550nm till 700nm. Images were taken with a 512x512 pixel resolution, using a 4x zoom, resulting in a pixel size of 123nm. 23 z-layers were recorded with a step size of 0.26 µm.

### Electron microscopy of macrophages infected with M. bovis BCG wild type and mutants

Mf4/4 cells were infected with *M*. *bovis* BCG wild type and mutants (MOI 10) for 3h. Following infection, the cells were washed 2 times in PBS, fixed in 4% paraformaldehyde and 2.5% glutaraldehyde in 0.1 M NaCacodylate buffer, pH 7.2 for 4 hours at RT followed by fixation O/N at 4°C. After washing 3 times 20 min in buffer, cells were dehydrated through a graded ethanol series, including a bulk staining with 1% uranyl acetate at the 50% ethanol step followed by embedding in Spurr's resin. Ultrathin sections of a gold interference color were cut using an ultramicrotome (Leica EM UC6), followed by a post-staining with uranyl acetate and lead citrate in a Leica EM AC20 and collected on formvar-coated copper slot grids. They were viewed with a transmission electron microscope 1010 (JEOL, Tokyo, Japan).

### Evaluation of phagolysosome fusion

Confocal images were acquired with a 63 x HCX PL APO 1,4 NA OIL UV-corrected objective on a Leica TCS sp5 AOBS laser scanning inverted DMi6000 microscope equipped with a 37°C incubation chamber (Leica

Microsystems, Wetzlar, Germany). FITC was excited with the 488nm line of a Multi Argon laser and emission light was captured between 500 and 540 nm. Lysotracker Red (Molecular Probes) was excited with a 543nm HeNe laser line and emission light was captured between 560 and 620 nm. Sequential scanning was always performed, scanning zoom was set at 2.0 and z-series image stacks were collected over the complete depth (in z-axis) of the macrophages. Depending on the experiment, two different imaging setups were used: in the first (low resolution) setup, the image size was 512x512 pixels and z-intervals were set at 1.0 µm, resulting in 240x240x1000 nm3 voxels. In the second and third (high resolution) imaging setups, 1024x1024 pixel images were taken with 0.25 µm z-stepsize, resulting in 120x120x250 nm3 voxels. Phagolysosome fusion was evaluated by analyzing colocalization between FITC and lysotracker Red with the JaCoPplugin of the ImageJ 1.3i public domain imaging software (Abramoff et al, 2004; Rasband & ImageJ, 2008). Thresholded Manders' M1 coefficients were used to measure the amount of bacteria that had undergone phagolysosomal fusion and are defined as the ratio of the "summed intensities of pixels from the FITC channel for which the intensity in the lysotracker Red channel is above zero" to the "total intensity in the FITC channel" (Bolte & Cordelières, 2006). Thresholds for the Manders' M1 coefficient were set automatically without user intervention based on the image histogram. Three independent experiments were performed in which 20 (1st experiment; low resolution setup) or 10 (2nd and 3rd experiment; high resolution setup) z-series image stacks were taken per strain.

### Replication of M. bovis BCG following infection of mice

8-week-old female Balb/cJ mice (Janvier, France) were infected intravenously with 1.10⁶ CFU *M*. *bovis* BCG wild type and mutants in 200µL PBS. 2 weeks, 4 weeks and 12 weeks post-infection, five mice per group were sacrificed; lungs and spleens were removed aseptically, and homogenized in PBS. Neat, 1/10 and 1/500 dilutions were made, 100µl was plated in duplicate on 7H10-OADC agar, and the colonies were counted at 21 days. The CFU counts were based on the highest dilution showing distinct colonies.

### Granuloma formation and cytokine measurements following infection of mice with M. bovis BCG

C57BL/6 mice were anesthetized and infected intravenously with 1.10⁶ CFU, or intratracheally with 0.5.106 CFU, *M*. *bovis* BCG wild type and mutants. After 3 and 4 weeks respectively, five mice per group were sacrificed, livers and lungs were removed aseptically, and fixed in 3,7% paraformaldehyde prior to embedding in paraffin for thin sectioning. General morphology and inflammation were examined by hematoxylin/eosin staining (Merck, VWR international). Quantitative studies were performed by direct microscopic examination and analysis with ImageJ software. Bronchoalveolar lavage (BAL) was performed 4 weeks post-infection and cytokine levels in BAL fluid were determined using Cytometric Bead Array and Flex sets according to manufacturer's instructions (BD Biosciences).

### Cytokine production following infection of BM-DCs with M. bovis BCG wild type versus SapM::T

BM-DCs were infected for 6h with *M. bovis* BCG wild type versus mutants. Following infection, the BM-DCs are collected by centrifuging at 150xg, 10min, 4°C. Cells are washed in PBS and brought back in culture for 24h and 48h. At the indicated time points, supernatant was removed and analyzed for the presence of cytokines by flowcytometry (BioPlex Pro Cytokine Express Assay, Biorad).

### Coculture of BM-DCs and iNKT hybridomas

Wild type and CD1d-/- BM-DCs (1.10⁶ cells) were infected with *M. bovis* BCG wild type versus mutants for 24h (MOI2). Following infection, the cells were washed with PBS and put in coculture with the iNKT cell hybridoma 2C12 (Brossay et al, 1998a; Brossay et al, 1998b) (50.103 BM-DCs:25.103 iNKT). As a control, BM-DCs were loaded with α-GalCer (100ng/mL per 1.106 cells) and incubated at 37°C for 2 hours before setting up the coculture with the 2C12 cells. At the indicated time points, supernatans were collected and IL-2 and IL-12p40 production was analyzed by flowcytometry (BioPlex Pro Cytokine Express Assay, Biorad). Cells were labeled with panel 1 (CD11c-PerCP-Cy5.5, MHCII-FITC, PD-L1-PE, CD86-PE-Cy7, CD80-V450, CD40-APC), panel 2 (CD11c-PerCP-Cy5.5, MHCII-FITC, CD1d-PE) and panel 3 (CD11c-PerCP-Cy5.5, TCRβ-FITC, CD1d aGalCer tetramer-PE, CD69-V450) and analyzed on a LSRII flowcytometer (BDTM). Analysis was performed using FACSdiva and Flowjo software (Tree Star, Inc.).

### Example 1. Generation and screening of M. bovis BCG mutants

Transposon delivery by mycophages is a highly efficient method for the generation of saturation-mutagenesis libraries of mycobacteria (54). The method makes it possible to generate a wide variety of mutants in an isogenic background, which is ideal for comparison of the effects of different gene mutations. The insertion of the transposon into a gene most often leads to its inactivation and reversion frequencies are usually very low (47). The transposon can also be used to deliver additional markers like antibiotic resistance genes. It also allows for the transposon flanking genomic regions to be amplified and identified by sequencing (27). Transposon mutagenesis has been successfully used in both fast and slow growing Mycobacterial strains (1, 10, 32, 51). Here, we have created a 96 x 96 clone, ordered M. *bovis* BCG transposon insertion mutant library using the Himar1-derived minitransposon delivered by the temperature sensitive MycomarT7 phage (49).

Moreover, we have developed a PCR-based screen of this ordered library to rapidly identify insertions in targeted open reading frames of interest. The system was validated by screening the library for insertions in genes involved in cell wall component biosynthesis or of reported importance in mycobacterial virulence.

### Library construction and ordering

*M. bovis* BCG 1721 (36), a derivative of *M*. *bovis* BCG Pasteur, carrying a non-restrictive rpsL alteration resulting in streptomycin resistance, was used as parental strain in the construction of the ordered transposon insertion library in *M. bovis* BCG by using a Himar1-based mariner transposon and mycomarT7 donor phagemid (56) (Fig. 1A). Mutants were selected for double antibiotic resistance (kanamycin and streptomycin). The library yielded about 100,000 mutants, 9216 (96 x 96 well plates) of which were hand-picked to create an ordered library as previously described (29) (Fig. 1B). Briefly, the single clones from each row of a 96-well plate were pooled to form a 'secondary pool' (Fig. 1B) and aliquots from each column of this secondary pool plate were pooled into a single well to form a 'tertiary pool'. Due to the property of Mycobacteria to clump and thus frequently form fused colonies, robotic clone picking would be very difficult. Hand picking of clearly individual clones from the plates was deemed necessary to reduce the frequency of mixed culture in the ordered library. The genome size of *M. bovis* BCG is 4.4Mb and as the transposon can insert in every TA sequence (28), theoretically, an insertion is expected in our library every ∼460bp.

### PCR Screening

Using a primer hybridizing to the inverted repeats at either ends of the transposon, and genespecific primers either upstream or downstream (and occasionally in) the ORF of the target gene, we performed PCR screening of the ordered library (Fig. 1C). The library has been manually ordered into a series of pools in order to enable efficient PCR screening. Due to this, only two rounds of PCR (first 96 reactions, then 8 reactions) are needed to identify the 12-clone row in which the desired mutant is present (Fig. 1B). These 12 clones are then cultivated and genomic DNA is prepared for a final round of PCR, to identify the mutant. If a primer is identified which gives gene-specific amplification in PCR round one, then the entire screen is completed in about 4-5 weeks (with most of that time required to grow the 12 final cultures). We completed screens for insertions in 19 ORFs (annotated not to be essential for growth *in vitro* (based on the presence of transposon mutants in these genes in *M. tuberculosis* libraries that were analysed through TRASH (55)). Our screen was focused on genes involved in (Man)LAM biosynthesis and/or in interference with the phagosome-lysosome fusion event. The total screened size of these ORFs was ∼11,120 bp. In these ORFs, we found a total of 22 transposon insertions (1/500 bp approximately), providing mutants in 15 of the 19 ORFs. Not surprisingly, 3 out of 4 unsuccessful screenings were for the 3 smallest ORFs (less than 750 bp). In this screening, primer design was critical. For most screenings; several gene-specific primers had to be tested for one to be found that yielded gene-specific amplification. This was mainly due to the fact that the *M. bovis* BCG genome has a very high GC content, with an average percentage G + C of over 65% (16). This made it difficult to design primers that did not bind non-specifically elsewhere in the genome.

### Example 2. Biochemical characterization of transposon insertion mutants

### Southern analysis for confirmation of single transposon insertion

The sequence-confirmed mutants were further characterized by Southern blotting with the full length transposon as probe, to confirm the presence of a single transposon insertion in the genome. This is essential in order to ensure that the observed phenotype of a mutant is due to a transposon insertion, leading to the inactivation of the gene of interest alone. Apart from three mutants (Fig.2A) all the rest showed a single transposon insertion.

### Mutants in Mb1661c, Mb2203, Mb2196 and SapM

We obtained 2 mutants each for genes Mb1661c, Mb2203 and Mb2196 in our library screen. One of the mutants for the gene Mb2203 and for Mb2196 showed multiple transposon insertions when analyzed by southern blot analysis and was removed from further use. Among the two mutants for gene Mb1661c, we decided to work with the mutant where the insertion of the transposon was closer to the 5' end of the coding sequence (CDS), as proximity to the start of the CDS is even more likely to inactivate its functionality. Besides mutants in ManLAM biosynthesis, we also selected the mutant in the secreted acid phosphatase SapM as a control to assess any effects which the mere transposon presence might have on cell wall composition. For the gene SapM, we obtained only 1 mutant (Table 1). The site of transposon insertion in the 4 finally selected mutants was confirmed by cloning the fragments obtained by PCR screen using the Topo TA^{®} Cloning Kit (Invitrogen, USA) and sequencing the clones to determine the exact position of transposon insertion into the open reading frame (Table 1).

**Table 1 - List of mutants screened for by PCR in the ordered M. bovis BCG transposon insertion mutant library and described in this study**

| Gene in H37Rv | Homologue AF2122/97 | Length (bp) | Length (aa) | (putative) function | Screening primer sequence (5' to 3') | No of mutants | Insertion at (in bp from start codon) |
|---|---|---|---|---|---|---|---|
| ***Rv1635c*** | ***Mb1661c*** | ***1671*** | **556** | ***Man-T* mannose *capping of arabinan*** | ***CACCACGGTCTTATAGGCGC (SEQ ID NO: 19)*** | **2** | ***470 and 344 (mutant ii)*** |
| ***Rv2181*** | ***Mb2203*** | ***1284*** | ***427*** | ***Man-T addition to mannan core and caps*** | ***CCGTTCACCTCCACTG (SEQ ID NO: 20)*** | **2** | ***591* and 731 (mutant ii)*** |
| ***Rv3310*** | ***Mb3338*** | **900** | **299** | ***PI3P phosphatase SapM*** | ***ATCGTGGCTCGGTCCCCTAAA (SEQ ID NO: 21)*** | ***1*** | **-*8*** |
| ***Rv2174*** | ***Mb2196*** | ***1551*** | ***516*** | ***Man-T in synthesis of Man21-34*** | ***CGGTGACGACCTAAAGTCGG (SEQ ID NO: 22)*** | **2** | ***40** *and 6*** |

### The mutants show strong reduction in transcription of gene of interest and the mutant in SapM shows decreased phosphatase activity

Quantitative Real Time PCR (qRT-PCR) was performed on the wild type *M*. *bovis* BCG 1721 and the mutants to compare expression levels of the genes of interest. RNA was extracted from mid-log phase cultures and cDNA synthesized using standard protocols. Q-PCR was performed with 16S rRNA as a reference. All mutants have strongly decreased abundance of the transcripts from the mutated gene as compared to the wild type. The SapM mutant had almost no detectable transcription of the SapM gene (Fig. 2B) probably due to the insertion just upstream of the ORF, effectively separating the promoter from the ORF. Inactivity of SapM was also confirmed by a decreased phosphatase activity in the supernatans of the *M*. *bovis* BCG SapM::T culture compared to phosphatase activity in the supernatans of a wild type culture (Fig. 2C). Residual phosphatase activity in the supernatans of the SapM mutant can still be due to the presence of other phosphatases (3, 9).

### Mutants have unaltered ultra-structure and in vitro growth rate

As the LAM molecule forms part of the cell wall (it is anchored in the cell membrane and extends through the inner and outer cell walls into the capsule), we wanted to assess whether the mutants in LAM biosynthesis show differences in the cell wall ultra-structure as compared to the wild type. However, there were no overt differences in cell wall ultra-structure, as evident from the electron microscopy of thin sections stained to enhance the contrast of polar lipids and polysaccharides in the cell wall (data not shown). The mutants also show no retardation in growth in liquid medium as observed in the growth curve analysis (Fig. 3A). However, we did observe slight growth retardation in mutant Mb1661c when cultured on solid media (data not shown). All cultures grew at the same rate, although under our static growth conditions doubling times increased with time and culture density (Fig. 3B). Doubling times were around 20-24 hrs until an OD of 1-1.2 was reached, beyond which the doubling times steadily increased to 48-96 hrs at higher ODs (∼6) (for mutants as well as wild type).

### Cyanovirin-N staining and DSA-FACE analysis reveals differences in the α-1,2- mannose cap structures in the Mb2203 and Mb1661c mutants

We analyzed the density of -1,2-oligo-mannosyl glycotopes by flow cytometry with Cyanovirin-N lectin, which specifically binds oligo-α-1,2-mannose structures (60). As a binding specificity control, the mycobacterial cells were also treated with recombinant α-1,2-mannosidase prior to the lectin staining. The mutants in Mb1661c and Mb2203 showed lower staining when compared to the wild type indicating that these mutants display less α-1,2-mannose linkages (data not shown). The mutants in SapM and Mb2196 show a similar staining profile as the wild type, indicating that they have similar levels of α-1,2-oligomannoside linkages in their cell wall structure. These results are strengthened by the observation that when the wild type strain or the SapM or Mb2196 mutants are treated with α-1,2-mannosidase, it shows a reduction in subsequent Cyanovirin-N staining (data not shown). This reduction is not observed in the mutants Mb1661c::T and Mb2203::T. This shows that, while the wild type strain has α-1,2-mannose linkages available for the enzyme to act upon, the mutants Mb1661c::T and Mb2203::T do not.

To assess whether the reduced Cyanovirin-N staining in Mb1661c::T and Mb2203::T is reflected in the ManLAM capping structures, DNA sequencer-aided fluorophore-assisted carbohydrate electrophoresis (DSA-FACE) (31) was used to study the cap structures of ManLAM. We isolated the lipoglycan fractions from the cells using an extraction method which can be performed at small scale and yields all lipoglycan species, rather than ManLAM only, making it more suitable for comparative analysis of mutants that may be affected in the exact structure of ManLAM. The preparation thus obtained is less pure than the standard ManLAM purified through a lengthy, very selective procedure (62), but to the benefit of enabling more comprehensive lipoglycan profiling. Upon this isolation we performed mild acid hydrolysis of the lipoglycans, which causes the acid labile α-1,5-ara*f* linkages to be hydrolyzed, whereas the α-1,2- and α-1,6-mannose linkages are acid stable and are thus not hydrolyzed (41). This results in the release of the α-1,2- oligomannoside capped terminal structures of ManLAM. These samples were analyzed before and after α-1,2-mannosidase digestion. For the wild type and the mutants SapM::T and Mb2196::T, several peaks were sensitive to α-1,2-mannosidase digestion (data not shown). These peaks had the same mobility as the α-1,2-mannosidase-sensitive peaks in the profile of the highly purified standard ManLAM, identifying them as the α-1,2-mannose cap bearing acid resistant fragments of this molecule. The decrease in size by one or two hexose units corresponds to removal of one or two α-1,2-mannose residues from these ManLAM fragments. In the mutant Mb2203::T, no such α-1,2-mannosidase-sensitive structures are found, and instead, a profile is found that is consistently identical to the post- α-1,2-mannosidase profile of the wild type. For Mb1661c::T, much lower levels of the α-1,2-mannosidase sensitive structures were found than in wild type, SapM::T or Mb2196::T. However, these structures were still present, indicating that there must be another yet to be identified mannosyltransferase which can very partially complement the Mb1661c mutation. Strikingly, a regular series of peaks was observed with a Poisson-like intensity distribution with increasing degree of polymerization. This was reminiscent of the acid hydrolysate of an α-glucan, as is used frequently as size standard in oligosaccharide analysis (data not shown). Indeed, we confirmed through amyloglucosidase digestion that this is an α-1,4-linked gluco-oligosaccharide ladder. Such a glucan has been reported to be a major constituent of the capsular layer of the mycobacterial cell wall (44). Thus, our sample preparation procedure likely leads to co-purification of part of this glucan. In fact, its presence allows estimating the relative abundance of ManLAM-derived oligosaccharides in the preparations from different mutants. Therefore, we interpret our findings of lower abundance of cyanovirin ligands on the mycobacterial cell wall as the compound result of both a somewhat lower ManLAM abundance and a lower but not absolute deficiency in synthesizing the oligo-α-1,2-mannosyl cap structures.

### The mutants do not show major differences in cell wall arabinan-containing components

The monoclonal antibody CS-35 specifically binds α-1,5-linked arabinofuranosides (ara*f*) (22). This glycotope is present in the arabinan side-chains of the LAM molecule and in arabinogalactan. Staining with this antibody will thus yield some information on alterations in the arabinan content of the cell wall, in the mutants. The flow cytometry profiles of all the mutants with CS-35 antibody show no difference from wild type profiles (data not shown).

### Mutants Mb1661c::T and Mb2203::T display lower molecular weight and noncyanovirin-N-binding LAM

SDS-PAGE analysis of the purified lipoglycan samples from the mutants versus wild type was performed in order to determine the size of LAM and LM from the mutants when compared to wild type (data not shown). The analysis revealed that the mutants Mb1661c::T and Mb2203::T have LAM that is of a smaller size, and the Mb2203::T LAM is the smallest. The sizes of LAM from the mutants SapM::T and Mb2196::T are the same as that of wild type. The LM from all the samples appears to be of the same size perhaps with the exception of the Mb2203::T mutant, where it appears larger. An in-gel western on these LAM samples was performed with the lectin Cyanovirin-N-Alexa- 633 (data not shown). The results show that while the lectin binds the wild type LAM and that from the mutant SapM::T, it does not bind the LAM samples from the three LAM mutants indicating that these mutants lack the motifs for the lectin to bind under these conditions.

### Analysis of LAM from low OD cultures

Both *in vitro* and *in vivo* infection studies using mycobacteria described in the literature are typically carried out with cultures growing in early to mid log phase at ODs of 0.3-0.6 (referred to herein as low OD). Most of the above-observed biochemical analyses have been carried out with cultures growing at OD600 1.0. We have observed that cells at this culture density have higher *in vitro* infection efficiencies of macrophages than at low OD. We wanted to analyze the difference, if any, in the LAM content between the cultures growing at low OD600 0.5 and those grown to OD600 1.0. In order to do this, we grew cultures from same glycerol stocks to the two different ODs, isolated the lipoglycans, and analyzed them on SDS-PAGE followed by silver-PAS staining. We observed that at low OD, the amount of LAM produced is considerably less compared to the amount isolated from cells at a higher OD (data not shown). We also observed a presence, in greater amount, of the LM/LAM precursor, PIMs at low OD. We did not observe any PIMs in the lipoglycan samples from cultures at OD600 1.0. This indicates that LAM synthesis from the precursor PIMs only occurs efficiently when the cells are dividing slower, as occurs in our culture conditions as of OD600 1.0. The difference in LAM content may explain the difference in infection efficiencies, as interaction of ManLAM with the macrophage mannose receptor is one of the important entry pathways for the bacterium (58).

### ManLAM biosynthesis mutants do not show differences in cell wall lipid composition

As mentioned earlier, the mycobacterial cell envelope is important for its virulence and consists of a peptidoglycan/arabinogalactan/mycolic acid core, interacting with complex lipids ranging in polarity from apolar triacylglycerols (TAGs) and phthiocerol dimycocerosates (PDIMs) to polar glycolipids and lipo-oligosaccharides (40). We have analyzed apolar and polar lipids from *M*. *bovis* BCG wild type and its ManLAM biosynthesis mutants by twodimensional thin-layer chromatography. The SapM mutant was used as a control to assess any effects which the mere transposon presence might have. The plates were developed in a range of solvent systems, designed to cover the whole range of lipid polarities (data not shown). Mycolic acid methyl esters (MAMEs) were extracted from delipidated cells and also analyzed on TLC (data not shown). We did not observe any difference in lipid composition between wild type M. *bovis* BCG and the mutants, demonstrating that the transposon mutagenesis of ManLAM biosynthesis genes results in selective defects in this molecule and does not fundamentally alter the biosynthesis of other cell wall constituents nor the cell wall lipid composition.

### Discussion

Many pathways are involved in aiding pathogenic mycobacteria to survive successfully within its host, even in the presence of an elaborate immune response (7). An important aspect of this survival strategy is the inhibition by the bacterium of macrophage phagosome maturation. In the context of improved vaccine design for TB, inactivation of the virulence factors involved in e.g. phagosome maturation inhibition, is an attractive strategy. As the only currently licensed vaccine for TB is *M*. *bovis* BCG, we chose to set up a rapid system to obtain mutants in *M*. *bovis* BCG in order to rapidly evaluate the effects of such mutations on the target pathways and on immunogenicity. Furthermore, the deletions which have occurred in deriving the BCG strains from virulent *M*. *bovis* have inactivated one of the pathways involved in the blocking of the phagosome- lysosome fusion (2, 61), making it more likely that mutations in other pathways involved in this process will give a clearer phenotype in this background than in M. *tb* or *M*. *bovis,* as less phenotypic complementation is expected. Moreover, the vaccine strain is very similar, genetically (99.9%) and physiologically, to *M*. *tb* while having the advantage of being non-pathogenic to immunocompetent humans, thus allowing its manipulation under standard bio-safety conditions. This makes a rapidly screenable mutant collection a very useful resource for the wider TB research community. For all these reasons, we created a transposon insertion library of about 100,000 mutants in the *M*. *bovis* BCG background, 96x96 clones of which were manually picked and pooled to form an ordered library. This enabled rapid and efficient PCR screening of the library for mutants in any gene of interest, under condition that the gene is non-essential for *in vitro* growth. We obtained mutants for a majority of the genes that we screened for (15 out of 19). Screens for several more genes are currently ongoing. To validate the approach, we screened for mutants in genes involved in the biosynthetic pathway of ManLAM, a molecule known to contribute to key aspects of the pathogenesis of tuberculosis (6, 63). We also screened for mutants in the secreted acid phosphatase SapM which is also suspected to be important for intra-macrophage survival (64). For the biochemical characterization of ManLAM mutants, the mutant in the PI3P phosphatase SapM served as a control to exclude any effects which the mere presence of the transposon might have on lipoglycan biosynthesis. The genes Mb1661c and Mb2203 are predicted to be α-1,2-mannosyltransferases involved in ManLAM modification (either in the poly-α-1,6-mannosyl backbone or on the terminal cap structures), based on analysis of their orthologues in *M. tuberculosis* and *M. smegmatis* (12, 21). The current understanding is that in slow growing pathogenic Mycobacteria, Mb1661c is largely responsible for adding the first α-1,2-mannose residue in the cap structures, while the Mb2203-encoded enzyme synthesizes the di-mannosyl caps (and may elongate them to trimannosides as well) (24). However, in the study reporting the function of the Mb1661c homologue in *M*. *tb,* it was noted that the levels of ManLAM per cell in the knock-out was about 10-fold lower than in the wild type (11), indicating that this mutation has more profound effects on LAM synthesis than just affecting ManLAM capping. Consistent with this, we find that both Mb2203::T and Mb1661c::T mutants have strongly reduced cell surface abundance of α-1,2-oligomannosides, but this is reflected differently in the direct chemical lipoglycan analysis. Mb2203 inactivation yields a clear defect in synthesis of the di- and tri-mannoside caps, whereas, the Mb1661c inactivation results in a strongly reduced abundance of the caps, while the remaining ones still have intact structures, probably because of partial complementation of the defect by another, as yet to be identified transferase. Clearly, the Mb2203::T mutant is more specifically affected in the α-1,2-oligomannosyl capping of the ManLAM and is therefore more suitable for the analysis of the role of these caps in mycobacterial pathogenesis. Mb2196 has so far only had its orthologue characterized in the fast-growing non-pathogenic species *M*. *smegmatis* where its mutation caused the accumulation of a truncated LM and absence of LAM (23). Our mutant in *M. bovis* BCG, Mb2196::T, displayed entirely wild type behavior with regard to Cyanovirin-N cell wall staining and lipoglycan size analysis. In glycan analysis of acid hydrolysates of the lipoglycan extract, ManLAM cap structures were less abundant than for WT, but not as dramatic as for the Mb1661s mutant. This is partially inconsistent with the reported function of the homologous gene in *M. smegmatis* (one would expect no LAM synthesis which may be the case and accumulation of a truncated LM, which is not the case here). A further difference with the wild type which was found was that ManLAM of Mb2196::T mutant did not stain with Cyanovirin-N under SDS-PAGE conditions. We speculate that this may be because of subtle alterations in the positioning of the α-1,2-oligomannoside caps along the molecule. Cyanovirin-N binds with high avidity to several α-1,2-oligomannoside branches of N-glycans at the same time (60), while binding to single branches is much weaker. It may be that thus, under SDS-PAGE conditions, Mb2196 ManLAM is not properly structured for the high avidity binding. Further analysis is needed, but it is clear that Mb2196 mutation causes a more subtle phenotype in lipoglycan synthesis in *M*. *bovis* BCG than in *M. smegmatis.* It also illustrates that it is best to validate pathways with potential importance in pathogenesis in slowgrowing M. *tb* complex strains, and our easily screenable M. *bovis* BCG mutant library is an efficient tool to that effect. In conclusion, the screenable mutant collection in *M. bovis* BCG allows rapid identification and characterization of a mutant of interest, e.g. the effect of mutations on BCG vaccine efficiency, with the hope of improving the suboptimal protection obtained with the only vaccine presently available to prevent tuberculosis (*M. bovis* BCG).

### Example 3. Protective efficacy of M. bovis BCG wild type versus mutant SapM::T (Mb3338::T), Mb2203::T, Mb1661c::T and Mb2196::T in BALB/c mice

BALB/c mice were vaccinated subcutaneously with 105 CFU of the parental or mutant M. *bovis* BCG strains. Three months post-vaccination, animals were intravenously infected with 5.10⁴ CFU of luminescent M. *tuberculosis* H37Rv. Mice were sacrificed 2, 4 and 8 weeks post-infection and the number of bacteria in spleen and lungs was determined by luminometry. Both parental and three mutant BCG strains offered significant protection against M. *tuberculosis* as indicated by ten- to one-hundred fold reduced numbers of bacteria in spleen and lungs as compared to unvaccinated TB infected mice (the Mb2196 mutant is currently being tested in this setting and is not yet included) (Fig. 4A and Fig. 5). In spleen, there was no difference in efficacy between the parental and mutant strains (Fig. 5), whereas in lungs a significantly lower number of bacteria was found in mice vaccinated with the SapM::T mutant than in mice vaccinated with parental BCG at 8 weeks post-infection (Fig. 4A). It is interesting to note that at 8 weeks post-infection, the protective efficacy of the parental BCG started to decrease, whereas bacterial counts for all three mutants remained stable.

Mice were also followed for 11 months in a long term survival study and monitored weekly for mortality and weight loss. Vaccination with both ManLAM capping mutants (P<0.05) and the SapM::T mutant (P<0.005) resulted in significantly longer survival as compared to nonvaccinated mice, whereas vaccination with the parental BCG did not. Moreover, mice vaccinated with the SapM::T mutant survived significantly longer than mice vaccinated with the parental BCG (Median Survival Time of SapM::T mutant is 32 weeks cfr 26.5 weeks in mice vaccinated with parental BCG P=0.0145) (Fig. 4B-C). Of note, also the Mb2196::T mutant was tested in this setting and significantly longer survival was seen, with survival times between the ManLAM capping mutants and the SapM::T mutant (Fig. 4D). Vaccination with all BCG strains delayed the apparition of cachexia as compared to mice infected with TB alone. Whereas TB infected mice started to show weight loss at about week 15 post-infection, BCG vaccinated animals kept their initial body weight about ten weeks longer. Afterwards, cachexia developed also in BCG vaccinated mice and, as for the survival, mice vaccinated with Mb1661c::T, 2196::T and SapM::T mutant showed the smallest reductions in mean body weight (Fig. 4E).

We subsequently checked if results could be confirmed when the infection occurred intratracheally instead of intravenously 3 months post-vaccination. The number of bacteria in spleen and lungs was determined 4 and 8 weeks post-infection. We could clearly see that both parental and all four mutant BCG strains offered significant protection against M. *tuberculosis* as shown by ten- to one-hundred fold reduced numbers of bacteria in spleen and lungs as compared to unvaccinated TB infected mice. Both in spleens and lungs, a significantly lower number of bacteria was found in mice vaccinated with the SapM::T mutant than in mice vaccinated with parental BCG at 8 weeks post-infection (Fig. 4F-G).

Four weeks after vaccination, we also assessed T-cell immunity by comparing antigenspecific interferon (IFN)-y production by lung cells and splenocytes (Fig. 6). Significant IFNγ production in response to recAg85A (Rv3804c) and the synthetic 20-mer peptide p11 (Ag85A99-118aa, dominant CD4 T cell epitope (Denis et al, 1998)) could be measured in both spleen and lung cell culture supernatants from BALB/c mice vaccinated with *M. bovis* BCG wild type and the SapM locus mutant, demonstrating a clear Th1 response. Lung cells from mice vaccinated with *M. bovis* BCG SapM::T induced lower IFN-γ levels in response to p11 compared to lung cells from mice vaccinated with *M*. *bovis* BCG. No drastic differences could be observed between splenocytes from mice vaccinated with *M. bovis* BCG wild type versus mutants. Unvaccinated mice produced only minimal amounts of IFNγ.

### Effect of the mutations on uptake by macrophages and dendritic cells

Since ManLAM capping and SapM were described to be implicated in uptake of mycobacteria (Briken et al, 2004; Torrelles et al, 2006) and/or blockade of phagosomelysosome fusion (Vergne et al, 2005), we investigated if these pathways were indeed affected by the mutations under study, and could thus contribute to the observed improved protection. To study the effect of disruption of the SapM locus or deficiency in ManLAM capping on the efficiency of binding and phagocytosis of the mycobacteria, M. *bovis* BCG wild type and mutants were FITC labeled and uptake by murine macrophages was analyzed by flow cytometry. The percentage of FITC-positive macrophages is an indication of how many macrophages have bound or taken up FITC-labeled mycobacteria. We could show that wild type M. *bovis* BCG, SapM::T and capping mutants were taken up similarly by murine macrophages (∼60-70% FITC-positive macrophages 6 hours post-infection; Fig. 7A). Confocal and electron microscopy analysis demonstrated that mycobacteria are taken up by the macrophages and not just cell surface attached (data not shown). By calculating mean FITC intensities, we also demonstrate that similar amounts of bacteria are taken up by the macrophages in all conditions tested (Fig. 7A). We conclude that neither disruption of the SapM locus nor ManLAM capping deficiency strongly affect the binding and uptake of the bacterium to macrophages.

Similarly, we analyzed the uptake of the parental M. bovis BCG and the SapM::T mutant by bone-marrow-derived dendritic cells (BM-DCs). We could show that wild type *M*. *bovis* BCG and the SapM::T mutant were taken up similarly by murine DCs (∼25% FITC-positive DC's 24 hours post-infection; Fig. 7B). Similar amounts of bacteria are taken up by the DCs in both conditions tested as shown by comparable mean FITC intensities, demonstrating that neither disruption of the SapM locus nor ManLAM capping deficiency strongly affect the uptake of the bacterium by dendritic cells.

### Effect of the mutations on phagosome-lysosome fusion

Inhibition of phagolysosome fusion in infected macrophages by ManLAM is considered a hallmark of mycobacterial virulence (Fratti et al, 2003; Hmama et al, 2004; Kang et al, 2005; Vergne et al, 2003). *M. tuberculosis* PI3P phosphatase was shown to be required for reduction of PI3P levels of phagosomes and inhibition of phagosome maturation containing live mycobacteria (Vergne et al, 2005). To find out if the *M. bovis* BCG mutants (SapM::T, Mb2203::T and Mb1661c::T) were affected in phagosome maturation in macrophages, we performed colocalization experiments. Therefore, macrophages were infected with FITClabeled *M. bovis* BCG and subsequently labeled with Lysotracker Red. Colocalization of BCG-containing phagosomes and lysosomes was examined by confocal microscopy (Fig. 7C). No significant differences in colocalization of FITC-containing phagosomes with lysosomes could be seen between macrophages infected with the parental strain and mutants. These findings were confirmed by analyzing the survival of the Mb2203::T, Mb1661c::T and SapM::T mutants in macrophages. There was no significant difference in the survival and replication in Mf4/4 macrophages of *M*. *bovis* BCG mutants as compared to the wild type over a time period of 48h post-infection (Fig. 7D). The improved protective efficacy of the mutant BCG strains does not correlate with altered uptake or survival within macrophages *in vitro.*

### Oxidant activity following infection with M. bovis BCG wild type versus mutants

The ability of mycobacteria to survive within the hostile environment of macrophages also depends upon their ability to scavenge reactive oxygen species (ROS). Recently, it was shown that immunogenicity of *M*. *bovis* BCG can be enhanced by reducing the activity and secretion of microbial antioxidants (Sadagopal et al, 2009). Therefore we compared intracellular ROS production following infection of macrophages with M. *bovis* BCG wild type versus mutants, by means of dihydro-dichloro-fluorescein diacetate (CM-H2DCFDA), a cell-permeant indicator for ROS that is nonfluorescent until removal of the acetate groups by intracellular esterases and oxidation occurs within the cell. We document an increase in ROS production over time post-infection (Fig. 8), but no significant differences are seen between M. *bovis* BCG wild type and tested mutants.

### Effect of the mutations on the induction of autophagy in macrophages and BM-DCs following infection with M. bovis BCG

Recently, it was demonstrated that autophagy, a bulk protein and organelle degradation process essential for cell maintenance and viability, could enhance the efficacy of BCG as a vaccine by increasing antigen presentation in mouse dendritic cells (Jagannath et al, 2009). Microtubule-associated protein 1 light chain 3 (LC3), the mammalian homologue of yeast Atg8, is involved in autophagosome formation during autophagy. Tracking the conversion of LC3-I to LC3-II is indicative of autophagic activity. The amount of LC3-II correlates well with the number of autophagosomes. This characteristic conversion of LC3 was used to monitor autophagic activity following infection of macrophages and BM-DCs with M. *bovis* BCG wild type versus mutants. We could not observe any difference in conversion to LC3-II in wild type versus mutant BCG tested (data not shown), demonstrating that the improved vaccine efficacy can not be attributed to a higher degree of autophagy induction in the antigen-presenting cells (APCs) tested.

### Effect on lymph node DC trafficking and activation

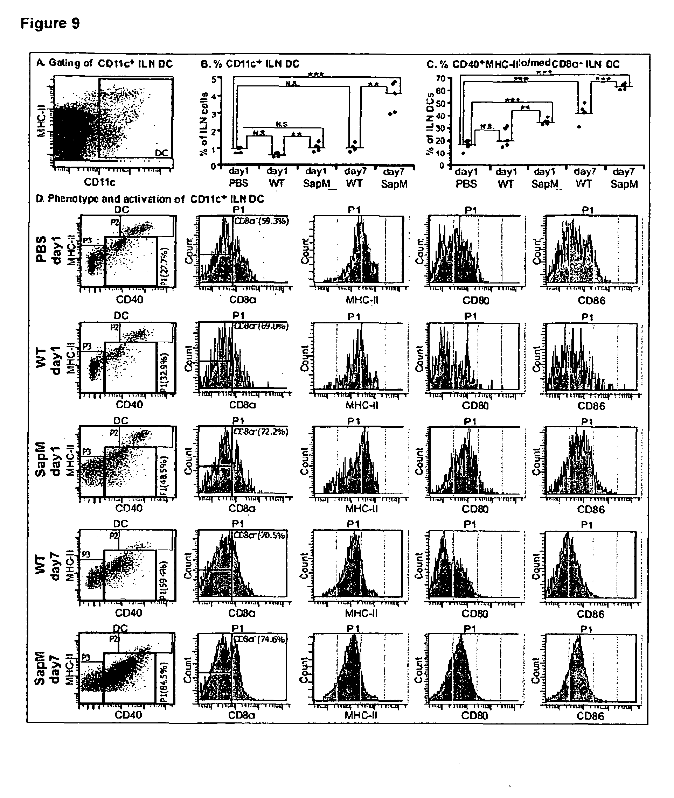

Dendritic cells have the property to migrate from sites of antigen uptake to lymphoid organs, thereby initiating adaptive immune responses. A difference in trafficking to the lymph nodes and/or activation of DCs might have a major impact on the final immune outcome. In this context, we evaluated the effect of vaccination of mice with the parental *M*. *bovis* BCG versus the SapM locus mutant. Therefore, BALB/c mice were infected subcutaneously with 10⁶ CFU of the parental or the *M*. *bovis* BCG SapM mutant strain. Mice were sacrificed at day 1 and day 7 post-infection and the infiltration of DCs in the inguinal and brachial/axillary lymph nodes and their activation status was examined flowcytometrically. At day 7 post-infection, a clear increase in the amount of CD11c+ DCs could be seen in the inguinal (Fig. 9A-B) and brachial/axillary lymph nodes (data not shown) from mice infected with the *M*. *bovis* BCG SapM mutant compared to the control and M. *bovis* BCG WT infected mice. Already at day 1 post-infection, the amount of CD40+ MHC-IIlo/med CD8α- DCs was raised in *M. bovis* BCG SapM-infected mice compared to the M. *bovis* BCG WT-infected mice, a difference which could also be observed 7 days post-infection (Fig 9C). The reduced MHC-II expression refers to the decreasing amount of resident DCs in the draining lymph nodes. Besides the increased recruitment of DCs to the draining lymph nodes upon vaccination with *M. bovis* BCG SapM, the DCs also demonstrate increased expression of CD80 and CD86, demonstrating improved activation (Fig 9D). We can thus conclude that vaccination with the M. *bovis* SapM locus mutant augments recruitment of DCs to the lymph nodes and their activation, which can consequently lead to increased antigen presentation and T cell activation.

### Effect of the mutations on bacterial replication of M. bovis BCG in infected mice

BALB/c mice were infected intravenously with *M. bovis* BCG and *M. bovis* BCG mutants (Mb2203::T, Mb1661c::T and SapM::T). The bacterial load in the lungs and spleen was determined at week 2, 4 and 12 post-infection. Two weeks after infection, slightly higher counts of the parental strain compared to the mutants could be measured in the lungs. Four weeks post-infection, the mutants reached similar numbers of CFU in the lungs and spleen as the wild type *M*. *bovis* BCG cells (Fig. 10A-B). Overall, bacterial numbers in lung and spleen decreased over time, demonstrating clearance.

### Effect of the mutations on cytokine production and granuloma formation in vivo

The uptake of M. *tb* by alveolar macrophages typically results in a local inflammatory response (van Crevel et al, 2002), mediated by the production of IL-12, IFNγ and TNF. This response finally leads to granuloma formation (Cooper et al, 1993; Dalton et al, 1993; Kamijo et al, 1993; Kindler et al, 1989). Progressive granuloma formation is a hallmark of chronic mycobacterial infection, which is associated by a partial shift to Th2-type immunity (Harris et al, 2008; Jiao et al, 2003; Orme et al, 1993; Rhodes et al, 2000). Therefore we investigated the influence of an altered (Man)LAM structure or inactivation of the PI3P phosphatase SapM on the local inflammatory response in the lungs. C57BL/6 mice were intratracheally instilled with *M*. *bovis* BCG wild type or mutants and IFNγ, TNF, IL-2, IL-4, IL-5, IL-6, IL-10 or ILS2 12p70 levels were quantified in the bronchioalveolar lavage (BAL) four weeks post-infection. We could detect the production of IFN-γ and TNF, but, no or very low amounts of IL-2, IL-4, IL-5, IL-6, IL-10 or IL-12p70 could be measured. No significant differences could be observed in the elicited cytokine response in the lungs following infection with wild type versus mutant BCG (Fig. 10C, Table 2).

**Table 2: Cytokine measurements in BALF following infection of mice with M. bovis BCG wild type versus mutants**

| Average | | | | | |
|---|---|---|---|---|---|
| Cytokine | PBS | wild type | 1661c | 2203 | SapM |
| IFNγ | 0,06 | 96,45 | 84,86 | 146,86 | 116,80 |
| TNF | 0,00 | 102,53 | 123,73 | 145,83 | 106,53 |
| IL-2 | 0,10 | 0,86 | 1,34 | 0,89 | 0,79 |
| IL-4 | 0,18 | 0,83 | 0,67 | 0,17 | 0,20 |
| IL-5 | 0,09 | 1,03 | 1,11 | 0,29 | 0,43 |
| IL-6 | 0,28 | 11,50 | 5,43 | 7,09 | 4,18 |
| IL-10 | 13,29 | 4,65 | 3,71 | 5,78 | 4,07 |
| IL-12p70 | 0,48 | 0,65 | 1,15 | 0,77 | 0,17 |
| | | | | | |

| SEM | | | | | |
|---|---|---|---|---|---|
| Cytokine | PBS | wildtype | 1661c | 2203 | SapM |
| IFNγ | 0,03 | 22,10 | 21,00 | 47,34 | 28,17 |
| TNF | 0,00 | 33,43 | 32,97 | 46,01 | 26,34 |
| IL-2 | 0,05 | 0,38 | 0,45 | 0,30 | 0,29 |
| IL-4 | 0,13 | 0,48 | 0,37 | 0,08 | 0,14 |
| IL-5 | 0,09 | 0,55 | 0,43 | 0,15 | 0,31 |
| IL-6 | 0,19 | 2,85 | 1,78 | 2,35 | 0,83 |
| IL-10 | 2,34 | 2,18 | 1,21 | 1,33 | 1,26 |
| IL-12p70 | 0,25 | 0,28 | 0,63 | 0,35 | 0,17 |
| | | | | | |

| Range | | | | | |
|---|---|---|---|---|---|
| Cytokine | PBS | wildtype | 1661c | 2203 | SapM |
| IFNγ | 0-0,29 | 32,35-180,58 | 8,79-194,68 | 2,44-454,73 | 58,40-291,09 |
| TNF | 0,00 | 0-270,97 | 31,33-306,61 | 11,04-434,85 | 33,59-246,74 |
| IL-2 | 0-0,38 | 0-2,93 | 0-4,01 | 0-2,51 | 0-2,38 |
| IL-4 | 0-1,08 | 0-3,76 | 0-3,12 | 0-0,66 | 0-1,16 |
| IL-5 | 0-0,73 | 0-4,17 | 0-3,49 | 0-1,34 | 0-2,5 |
| IL-6 | 0-1,51 | 0-21,98 | 1,62-17,27 | 0-21,26 | 1,12-7,49 |
| IL-10 | 5,76-27,03 | 0-19,32 | 0-8,72 | 0-13,43 | 0-8,84 |
| IL-12p70 | 0-1,69 | 0-1,72 | 0-5,03 | 0-2,79 | 0-1,36 |

We also examined the effect of the mutations on *M*. *bovis* BCG-induced protective granuloma formation. C57BL/6 mice were infected intravenously or were intratracheally instilled with *M. bovis* BCG wild type versus mutants. Livers and lungs were removed 3 weeks and 4 weeks post-infection, respectively. Haematoxylin-Eosin stainings on paraffin sections of these organs allowed visualization and counting granulomas. Livers of mice that were infected with Mb1661c::T and Mb2203::T showed slightly lower numbers of granulomas compared to mice that were infected with *M. bovis* BCG wild type and SapM::T (Fig. 10D). However, upon evaluation of the size of the granulomas, we observed that infection with these mutants appeared to induce the formation of slightly bigger granulomas compared to what happens in the livers of SapM::T or wild type infected mice (data not shown).

### Cytokine production following infection of BM-DCs with M. bovis BCG wild type versus SapM::T

In view of the increased recruitment to and activation of DCs in the draining lymph nodes upon infection of mice with the SapM::T mutant, we analyzed cytokine production upon infection of BM-DCs *in vitro.* BM-DCs were infected with M. *bovis* BCG wild type versus SapM::T for the indicated time points at which supernatant was taken to determine the levels of IL-1α, IL-1β, IL-2, IL-4, IL-6, IL-10, IL-12p40, IFNγ, TNFα. We could not detect significant levels of IL-2, IL-4, IL-10 nor IFNγ, however did see an increased production of IL-1β, IL-6, IL-12p40 and TNFα upon infection with *M. bovis* BCG SapM::T compared to wild type (Fig. 11), demonstrating an augmented pro-inflammatory response.

### Effect of the SapM::T mutation on the activation of iNKT cells

The role of non-MHC-restricted T cells in TB is still not clearly delineated. γδ T cells are frequently activated by a variety of pathogens including *M*. *tb.* Mice lacking γδ T cells succumb more rapidly than control mice following intravenous challenge with virulent M. *tb,* whereas this difference has not been observed when infection occurs via the aerosol route (D'Souza et al, 1997; Ladel et al, 1995). Although γδ T cells may not be required for optimum control of bacterial replication following pulmonary infection, γδ T cell deficient mice form disorganized granulomas dominated by foamy macrophages and granulocytes instead of lymphocytes (D'Souza et al, 1997). Invariant (i) NKT cells recognize lipids presented by the antigen-presenting molecule CD1d. It has been shown that granuloma formation upon subcutaneous injection of deproteinized cell walls of M. *tb* in mice depends on iNKT cells (Apostolou et al, 1999). Specific activation of NKT cells by the CD1d ligand α-galactosylceramide (αGalCer) protects susceptible mice from tuberculosis. While it had been previously shown that CD1d-/- mice are not more susceptible to tuberculosis (Chackerian et al, 2002), Sada-Ovalle and colleagues demonstrated that CD1d-restricted iNKT cells play a physiological role in mediating protection against aerosol M. *tb* infection *in vivo* (Sada-Ovalle et al, 2008). The vast majority of diverse CD1d-restricted T cells do not recognize αGalCer, while, in contrast, both human and mouse iNKT cells recognize αGalCer presented by CD1d (Behar & Porcelli, 2007). Activation of NKT cells occurs before that of MHC-restricted T cells during infection with *M. bovis* BCG in mice (*10*)*.* We have investigated the role of iNKT cells upon infection with *M. bovis* BCG WT versus mutants by analyzing cytokine responses and cell surface marker expression following cocultures of *M*. *bovis* BCG infected BM derived DC's with iNKT hybridomas. Coculture with *M*. *bovis* BCG SapM::T infected DC's did induce similar expression levels of IL-2 and IL-12p40 compared to coculture with *M. bovis* BCG WT. We could also not observe clear differences in cell surface marker expression (data not shown). This result is also in accordance with the findings that lipid and mycolic acid composition is similar for the parental *M. bovis* BCG and mutant strains (see Example 2) and that CD1d expression following infection of BM-DCs with the different strains is also comparable (data not shown).

### Discussion

Proof is presented herein of the important concept that a single null mutation in *M. bovis* BCG can result in a vaccine with improved long-term survival during pulmonary tuberculosis. The use of such a mutant BCG strain is easy to implement and will face less safety and regulatory issues than either transgenic BCG or attenuated *M. tuberculosis,* for which similar, (but not better), improvement in long-term survival of experimental animals has been reported and which are now in the first stages of clinical development (STOP-TB-Partnership, 2009). We hypothesized that mutation of mycobacterial components reportedly involved in phagosome maturation inhibition could yield better vaccines, since such mutations should result in better vaccine antigen processing and display. We therefore generated an ordered *M. bovis* BCG transposon insertion mutant library in which we identified and biochemically characterized mutants in two genes necessary for the alpha-1,2-oligomannosyl capping of cell wall ManLAM (Fratti et al, 2003; Hmama et al, 2004; Kang et al, 2005), a gene required for the addition of a linear α-1,6-mannose polymer of 21-34 residues to the PIM4 precursor in LM synthesis (ref. 23) and the PI3P phosphatase SapM gene locus (Vergne et al, 2005), amongst others (see Example 2). ManLAM and SapM were both previously reported to have the potential ofinterfering with macrophage phagosome-lysosome fusion, a major mycobacterium clearance mechanism (Fratti et al, 2003; Hmama et al, 2004; Kang et al, 2005; Vergne et al, 2003; Vergne et al, 2005). We show that vaccination with both ManLAM capping mutants, the LM synthesis mutant and the SapM::T mutant resulted in significantly longer survival following *M*. *tb* infection as compared to non-vaccinated mice. In addition, the SapM::T mutant vaccinees survived significantly longer as compared to parental BCG vaccinees, comparable to other engineered live vaccines currently in clinical trials.

*In vitro* studies showed that the mutant BCG strains survived and replicated equally well as parental BCG in macrophages. *In vivo* bacterial replication analysis in infected immunocompetent mice did not show any difference between wild type and mutants (Fig.10A-B). Similarly, no significant differences could be observed in the elicited cytokine response in the lungs following infection with wild type versus mutant BCG (Fig.10C). (Note that although the results with the Mb2196::T mutant have not been duplicated yet, initial results are similar to those obtained with the other BCG mutants.)

However, there was a great variability in the levels of some cytokines, a problem which has been reported before (Appelmelk et al, 2008). We could also not detect huge differences in the amount of granulomas formed in livers and lungs post-infection. Livers of mice infected with Mb1661c::T and Mb2203::T showed slightly lower numbers of granulomas (Fig. 10D). However, upon evaluation of the size of the granulomas, we observed that infection with these mutants appeared to induce the formation of slightly bigger granulomas compared to what happens in the livers of SapM::T or wild type infected mice (data not shown). Importantly, all of these data show that these mutated BCG strains behave indistinguishably from parental wild type BCG in immunocompetent mice, which confirms their expected safety. Moreover, these results demonstrate that neither SapM nor ManLAM capping play major roles in phagosome maturation arrest, in contrast to accepted dogmas, and probably do not explain the improved vaccine efficiency of the mutants. The reason for this discrepancy on the role of ManLAM and SapM in phagosome maturation is not clear. However, a serious problem with most of the studies previously described is that only purified ManLAM or ManLAM-coated beads were used to proof their point. The same is true for SapM, where its role in phagosome maturation was demonstrated by using purified SapM in a phagosome-late endosome *in vitro* fusion reaction (Vergne et al, 2005). By omitting the use of intact bacteria selectively deficient in ManLAM or SapM, it is by no means clear whether the potential activities of ManLAM and SapM identified in these studies have any relevance for the course of a mycobacterial infection. Appelmelk and colleagues begun to address this important issue by studying the Rv1635c capping mutant of *M*. *bovis* BCG and *M. marinum* (Appelmelk et al, 2008). Indeed, they could show that these mutants were not affected in survival in phagocytes (Appelmelk et al, 2008), suggesting that ManLAM does not dominate the interaction between mycobacteria and the host, in contrast to what was long assumed. However, this mutation also induces a ∼10-fold lower LAM abundance in the cell wall (Dinadayala et al, 2006), which could lead to compensating alterations in the cell wall that mask the phenotype of LAM capping. To resolve this issue, we also made use of the Mb2203 mutant (or Rv2181 in *M*. *tb*), which is actually more selectively affecting the α-1,2-oligomannosyl cap structure synthesis than mutation of Rv1635c, and leaves LAM levels intact (Kaur et al, 2006; Kaur et al, 2008).Therefore, the Mb2203 mutant is more suitable to elucidate the role of α-1,2-oligomannosyl capping in mycobacterial uptake by macrophages and mycobacterial persistence following uptake.

We cannot fail to mention that the ability of *M*. *tb* to survive within the hostile environment of macrophages also depends on its property to scavenge reactive oxygen species. Oxidants have important signaling roles in the maturation of dendritic cells (Kantengwa et al, 2003), proliferation of T cells (van der Veen et al, 2004) and the activation and apoptosis of macrophages (Forman & Torres, 2001). The inactivation of host-generated oxidants by mycobacterial antioxidants may disrupt redox signaling during early infection and promote TB pathogenesis by weakening host immune responses and promoting tissue-damaging immunopathology. We analyzed if the improved protection against *M*. *tb* infection by the *M. bovis* SapM::T mutant could be due to a decreased production of oxidants. However, in all mutants tested, we could not detect any change in oxidant activity following infection of macrophages, showing that also this feature does not contribute to the higher efficacy of the M. *bovis* SapM::T vaccine. We can also exclude that the latter is due to an increased induction of autophagy upon infection of macrophages, since we did not observe differences in the conversion from LC3-I to LC3-II, indicative of autophagic activity, following infection with *M. bovis* BCG wild type versus mutants. However, we do clearly show that subcutaneous vaccination with M. *bovis* BCG SapM::T induces an increased recruitment of CD11c+ DCs to the draining lymph nodes compared to vaccination with the parental vaccine strain. Additionally, these DCs are also in an increased activation status, which can contribute to increased antigen presentation and immune responses, which seem to be pro-inflammatory (Fig. 11). A possible contribution of iNKT cells has been excluded *in vitro* (data not shown).Our observations are also of extreme importance in view of the recent model describing that the decrease in the effectiveness of the BCG vaccine against pulmonary tuberculosis is not a consequence of overattenuation but could be because of increased immune suppression (Kernodle, 2010). If we look to the genome of the Tokyo 172 and Danish 1331 strain, we see that they contain the DU2 duplication unit, enclosing antioxidant genes and Mb3338 (SapM) (Brosch et al, 2007). This DU2 duplication does however not occur in the BCG Pasteur strain. The fact that the Tokyo 172 and Danish 1331 strain are less effective against pulmonary tuberculosis can thus be explained by the presence of multiple copies of antioxidant genes (Kernodle, 2010; Sadagopal et al, 2009) and SapM, which all seem to be immune evasion genes in BCG. It would thus be valuable to combine knockouts of antioxidant genes as described in Sadagopal et al (Sadagopal et al, 2009) with a SapM knockout, as this is expected to further improve the vaccine potency. The implementation of these mutations through standard 'clean, resistancemarker free' gene disruption for clinical trials is important and ongoing.

### References

### Numeric references

1. Bardarov, S., J. Kriakov, C. Carriere, S. Yu, C. Vaamonde, R. A. McAdam, B. R. Bloom, G. F. Hatfull, and W. R. Jacobs, Jr. 1997. Conditionally replicating mycobacteriophages: a system for transposon delivery to Mycobacterium tuberculosis. Proceedings of the National Academy of Sciences of the United States of America 94:10961-6.
2. Behr, M. A., M. A. Wilson, W. P. Gill, H. Salamon, G. K. Schoolnik, S. Rane, and P. M. Small. 1999. Comparative genomics of BCG vaccines by whole-genome DNA microarray. Science (New York, N.Y 284:1520-3**.**
3. Beresford, N. J., D. Mulhearn, B. Szczepankiewicz, G. Liu, M. E. Johnson, A. Fordham-Skelton, C. Abad-Zapatero, J. S. Cavet, and L. Tabernero. 2009. Inhibition of MptpB phosphatase from Mycobacterium tuberculosis impairs mycobacterial survival in macrophages. J Antimicrob Chemother 63:928-36.
4. Berg, S., J. Starbuck, J. B. Torrelles, V. D. Vissa, D. C. Crick, D. Chatterjee, and P. J. Brennan. 2005. Roles of conserved proline and glycosyltransferase motifs of EmbC in biosynthesis of lipoarabinomannan. The Journal of biological chemistry 280:5651-63.
5. Brennan, P. J. 2003. Structure, function, and biogenesis of the cell wall of Mycobacterium tuberculosis. Tuberculosis (Edinburgh, Scotland) 83:91-7.
6. Briken, V., S. A. Porcelli, G. S. Besra, and L. Kremer. 2004. Mycobacterial lipoarabinomannan and related lipoglycans: from biogenesis to modulation of the immune response. Mol Microbiol 53:391-403.
7. Briken, V., S. A. Porcelli, G. S. Besra, and L. Kremer. 2004. Mycobacterial lipoarabinomannan and related lipoglycans: from biogenesis to modulation of the immune response. Molecular microbiology 53:391-403.
8. Brosch, R., S. V. Gordon, T. Garnier, K. Eiglmeier, W. Frigui, P. Valenti, S. Dos Santos, S. Duthoy, C. Lacroix, C. Garcia-Pelayo, J. K. Inwald, P. Golby, J. N. Garcia, R. G. Hewinson, M. A. Behr, M. A. Quail, C. Churcher, B. G. Barrell, J. Parkhill, and S. T. Cole. 2007. Genome plasticity of BCG and impact on vaccine efficacy. Proc Natl Acad Sci U S A 104:5596-601.
9. Castandet, J., J. F. Prost, P. Peyron, C. Astarie-Dequeker, E. Anes, A. J. Cozzone, G. Griffiths, and I. Maridonneau-Parini. 2005. Tyrosine phosphatase MptpA of Mycobacterium tuberculosis inhibits phagocytosis and increases actin polymerization in macrophages. Res Microbiol 156:1005-13.
10. Cavaignac, S. M., S. J. White, G. W. de Lisle, and D. M. Collins. 2000. Construction and screening of Mycobacterium paratuberculosis insertional mutant libraries. Archives of microbiology 173:229-31.
11. Dinadayala, P., D. Kaur, S. Berg, A. G. Amin, V. D. Vissa, D. Chatterjee, P. J. Brennan, and D. C. Crick. 2006. Genetic basis for the synthesis of the immunomodulatory mannose caps of lipoarabinomannan in Mycobacterium tuberculosis. The Journal of biological chemistry 281:20027-35.
12. Dinadayala, P., D. Kaur, S. Berg, A. G. Amin, V. D. Vissa, D. Chatterjee, P. J. Brennan, and D. C. Crick. 2006. Genetic basis for the synthesis of the immunomodulatory mannose caps of lipoarabinomannan in Mycobacterium tuberculosis. J Biol Chem 281:20027-35.
13. Ehrt, S., and D. Schnappinger. 2009. Mycobacterial survival strategies in the phagosome: defence against host stresses. Cell Microbiol 11:1170-8.
14. Flannagan, R. S., G. Cosio, and S. Grinstein. 2009. Antimicrobial mechanisms of phagocytes and bacterial evasion strategies. Nat Rev Microbiol 7:355-66.
15. Fratti, R. A., J. Chua, I. Vergne, and V. Deretic. 2003. Mycobacterium tuberculosis glycosylated phosphatidylinositol causes phagosome maturation arrest. Proceedings of the National Academy of Sciences of the United States of America 100:5437-42.
16. Garnier, T., K. Eiglmeier, J. C. Camus, N. Medina, H. Mansoor, M. Pryor, S. Duthoy, S. Grondin, C. Lacroix, C. Monsempe, S. Simon, B. Harris, R. Atkin, J. Doggett, R. Mayes, L. Keating, P. R. Wheeler, J. Parkhill, B. G. Barrell, S. T. Cole, S. V. Gordon, and R. G. Hewinson. 2003. The complete genome sequence of Mycobacterium bovis. Proceedings of the National Academy of Sciences of the United States of America 100:7877-82.
17. Gordon, S. V., D. Bottai, R. Simeone, T. P. Stinear, and R. Brosch. 2009. Pathogenicity in the tubercle bacillus: molecular and evolutionary determinants. Bioessays 31:378-88.
18. Guenin-Mace, L., R. Simeone, and C. Demangel. 2009. Lipids of pathogenic Mycobacteria: contributions to virulence and host immune suppression. Transboundary and emerging diseases 56:255-68.
19. Gurcha, S. S., A. R. Baulard, L. Kremer, C. Locht, D. B. Moody, W. Muhlecker, C. E. Costello, D. C. Crick, P. J. Brennan, and G. S. Besra. 2002. Ppm1, a novel polyprenol monophosphomannose synthase from Mycobacterium tuberculosis. The Biochemical journal 365:441-50.
20. Kaur, D., S. Berg, P. Dinadayala, B. Gicquel, D. Chatterjee, M. R. McNeil, V. D. Vissa, D. C. Crick, M. Jackson, and P. J. Brennan. 2006. Biosynthesis of mycobacterial lipoarabinomannan: role of a branching mannosyltransferase. Proceedings of the National Academy of Sciences of the United States of America 103:13664-9.
21. Kaur, D., S. Berg, P. Dinadayala, B. Gicquel, D. Chatterjee, M. R. McNeil, V. D. Vissa, D. C. Crick, M. Jackson, and P. J. Brennan. 2006. Biosynthesis of mycobacterial lipoarabinomannan: role of a branching mannosyltransferase. Proc Natl Acad Sci U S A 103:13664-9.
22. Kaur, D., T. L. Lowary, V. D. Vissa, D. C. Crick, and P. J. Brennan. 2002. Characterization of the epitope of anti-lipoarabinomannan antibodies as the terminal hexaarabinofuranosyl motif of mycobacterial arabinans. Microbiology (Reading, England) 148:3049-57.
23. Kaur, D., M. R. McNeil, K. H. Khoo, D. Chatterjee, D. C. Crick, M. Jackson, and P. J. Brennan. 2007. New insights into the biosynthesis of mycobacterial lipomannan arising from deletion of a conserved gene. The Journal of biological chemistry 282:27133-40.
24. Kaur, D., A. Obregon-Henao, H. Pham, D. Chatterjee, P. J. Brennan, and M. Jackson. 2008. Lipoarabinomannan of Mycobacterium: mannose capping by a multifunctional terminal mannosyltransferase. Proceedings of the National Academy of Sciences of the United States of America 105:17973-7.
25. Kordulakova, J., M. Gilleron, K. Mikusova, G. Puzo, P. J. Brennan, B. Gicquel, and M. Jackson. 2002. Definition of the first mannosylation step in phosphatidylinositol mannoside synthesis. PimA is essential for growth of mycobacteria. The Journal of biological chemistry 277:31335-44.
26. Kremer, L., S. S. Gurcha, P. Bifani, P. G. Hitchen, A. Baulard, H. R. Morris, A. Dell, P. J. Brennan, and G. S. Besra. 2002. Characterization of a putative alphamannosyltransferase involved in phosphatidylinositol trimannoside biosynthesis in Mycobacterium tuberculosis. The Biochemical journal 363:437-47.
27. Lamichhane, G., M. Zignol, N. J. Blades, D. E. Geiman, A. Dougherty, J. Grosset, K. W. Broman, and W. R. Bishai. 2003. A postgenomic method for predicting essential genes at subsaturation levels of mutagenesis: application to Mycobacterium tuberculosis. Proceedings of the National Academy of Sciences of the United States of America 100:7213-8.
28. Lampe, D. J., M. E. Churchill, and H. M. Robertson. 1996. A purified mariner transposase is sufficient to mediate transposition in vitro. The EMBO journal 15:5470-9.
29. Lane, J. M., and E. J. Rubin. 2006. Scaling down: a PCR-based method to efficiently screen for desired knockouts in a high density Mycobacterium tuberculosis picked mutant library. Tuberculosis (Edinburgh, Scotland) 86:310-3.
30. Laroy, W., R. Contreras, and N. Callewaert. 2006. Glycome mapping on DNA sequencing equipment. Nat Protoc 1:397-405.
31. Laroy, W., R. Contreras, and N. Callewaert. 2006. Glycome mapping on DNA sequencing equipment. Nature protocols 1:397-405.
32. Machowski, E. E., R. A. McAdam, K. M. Derbyshire, and V. Mizrahi. 2000. Construction and application of mycobacterial reporter transposons. Gene 253:67-75.
33. Mahairas, G. G., P. J. Sabo, M. J. Hickey, D. C. Singh, and C. K. Stover. 1996. Molecular analysis of genetic differences between Mycobacterium bovis BCG and virulent M. bovis. Journal of bacteriology 178:1274-82.
34. Maras, M., N. Callewaert, K. Piens, M. Claeyssens, W. Martinet, S. Dewaele, H. Contreras, I. Dewerte, M. Penttila, and R. Contreras. 2000. Molecular cloning and enzymatic characterization of a Trichoderma reesei 1,2-alpha-D-mannosidase. Journal of biotechnology 77:255-63.
35. Master, S. S., S. K. Rampini, A. S. Davis, C. Keller, S. Ehlers, B. Springer, G. S. Timmins, P. Sander, and V. Deretic. 2008. Mycobacterium tuberculosis prevents inflammasome activation. Cell Host Microbe 3:224-32.
36. Master, S. S., S. K. Rampini, A. S. Davis, C. Keller, S. Ehlers, B. Springer, G. S. Timmins, P. Sander, and V. Deretic. 2008. Mycobacterium tuberculosis prevents inflammasome activation. Cell host & microbe 3:224-32.
37. McAdam, R. A., S. Quan, D. A. Smith, S. Bardarov, J. C. Betts, F. C. Cook, E. U. Hooker, A. P. Lewis, P. Woollard, M. J. Everett, P. T. Lukey, G. J. Bancroft, W. R. Jacobs Jr, Jr., and K. Duncan. 2002. Characterization of a Mycobacterium tuberculosis H37Rv transposon library reveals insertions in 351 ORFs and mutants with altered virulence. Microbiology (Reading, England) 148:2975-86.
38. McCarthy, T. R., J. B. Torrelles, A. S. MacFarlane, M. Katawczik, B. Kutzbach, L. E. Desjardin, S. Clegg, J. B. Goldberg, and L. S. Schlesinger. 2005. Overexpression of Mycobacterium tuberculosis manB, a phosphomannomutase that increases phosphatidylinositol mannoside biosynthesis in Mycobacterium smegmatis and mycobacterial association with human macrophages. Molecular microbiology 58:774-90.
39. McFadden, J. 1996. Recombination in mycobacteria. Molecular microbiology 21:205-11.
40. Minnikin, D. E., L. Kremer, L. G. Dover, and G. S. Besra. 2002. The methylbranched fortifications of Mycobacterium tuberculosis. Chem Biol 9:545-53.
41. Monsarrat, B., T. Brando, P. Condouret, J. Nigou, and G. Puzo. 1999. Characterization of mannooligosaccharide caps in mycobacterial lipoarabinomannan by capillary electrophoresis/electrospray mass spectrometry. Glycobiology 9:335-42.
42. Mueller, P., and J. Pieters. 2006. Modulation of macrophage antimicrobial mechanisms by pathogenic mycobacteria. Immunobiology 211:549-56.
43. Murry, J. P., C. M. Sassetti, J. M. Lane, Z. Xie, and E. J. Rubin. 2008. Transposon site hybridization in Mycobacterium tuberculosis. Methods in molecular biology (Clifton, N.J 416:45-59.
44. Ortalo-Magne, A., M. A. Dupont, A. Lemassu, A. B. Andersen, P. Gounon, and M. Daffe. 1995. Molecular composition of the outermost capsular material of the tubercle bacillus. Microbiology (Reading, England) 141 (Pt 7):1609-20**.**
45. Owens, R. M., F. F. Hsu, B. C. VanderVen, G. E. Purdy, E. Hesteande, P. Giannakas, J. C. Sacchettini, J. D. McKinney, P. J. Hill, J. T. Belisle, B. A. Butcher, K. Pethe, and D. G. Russell. 2006. M. tuberculosis Rv2252 encodes a diacylglycerol kinase involved in the biosynthesis of phosphatidylinositol mannosides (PIMs). Molecular microbiology 60:1152-63.
46. Packer, N. H., M. A. Lawson, D. R. Jardine, and J. W. Redmond. 1998. A general approach to desalting oligosaccharides released from glycoproteins. Glycoconjugate journal 15:737-47.
47. Parish, T., and N. G. Stoker. 1999. Mycobacteria: bugs and bugbears (two steps forward and one step back). Molecular biotechnology 13:191-200.
48. Prinzis, S., D. Chatterjee, and P. J. Brennan. 1993. Structure and antigenicity of lipoarabinomannan from Mycobacterium bovis BCG. Journal of general microbiology 139:2649-58.
49. Rubin, E. J., B. J. Akerley, V. N. Novik, D. J. Lampe, R. N. Husson, and J. J. Mekalanos. 1999. In vivo transposition of mariner-based elements in enteric bacteria and mycobacteria. Proceedings of the National Academy of Sciences of the United States of America 96:1645-50.
50. Russell, D. G. 2001. Mycobacterium tuberculosis: here today, and here tomorrow. Nature reviews 2:569-77.
51. Rybniker, J., M. Wolke, C. Haefs, and G. Plum. 2003. Transposition of Tn5367 in Mycobacterium marinum, using a conditionally recombinant mycobacteriophage. Journal of bacteriology 185:1745-8.
52. Saleh, M. T., and J. T. Belisle. 2000. Secretion of an acid phosphatase (SapM) by Mycobacterium tuberculosis that is similar to eukaryotic acid phosphatases. J Bacteriol 182:6850-3.
53. Sander, P., M. Rezwan, B. Walker, S. K. Rampini, R. M. Kroppenstedt, S. Ehlers, C. Keller, J. R. Keeble, M. Hagemeier, M. J. Colston, B. Springer, and E. C. Bottger. 2004. Lipoprotein processing is required for virulence of Mycobacterium tuberculosis. Molecular microbiology 52:1543-52.
54. Sassetti, C. M., D. H. Boyd, and E. J. Rubin. 2001. Comprehensive identification of conditionally essential genes in mycobacteria. Proceedings of the National Academy of Sciences of the United States of America 98:12712-7.
55. Sassetti, C. M., D. H. Boyd, and E. J. Rubin. 2003. Genes required for mycobacterial growth defined by high density mutagenesis. Mol Microbiol 48:77-84.
56. Sassetti, C. M., D. H. Boyd, and E. J. Rubin. 2003. Genes required for mycobacterial growth defined by high density mutagenesis. Molecular microbiology 48:77-84.
57. Schaeffer, M. L., K. H. Khoo, G. S. Besra, D. Chatterjee, P. J. Brennan, J. T. Belisle, and J. M. Inamine. 1999. The pimB gene of Mycobacterium tuberculosis encodes a mannosyltransferase involved in lipoarabinomannan biosynthesis. The Journal of biological chemistry 274:31625-31.
58. Schlesinger, L. S., T. M. Kaufman, S. Iyer, S. R. Hull, and L. K. Marchiando. 1996. Differences in mannose receptor-mediated uptake of lipoarabinomannan from virulent and attenuated strains of Mycobacterium tuberculosis by human macrophages. Journal of immunology (Baltimore, Md. 157:4568-75.
59. Sharma, K., M. Gupta, M. Pathak, N. Gupta, A. Koul, S. Sarangi, R. Baweja, and Y. Singh. 2006. Transcriptional control of the mycobacterial embCAB operon by PknH through a regulatory protein, EmbR, in vivo. Journal of bacteriology 188:2936-44.
60. Shenoy, S. R., L. G. Barrientos, D. M. Ratner, B. R. O'Keefe, P. H. Seeberger, A. M. Gronenborn, and M. R. Boyd. 2002. Multisite and multivalent binding between cyanovirin-N and branched oligomannosides: calorimetric and NMR characterization. Chemistry & biology 9:1109-18.
61. Tan, T., W. L. Lee, D. C. Alexander, S. Grinstein, and J. Liu. 2006. The ESAT-6/CFP-10 secretion system of Mycobacterium marinum modulates phagosome maturation. Cellular microbiology 8:1417-29.
62. Venisse, A., J. M. Berjeaud, P. Chaurand, M. Gilleron, and G. Puzo. 1993. Structural features of lipoarabinomannan from Mycobacterium bovis BCG. Determination of molecular mass by laser desorption mass spectrometry. The Journal of biological chemistry 268:12401-11.
63. Vergne, I., J. Chua, and V. Deretic. 2003. Tuberculosis toxin blocking phagosome maturation inhibits a novel Ca2+/calmodulin-PI3KhVPS34 cascade. J Exp Med 198:653-9.
64. Vergne, I., J. Chua, H. H. Lee, M. Lucas, J. Belisle, and V. Deretic. 2005. Mechanism of phagolysosome biogenesis block by viable Mycobacterium tuberculosis. Proc Natl Acad Sci U S A 102:4033-8.
65. Zhang, N., J. B. Torrelles, M. R. McNeil, V. E. Escuyer, K. H. Khoo, P. J. Brennan, and D. Chatterjee. 2003. The Emb proteins of mycobacteria direct arabinosylation of lipoarabinomannan and arabinogalactan via an N-terminal recognition region and a C-terminal synthetic region. Molecular microbiology 50:69-76.

### Alphabetic references

Abramoff MD, Magelhaes PJ, Ram SJ (2004) Image processing with ImageJ. Biophotonics International 11(7): 36-42
Apostolou I, Takahama Y, Belmant C, Kawano T, Huerre M, Marchal G, Cui J, Taniguchi M, Nakauchi H, Fournie JJ, Kourilsky P, Gachelin G (1999) Murine natural killer T(NKT) cells [correction of natural killer cells] contribute to the granulomatous reaction caused by mycobacterial cell walls. Proc Natl Acad Sci U S A 96(9): 5141-5146
Appelmelk BJ, den Dunnen J, Driessen NN, Ummels R, Pak M, Nigou J, Larrouy-Maumus G, Gurcha SS, Movahedzadeh F, Geurtsen J, Brown EJ, Eysink Smeets MM, Besra GS, Willemsen PT, Lowary TL, van Kooyk Y, Maaskant JJ, Stoker NG, van der Ley P, Puzo G, Vandenbroucke-Grauls CM, Wieland CW, van der Poll T, Geijtenbeek TB, van der Sar AM, Bitter W (2008) The mannose cap of mycobacterial lipoarabinomannan does not dominate the Mycobacterium-host interaction. Cell Microbiol 10(4): 930-944
Barker LF, Brennan MJ, Rosenstein PK, Sadoff JC (2009) Tuberculosis vaccine research: the impact of immunology. Curr Opin Immunol 21(3): 331-338
Bastos RG, Borsuk S, Seixas FK, Dellagostin OA. (2009) Recombinant Mycobacterium bovis BCG. Vaccine 27(47): 6495-503.
Behar SM, Porcelli SA (2007) CD1-restricted T cells in host defense to infectious diseases. Curr Top Microbiol Immunol 314: 215-250
Bolte S, Cordelières FP (2006) A guided tour into subcellular colocalization analysis in light microscopy. J Microsc 224(3): 213-232
Briken V, Porcelli SA, Besra GS, Kremer L (2004) Mycobacterial lipoarabinomannan and related lipoglycans: from biogenesis to modulation of the immune response. Mol Microbiol 53(2): 391-403
Brosch R, Gordon SV, Gamier T, Eiglmeier K, Frigui W, Valenti P, Dos Santos S, Duthoy S, Lacroix C, Garcia-Pelayo C, Inwald JK, Golby P, Garcia JN, Hewinson RG, Behr MA, Quail MA, Churcher C, Barrell BG, Parkhill J, Cole ST (2007) Genome plasticity of BCG and impact on vaccine efficacy. Proc Natl Acad Sci USA 104(13): 5596-5601
Brossay L, Naidenko O, Burdin N, Matsuda J, Sakai T, Kronenberg M (1998a) Structural requirements for galactosylceramide recognition by CD1-restricted NK T cells. J Immunol 161(10): 5124-5128
Brossay L, Tangri S, Bix M, Cardell S, Locksley R, Kronenberg M (1998b) Mouse CD1-autoreactive T cells have diverse patterns of reactivity to CD1+ targets. J Immunol 160(8): 3681-3688
Castanon-Arreola M, Lopez-Vidal Y, Espitia-Pinzon C, Hernandez-Pando R (2005) A new vaccine against tuberculosis shows greater protection in a mouse model with progressive pulmonary tuberculosis. Tuberculosis (Edinb) 85(1-2): 115-126
Chackerian A, Alt J, Perera V, Behar SM (2002) Activation of NKT cells protects mice from tuberculosis. Infect Immun 70(11): 6302-6309
Colditz GA, Brewer TF, Berkey CS, Wilson ME, Burdick E, Fineberg HV, Mosteller F (1994) Efficacy of BCG vaccine in the prevention of tuberculosis. Meta-analysis of the published literature. Jama 271(9): 698-702
Cooper AM, Dalton DK, Stewart TA, Griffin JP, Russell DG, Orme IM (1993) Disseminated tuberculosis in interferon gamma gene-disrupted mice. JExp Med 178(6): 2243-2247
Copenhaver RH, Sepulveda E, Armitige LY, Actor JK, Wanger A, Norris SJ, Hunter RL, Jagannath C (2004) A mutant of Mycobacterium tuberculosis H37Rv that lacks expression of antigen 85A is attenuated in mice but retains vaccinogenic potential. Infect Immun 72(12): 7084-7095
D'Souza CD, Cooper AM, Frank AA, Mazzaccaro RJ, Bloom BR, Orme IM (1997) An anti-inflammatory role for gamma delta T lymphocytes in acquired immunity to Mycobacterium tuberculosis. J Immunol 158(3): 1217-1221
Dalton DK, Pitts-Meek S, Keshav S, Figari IS, Bradley A, Stewart TA (1993) Multiple defects of immune cell function in mice with disrupted interferon-gamma genes. Science 259(5102): 1739-1742
Denis O, Tanghe A, Palfliet K, Jurion F, van den Berg TP, Vanonckelen A, Ooms J, Saman E, Ulmer JB, Content J, Huygen K (1998) Vaccination with plasmid DNA encoding mycobacterial antigen 85A stimulates a CD4+ and CD8+ T-cell epitopic repertoire broader than that stimulated by Mycobacterium tuberculosis H37Rv infection. Infect Immun 66(4): 1527-1533
Dennehy M, Williamson AL. (2005) Factors influencing the immune response to foreign antigen expressed in recombinant BCG vaccines. Vaccine 23(10): 1209-24.
Desmedt M, Rottiers P, Dooms H, Fiers W, Grooten J (1998) Macrophages induce cellular immunity by activating Th1 cell responses and suppressing Th2 cell responses. J Immunol 160(11): 5300-5308
Dinadayala P, Kaur D, Berg S, Amin AG, Vissa VD, Chatterjee D, Brennan PJ, Crick DC (2006) Genetic basis for the synthesis of the immunomodulatory mannose caps of lipoarabinomannan in Mycobacterium tuberculosis. J Biol Chem 281(29): 20027-20035
Forman HJ, Torres M (2001) Signaling by the respiratory burst in macrophages. IUBMB Life 51(6): 365-371
Fratti RA, Chua J, Vergne I, Deretic V (2003) Mycobacterium tuberculosis glycosylated phosphatidylinositol causes phagosome maturation arrest. Proc Natl Acad Sci U S A 100(9): 5437-5442
Grode L, Seiler P, Baumann S, Hess J, Brinkmann V, Nasser Eddine A, Mann P, Goosmann C, Bandermann S, Smith D, Bancroft GJ, Reyrat JM, van Soolingen D, Raupach B, Kaufmann SH (2005) Increased vaccine efficacy against tuberculosis of recombinant Mycobacterium bovis bacille Calmette-Guerin mutants that secrete listeriolysin. J Clin Invest 115(9): 2472-2479
Gutierrez MG, Master SS, Singh SB, Taylor GA, Colombo MI, Deretic V (2004) Autophagy is a defense mechanism inhibiting BCG and Mycobacterium tuberculosis survival in infected macrophages. Cell 119(6): 753-766
Harris J, Master SS, De Haro SA, Delgado M, Roberts EA, Hope JC, Keane J, Deretic V (2008) Th1-Th2 polarisation and autophagy in the control of intracellular mycobacteria by macrophages. Vet Immunol Immunopathol
Hinchey J, Lee S, Jeon BY, Basaraba RJ, Venkataswamy MM, Chen B, Chan J, Braunstein M, Orme IM, Derrick SC, Morris SL, Jacobs WR, Jr., Porcelli SA (2007) Enhanced priming of adaptive immunity by a proapoptotic mutant of Mycobacterium tuberculosis. J Clin Invest 117(8): 2279-2288
Hmama Z, Sendide K, Talal A, Garcia R, Dobos K, Reiner NE (2004) Quantitative analysis of phagolysosome fusion in intact cells: inhibition by mycobacterial lipoarabinomannan and rescue by an 1alpha,25-dihydroxyvitamin D3-phosphoinositide 3-kinase pathway. J Cell Sci 117(Pt 10): 2131-2140
Horwitz MA, Harth G (2003) A new vaccine against tuberculosis affords greater survival after challenge than the current vaccine in the guinea pig model of pulmonary tuberculosis. Infect Immun 71(4): 1672-1679
Horwitz MA (2005) Recombinant BCG expressing Mycobacterium tuberculosis major extracellular proteins. Microbes Infect 7(5-6):947-54.
Jagannath C, Lindsey DR, Dhandayuthapani S, Xu Y, Hunter RL, Jr., Eissa NT (2009) Autophagy enhances the efficacy of BCG vaccine by increasing peptide presentation in mouse dendritic cells. Nat Med 15(3): 267-276
Jiao X, Lo-Man R, Winter N, Deriaud E, Gicquel B, Leclerc C (2003) The shift of Th1 to Th2 immunodominance associated with the chronicity of Mycobacterium bovis bacille Calmette-Guerin infection does not affect the memory response. J Immunol 170(3): 1392-1398
Kamath AT, Fruth U, Brennan MJ, Dobbelaer R, Hubrechts P, Ho MM, Mayner RE, Thole J, Walker KB, Liu M, Lambert PH (2005) New live mycobacterial vaccines: the Geneva consensus on essential steps towards clinical development. Vaccine 23(29): 3753-3761
Kamijo R, Le J, Shapiro D, Havell EA, Huang S, Aguet M, Bosland M, Vilcek J (1993) Mice that lack the interferon-gamma receptor have profoundly altered responses to infection with Bacillus Calmette-Guerin and subsequent challenge with lipopolysaccharide. JExp Med 178(4): 1435-1440
Kang PB, Azad AK, Torrelles JB, Kaufman TM, Beharka A, Tibesar E, DesJardin LE, Schlesinger LS (2005) The human macrophage mannose receptor directs Mycobacterium tuberculosis lipoarabinomannan-mediated phagosome biogenesis. JExp Med 202(7): 987-999
Kantengwa S, Jornot L, Devenoges C, Nicod LP (2003) Superoxide anions induce the maturation of human dendritic cells. Am J Respir Crit Care Med 167(3): 431-437
Kaur D, Berg S, Dinadayala P, Gicquel B, Chatterjee D, McNeil MR, Vissa VD, Crick DC, Jackson M, Brennan PJ (2006) Biosynthesis of mycobacterial lipoarabinomannan: role of a branching mannosyltransferase. Proc Natl Acad Sci USA 103(37): 13664-13669
Kaur D, Obregon-Henao A, Pham H, Chatterjee D, Brennan PJ, Jackson M (2008) Lipoarabinomannan of Mycobacterium: mannose capping by a multifunctional terminal mannosyltransferase. Proc Natl Acad Sci U S A 105(46): 17973-17977
Kernodle DS (2010) Decrease in the effectiveness of Bacille Calmette-Guerin vaccine against pulmonary tuberculosis: a consequence of increased immune suppression by microbial antioxidants, not overattenuation. Clin Infect Dis 51(2): 177-184
Kindler V, Sappino AP, Grau GE, Piguet PF, Vassalli P (1989) The inducing role of tumor necrosis factor in the development of bactericidal granulomas during BCG infection. Cell 56(5): 731-740
Krzywinska E, Krzywinski J, Schorey JS (2004) Naturally occurring horizontal gene transfer and homologous recombination in Mycobacterium. Microbiology 150(Pt 6): 1707-1712
Ladel CH, Blum C, Dreher A, Reifenberg K, Kaufmann SH (1995) Protective role of gamma/delta T cells and alpha/beta T cells in tuberculosis. Eur J Immunol 25(10): 2877-2881
Luo Y, Yamada H, Chen X, Ryan AA, Evanoff DP, Triccas JA, O'Donnell MA (2004) Recombinant Mycobacterium bovis bacillus Calmette-Guérin (BCG) expressing mouse IL-18 augments Th1 immunity and macrophage cytotoxicity. Clin Exp Immunol 137(1): 24-34.
Martin C, Williams A, Hernandez-Pando R, Cardona PJ, Gormley E, Bordat Y, Soto CY, Clark SO, Hatch GJ, Aguilar D, Ausina V, Gicquel B (2006) The live Mycobacterium tuberculosis phoP mutant strain is more attenuated than BCG and confers protective immunity against tuberculosis in mice and guinea pigs. Vaccine 24(17): 3408-3419
Master SS, Rampini SK, Davis AS, Keller C, Ehlers S, Springer B, Timmins GS, Sander P, Deretic V (2008) Mycobacterium tuberculosis prevents inflammasome activation. Cell Host Microbe 3(4): 224-232
Orme IM, Roberts AD, Griffin JP, Abrams JS (1993) Cytokine secretion by CD4 T lymphocytes acquired in response to Mycobacterium tuberculosis infection. J Immunol 151(1): 518-525
Pym AS, Brodin P, Majlessi L, Brosch R, Demangel C, Williams A, Griffiths KE, Marchal G, Leclerc C, Cole ST (2003) Recombinant BCG exporting ESAT-6 confers enhanced protection against tuberculosis. Nat Med 9(5): 533-539
Rasband WS, ImageJ (2008), Vol. t: U.S. National Institutes of Health, Bethesda, Maryland, USA.
Rhodes SG, Palmer N, Graham SP, Bianco AE, Hewinson RG, Vordermeier HM (2000) Distinct response kinetics of gamma interferon and interleukin-4 in bovine tuberculosis. Infect Immun 68(9): 5393-5400
Rodrigues LC, Diwan VK, Wheeler JG (1993) Protective effect of BCG against tuberculous meningitis and miliary tuberculosis: a meta-analysis. Int J Epidemiol 22(6): 1154-1158
Romano M, D'Souza S, Adnet PY, Laali R, Jurion F, Palfliet K, Huygen K (2006) Priming but not boosting with plasmid DNA encoding mycolyl-transferase Ag85A from Mycobacterium tuberculosis increases the survival time of Mycobacterium bovis BCG vaccinated mice against low dose intravenous challenge with M. tuberculosis H37Rv. Vaccin 24(16): 3353-3364
Sada-Ovalle I, Chiba A, Gonzales A, Brenner MB, Behar SM (2008) Innate invariant NKT cells recognize Mycobacterium tuberculosis-infected macrophages, produce interferongamma, and kill intracellular bacteria. PLoS Pathog 4(12): e1000239
Sadagopal S, Braunstein M, Hager CC, Wei J, Daniel AK, Bochan MR, Crozier I, Smith NE, Gates HO, Barnett L, Van Kaer L, Price JO, Blackwell TS, Kalams SA, Kernodle DS (2009) Reducing the activity and secretion of microbial antioxidants enhances the immunogenicity of BCG. PLoS One 4(5): e5531
Sambandamurthy VK, Derrick SC, Jalapathy KV, Chen B, Russell RG, Morris SL, Jacobs WR, Jr. (2005) Long-term protection against tuberculosis following vaccination with a severely attenuated double lysine and pantothenate auxotroph of Mycobacterium tuberculosis. Infect Immun 73(2): 1196-1203
Sambandamurthy VK, Wang X, Chen B, Russell RG, Derrick S, Collins FM, Morris SL, Jacobs WR, Jr. (2002) A pantothenate auxotroph of Mycobacterium tuberculosis is highly attenuated and protects mice against tuberculosis. Nat Med 8(10): 1171-1174 STOP-TB-Partnership. (2009) TB vaccines pipeline.
Stover CK, de la Cruz VF, Fuerst TR, Burlein JE, Benson LA, Bennett LT, Bansal GP, Young JF, Lee MH, Hatfull GF, et al. (1991) New use of BCG for recombinant vaccines. Nature 351(6326): 456-60.
Sun R, Skeiky YA, Izzo A, Dheenadhayalan V, Imam Z, Penn E, Stagliano K, Haddock S, Mueller S, Fulkerson J, Scanga C, Grover A, Derrick SC, Morris S, Hone DM, Horwitz MA, Kaufmann SH, Sadoff JC (2009) Novel recombinant BCG expressing perfringolysin O and the over-expression of key immunodominant antigens; pre-clinical characterization, safety and protection against challenge with Mycobacterium tuberculosis. Vaccine 27(33): 4412-4423 Tanghe A, D'Souza S, Rosseels V, Denis O, Ottenhoff TH, Dalemans W, Wheeler C, Huygen K (2001) Improved immunogenicity and protective efficacy of a tuberculosis DNA vaccine encoding Ag85 by protein boosting. Infection and immunity 69(5): 3041-3047
Tchilian EZ, Desel C, Forbes EK, Bandermann S, Sander CR., Hill AV, McShane H, Kaufmann SH (2009) Immunogenicity and protective efficacy of prime-boost regimens with recombinant (delta)ureC hly+ Mycobacterium bovis BCG and modified vaccinia virus Ankara expressing M. tuberculosis antigen 85A against murine tuberculosis. Infect Immun 77(2): 622-631
Torrelles JB, Azad AK, Schlesinger LS (2006) Fine discrimination in the recognition of individual species of phosphatidyl-myo-inositol mannosides from Mycobacterium tuberculosis by C-type lectin pattern recognition receptors. J Immunol 177(3): 1805-1816
van der Veen RC, Dietlin TA, Karapetian A, Holland SM, Hofman FM (2004) Extra-cellular superoxide promotes T cell expansion through inactivation of nitric oxide. J Neuroimmunol 153(1-2): 183-189
van Crevel R, Ottenhoff TH, van der Meer JW (2002) Innate immunity to Mycobacterium tuberculosis. Clin Microbiol Rev 15(2): 294-309
Velmurugan K, Chen B, Miller JL, Azogue S, Gurses S, Hsu T, Glickman M, Jacobs WR, Jr., Porcelli SA, Briken V (2007) Mycobacterium tuberculosis nuoG is a virulence gene that inhibits apoptosis of infected host cells. PLoS Pathog 3(7): e110
Vergne I, Chua J, Deretic V (2003) Tuberculosis toxin blocking phagosome maturation inhibits a novel Ca2+/calmodulin-PI3K hVPS34 cascade. JExp Med 198(4): 653-659
Vergne I, Chua J, Lee HH, Lucas M, Belisle J, Deretic V (2005) Mechanism of phagolysosome biogenesis block by viable Mycobacterium tuberculosis. Proc Natl Acad Sci U S A 102(11): 4033-4038
Verreck FA, Vervenne RA, Kondova I, van Kralingen KW, Remarque EJ, Braskamp G, van der WerffNM, Kersbergen A, Ottenhoff TH, Heidt PJ, Gilbert SC, Gicquel B, Hill AV, Martin C, McShane H, Thomas AW (2009) MVA.85A boosting of BCG and an attenuated, phoP deficient M. tuberculosis vaccine both show protective efficacy against tuberculosis in rhesus macaques. PLoS One 4(4): e5264
Walker KB, Brennan MJ, Ho MM, Eskola J, Thiry G, Sadoff J, Dobbelaer R, Grode L, Liu MA, Fruth U, Lambert PH (2010) The second Geneva Consensus: Recommendations for novel live TB vaccines. Vaccine WHO Report (2008) Global tuberculosis control - surveillance, planning, financing.

## Claims

1. A mycobacterium which is a member of the *Mycobacterium tuberculosis* complex comprising a genetically engineered inactivating mutation in at least the endogenous gene SapM, wherein the SapM gene does not encode the hypothetical protein MAP3432.

2. The mycobacterium of claim 1, wherein the endogenous SapM gene comprises SEQ ID NO: 1, contiguous portions thereof, or sequences at least 95%, at least 98%, or at least 99% identical thereto; or comprises a sequence encoding SEQ ID NO: 2, contiguous portions thereof, or sequences at least 95%, at least 98%, or at least 99% identical thereto.

3. The mycobacterium of claim 1 or 2, which is a *Mycobacterium tuberculosis, Mycobacterium bovis* or M. *bovis* Bacille Calmette-Guérin (BCG).

4. The mycobacterium of any one of claims 1 to 3, wherein the genetically engineered mutation is created by insertion mutagenesis.

5. The mycobacterium according to any one of claims 1 to 4, which is from the deposited strain LMG P-25310.

6. The mycobacterium according to any one of claims 1 to 5, further comprising a genetically engineered mutation in at least one endogenous gene selected from the endogenous ManLAM α-1,2-mannosyltransferase corresponding to Mb1661c in *Mycobacterium bovis,* the endogenous ManLAM α-1,2-mannosyltransferase corresponding to Mb2203 in *Mycobacterium bovis* and the endogenous LM α-1,6-mannosyltransferase corresponding to Mb2196 in *Mycobacterium bovis.*

7. The mycobacterium according to any one of claims 1 to 6, further comprising a genetically engineered mutation in at least one endogenous gene selected from secA2, sigH, and SodA.

8. Use of a mycobacterium according to any one of claims 1 to 7 for the preparation of a recombinant vaccine.

9. The use according to claim 8, wherein the vaccine is a vaccine against tuberculosis.

10. The use according to claim 8 or 9, wherein the vaccine is a M. *bovis, M. bovis* Bacille Calmette-Guérin (BCG), or M. *tuberculosis-*based vaccine.

11. The use according to any one of claims 8 to 10, wherein the vaccine comprises a live attenuated vaccine.

12. A vaccine comprising a mycobacterium which is a member of the *Mycobacterium tuberculosis* complex comprising a genetically engineered inactivating mutation in at least the endogenous gene SapM, wherein the SapM gene does not encode the hypothetical protein MAP3432, in a pharmaceutically acceptable carrier or excipient.

13. The vaccine of claim 12 against tuberculosis, wherein the vaccine is suitable to protect a mammal from challenge by a virulent mycobacterium selected from M. *bovis* or M. *tuberculosis.*

14. The vaccine of claim 12 or 13, wherein the vaccine comprises a live attenuated vaccine.

15. The mycobacterium of any one of claims 1 to 7 or vaccine of any one of claims 12 to 14 for use in treatment of tuberculosis.

## Patentansprüche

1. Mykobakterium, das ein Element des *Mycobacterium-tuberculosis-*Komplexes ist und das eine gentechnisch konstruierte inaktivierende Mutation in mindestens dem endogenen Gen SapM umfasst, wobei das SapM-Gen das hypothetische Protein MAP3432 nicht kodiert.

2. Mykobakterium nach Anspruch 1, wobei das endogene SapM-Gen SEQ ID Nr. 1, zusammenhängende Abschnitte davon oder Sequenzen, die zu mindestens 95 %, mindestens 98 % oder mindestens 99 % dazu identisch sind, umfasst oder eine Sequenz umfasst, die SEQ ID Nr. 2, zusammenhängende Abschnitte davon oder Sequenzen, die zu mindestens 95 %, mindestens 98 % oder mindestens 99 % dazu identisch sind, kodieren.

3. Mykobakterium nach Anspruch 1 oder 2, wobei es sich um ein *Mycobacterium tuberculosis, Mycobacterium bovis* oder *M.-bovis*-Bacille Calmette-Guérin (BCB) handelt.

4. Mykobakterium nach einem der Ansprüche 1 bis 3, wobei die gentechnisch konstruierte Mutation durch Insertionsmutagenese erzeugt wird.

5. Mykobakterium nach einem der Ansprüche 1 bis 4, das von dem hinterlegten Stamm LMG P-25310 stammt.

6. Mykobakterium nach einem der Ansprüche 1 bis 5, das weiterhin eine gentechnisch konstruierte Mutation in mindestens einem endogenen Gen umfasst, das aus der endogenen ManLAM-α-1,2-Mannosyltransferase, die Mb1661c in *Mycobacterium bovis* entspricht, der endogenen ManLAM-α-1,2-Mannosyltransferase, die Mb2203 in *Mycobacterium bovis* entspricht, und der endogenen LM-α-1,6-Mannosyltransferase, die Mb2196 in *Mycobacterium bovis* entspricht, ausgewählt ist.

7. Mykobakterium nach einem der Ansprüche 1 bis 6, das weiterhin eine gentechnisch konstruierte Mutation in mindestens einem endogenen Gen umfasst, das aus secA2, sigH und SodA ausgewählt ist.

8. Verwendung eines Mykobakteriums nach einem der Ansprüche 1 bis 7 zur Herstellung eines rekombinanten Impfstoffs.

9. Verwendung nach Anspruch 8, wobei der Impfstoff ein Impfstoff gegen Tuberkulose ist.

10. Verwendung nach Anspruch 8 oder 9, wobei der Impfstoff ein Impfstoff auf *M.-bovis-, M.-bovis-*Bacille-Calmette-Guerin- (BCG) oder *M.-tuberculosis-Basis* ist.

11. Verwendung nach einem der Ansprüche 8 bis 10, wobei der Impfstoff einen lebend-attenuierten Impfstoff umfasst.

12. Impfstoff, der ein Mykobakterium, das ein Element des *Mycobacterium-tuberculosis-*Komplexes ist und das eine gentechnisch konstruierte inaktivierende Mutation in mindestens dem endogenen Gen SapM umfasst, wobei das SapM-Gen das hypothetische Protein MAP3432 nicht kodiert, in einem pharmazeutisch unbedenklichen Träger oder Hilfsstoff umfasst.

13. Impfstoff nach Anspruch 12 gegen Tuberkulose, wobei der Impfstoff dafür geeignet ist, ein Säugetier vor einer Belastung durch ein virulentes Mykobakterium zu schützen, das aus M. *bovis* oder M. *tuberculosis* ausgewählt ist.

14. Impfstoff nach Anspruch 12 oder 13, wobei der Impfstoff einen lebend-attenuierten Impfstoff umfasst.

15. Mykobakterium nach einem der Ansprüche 1 bis 7 oder Impfstoff nach einem der Ansprüche 12 bis 14 zur Verwendung bei der Behandlung von Tuberkulose.

## Revendications

1. Mycobactérie qui est un membre du complexe *Mycobacterium tuberculosis* comprenant une mutation de désactivation créée par génie génétique dans au moins le gène endogène SapM, dans laquelle le gène SapM ne code pas pour la protéine hypothétique MAP3432.

2. Mycobactérie selon la revendication 1, dans lequel le gène SapM endogène comprend SEQ ID N° : 1, des portions contiguës de celle-ci ou des séquences identiques à au moins 95 %, au moins 98 % ou au moins 99 % à celle-ci ; ou comprend une séquence codant pour SEQ ID N° : 2, des portions contiguës de celle-ci ou des séquences identiques à au moins 95 %, au moins 98 % ou au moins 99 % à celle-ci.

3. Mycobactérie selon la revendication 1 ou 2, qui est une *Mycobacterium tuberculosis, Mycobacterium bovis* ou une Bacille Calmette-Guérin (BCG) de M. *bovis.*

4. Mycobactérie selon l'une quelconque des revendications 1 à 3, dans laquelle la mutation créée par génie génétique est créée par mutagenèse par insertion.

5. Mycobactérie selon l'une quelconque des revendications 1 à 4, qui provient de la souche déposée LMG P-25310.

6. Mycobactérie selon l'une quelconque des revendications 1 à 5, comprenant en outre une mutation créée par génie génétique dans au moins un gène endogène sélectionné parmi la ManLAM α-1,2-mannosyltransférase endogène correspondant à Mb1661c dans *Mycobacterium bovis,* la ManLAM α-1,2-mannosyltransférase endogène correspondant à Mb2203 dans *Mycobacterium bovis* et la LM α-1,6-mannosyltransférase endogène correspondant à Mb2196 dans *Mycobacterium bovis.*

7. Mycobactérie selon l'une quelconque des revendications 1 à 6, comprenant en outre une mutation créée par génie génétique dans au moins un gène endogène sélectionné parmi secA2, sigH et SodA.

8. Utilisation d'une mycobactérie selon l'une quelconque des revendications 1 à 7 pour la préparation d'un vaccin recombiné.

9. Utilisation selon la revendication 8, dans laquelle le vaccin est un vaccin contre la tuberculose.

10. Utilisation selon la revendication 8 ou 9, dans laquelle le vaccin est un vaccin à base de *M. bovis,* Bacille Calmette-Guérin (BCG) de M. *bovis* ou M. *tuberculosis.*

11. Utilisation selon l'une quelconque des revendications 8 à 10, dans laquelle le vaccin comprend un vaccin vivant atténué.

12. Vaccin comprenant une mycobactérie qui est un membre du complexe *Mycobacterium tuberculosis* comprenant une mutation de désactivation créée par génie génétique dans au moins le gène endogène SapM, dans lequel le gène SapM ne code pas pour la protéine hypothétique MAP3432A, dans un support ou excipient pharmaceutiquement acceptable.

13. Vaccin selon la revendication 12 contre la tuberculose, dans lequel le vaccin convient à protéger un mammifère d'une provocation par une mycobactérie virulente sélectionnée parmi M. *bovis* ou M. *tuberculosis.*

14. Vaccin selon la revendication 12 ou 13, dans lequel le vaccin comprend un vaccin vivant atténué.

15. Mycobactérie selon l'une quelconque des revendications 1 à 7 ou vaccin selon l'une quelconque des revendications 12 à 14 destiné(e) à être utilisé(e) dans le traitement de la tuberculose.
